(19) Europäisches Patentamt
European Patent Office
Office européen des brevets

(11) Veröffentlichungsnummer: **0 114 573**
**B1**

# (12) EUROPÄISCHE PATENTSCHRIFT

(45) Veröffentlichungstag der Patentschrift: **20.04.88**

(21) Anmeldenummer: **83810596.3**

(22) Anmeldetag: **15.12.83**

(51) Int. Cl.[4]: **C 07 D 471/04**, A 61 K 31/44 // C07D213/36, C07D213/55, C07D213/65, C07D213/84, C07D213/89 ,(C07D471/04, 235:00, 221:00)

## (54) **Imidazo(1,5-a)pyridin-Derivate.**

(30) Priorität: **21.12.82 US 451902**

(43) Veröffentlichungstag der Anmeldung: **01.08.84 Patentblatt 84/31**

(45) Bekanntmachung des Hinweises auf die Patenterteilung: **20.04.88 Patentblatt 88/16**

(84) Benannte Vertragsstaaten: **AT BE CH DE FR GB IT LI LU NL SE**

(56) Entgegenhaltungen:
**EP - A - 0 068 386**
**FR - A - 2 130 215**

**TETRAHEDRON LETTERS, Band 21, Nr. 22, 1980, Seiten 2195-2196, Pergamon Press Ltd., Oxford, GB, P. BLATCHER et al.: "A direct method for the substitution of imidazo(1,5-a)pyridines at position 5"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Browne, Leslie J., Dr., 41 Malapardis Road, Morris Plains, N.J. 07950 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft Imidazo[1,5-a]pyridine, deren Salze und Verfahren zu ihrer Herstellung.

FR-A-2 130 215 beschreibt 3-Amino-imidazo[1,5-a]pyridine mit antisekretorischer Wirkung im Magen ohne anticholinerge Nebenwirkungen. In Tetrahedron Letters 21, 2195–2196 (1980) wird ein Syntheseweg zur direkten Substitution von Imidazo[1,5-a]pyridinen in 5-Stellung angegeben. In einer früheren Anmeldung, EP-A-68 386, und einer parallelen Anmeldung, EP-A-114 572, der Patentanmelderin sind substituierte Imidazo[1,5-a]pyridine beschrieben, die eine Hemmung der Thromboxan-Synthese bewirken.

Die vorliegende Erfindung betrifft Imidazo[1,5-a]pyridin-Derivate der Formel I

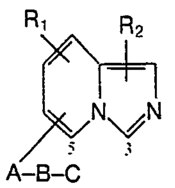

(I)

und ihre 5,6,7,8-Tetrahydroderivate, worin $R_1$ Wasserstoff, Halogen, Niederalkyl, Niederalkoxy, Hydroxy oder Arylniederalkoxy, worin Aryl Phenyl oder durch Niederalkoxy, Niederalkyl, Halogen oder Trifluormethyl mono- oder disubstituiertes Phenyl ist, bedeutet, $R_2$ Wasserstoff, Halogen oder Niederalkyl darstellt, C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

(a) A unsubstituiertes oder durch Niederalkyl substituiertes Äthenylen (Vinylen) und B Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen, Phenylen-(thio oder oxy)-$C_1$–$C_4$-alkylen, oder

(b) A eine Bindung und B $C_1$–$C_4$-Alkylen-phenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, und ihre Salze, insbesondere ihre therapeutisch verwendbaren Salze, wobei die mit «nieder» bezeichneten Reste höchstens 7 Kohlenstoffatome aufweisen, sowie Verfahren zu ihrer Herstellung, pharmazeutische Präparate enthaltend diese Verbindungen, sowie ihre therapeutische Verwendung.

Bevorzugt sind Verbindungen der allgemeinen Formel I, in welchen die Gruppe –A–B–C an die 5-Stellung gebunden ist.

Hervorzuheben sind Verbindungen der Formel I, worin $R_1$ Wasserstoff, Halogen, Niederalkyl, Niederalkoxy, Hydroxy, Phenyl-niederalkoxy oder Phenyl-niederalkoxy, das im Phenylring durch Niederalkoxy, Niederalkyl oder Halogen mono- oder disubstituiert ist, bedeutet, $R_2$ Wasserstoff, Halogen oder Niederalkyl darstellt, A Äthenylen oder durch Niederalkyl substituiertes Äthenylen bedeutet, B für Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen oder Phenylen-(thio oder oxy)-$C_1$–$C_4$-alkylen steht, C Carboxy, Niederalkoxycarbonyl, unsubstituiertes, mono- oder di-niederalkyl-substituiertes Carbamoyl, Cyan, Hydroxymethyl, Formyl, 5-Tetrazolyl, unsubstituiertes oder durch Niederalkyl substituiertes 4,5-Dihydro-2-oxazolyl, oder Hydroxycarbamoyl bedeutet, und ihre Salze, insbesondere ihre therapeutisch verwendbaren Salze.

Bevorzugt sind Verbindungen der allgemeinen Formel I, worin A Äthenylen (= Vinyl) bedeutet, B Alkylen mit 1 bis 12 Kohlenstoffatomen, Alkenylen mit 2 bis 12 Kohlenstoffatomen, Phenylen oder Phenylenoxy-niederalkylen bedeutet und $R_1$, $R_2$ und C die oben definierte Bedeutung haben, und ihre Salze, insbesondere ihre therapeutisch verwendbaren Salze.

Besonders bevorzugt sind Verbindungen der Formel I, worin A Äthenylen bedeutet, B für Alkylen oder Alkenylen mit 2 bis 6 Kohlenstoffatomen, Phenylen oder Phenylen-oxy-alkylen mit 7 bis 11 Kohlenstoffatomen steht, $R_1$ und $R_2$ Wasserstoff bedeuten und C Carboxy oder Niederalkoxycarbonyl darstellt, und die Gruppe A–B–C an die 5-Stellung gebunden ist.

In erster Linie bevorzugt sind Verbindungen der Formel II

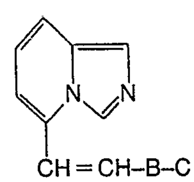

(II)

worin B Alkylen oder Alkenylen mit jeweils 2 bis 4 Kohlenstoffatomen bedeutet, C Carboxy oder Niederalkoxycarbonyl darstellt, und ihre Salze, insbesondere ihre therapeutisch verwendbaren Salze.

Bevorzugt sind weiter Verbindungen der Formel I oder ihre 5,6,7,8-Tetrahydro-Derivate, worin $R_1$ Wasserstoff, Halogen, Niederalkyl, Niederalkoxy, Hydroxy oder Aryl-niederalkoxy, worin Aryl unsubstituiertes oder durch Niederalkoxy, Niederalkyl, Halogen oder Trifluormethyl mono- oder di-substituiertes Phenyl ist, bedeutet, $R_2$ Wasserstoff, Halogen oder Niederalkyl darstellt, C Carboxy, Niederalkoxycarbonyl, unsubstituiertes oder mono- oder di-niederalkyl-substituiertes Carbamoyl, Cyan, Hydroxymethyl, Formyl, 5-Tetrazolyl, unsubstituiertes oder durch Niederalkyl substituiertes 4,5-Dihydro-2-oxazolyl, oder Hydroxycarbamoyl bedeutet, A für eine einfache Bindung steht und B $C_1$–$C_4$-Alkylenphenylen-$C_1$–$C_4$-alkylen, $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-

alkenylen oder Alkadienylen mit 5 bis 12 Kohlenstoffatomen bedeutet, und ihre Salze, insbesondere ihre therapeutisch verwendbaren Salze, wobei die mit «nieder» bezeichneten Reste höchstens 7 Kohlenstoffatome aufweisen.

Bevorzugt sind Verbindungen der Formel I, worin A eine einfache Bindung bedeutet, B Phenylen, Alkylen-phenylen, Phenylen-alkylen, Alkylenthiophenylen oder Alkylenoxyphenylen mit jeweils 7 bis 10 Kohlenstoffatomen, Niederalkylen-(thio oder oxy)-niederalkylen mit 4 bis 10 Kohlenstoffatomen oder Phenylen-niederalkenylen mit 8 bis 10 Kohlenstoffatomen bedeutet, C Carboxy, Niederalkoxycarbonyl, Carbamoyl, Hydroxycarbamoyl, 5-Tetrazolyl oder Hydroxymethyl bedeutet, und $R_1$ und $R_2$ Wasserstoff darstellen, und ihre Salze, insbesondere ihre therapeutisch verwendbaren Salze.

Besonders bevorzugt sind schliesslich Verbindungen der Formel I, worin B Niederalkylen-(thio oder oxy)-niederalkylen mit 4 bis 10 Kohlenstoffatomen, Phenylen oder Phenylen-niederalkenylen mit 8 bis 10 Kohlenstoffatomen bedeutet, und A, C, $R_1$ und $R_2$ die oben definierte Bedeutung haben.

Besonders wertvoll sind Verbindungen der Formel I, worin die Gruppe –B–C direkt an die 5-Stellung des Imidazo[1,5-a]pyridin-Kerns gebunden ist.

Ganz besonders bevorzugt sind Verbindungen der Formel III

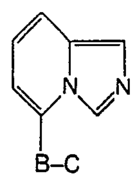

(III),

worin B Niederalkylen-(thio oder oxy)-niederalkylen mit 4 bis 10 Kohlenstoffatomen, Phenylen, Phenylen-niederalkylen oder Niederalkylen-phenylen mit jeweils 7 bis 10 Kohlenstoffatomen, Alkylenthiophenylen oder Alkylenoxyphenylen mit jeweils 7 bis 10 Kohlenstoffatomen oder Phenylen-niederalkenylen mit 8 bis 10 Kohlenstoffatomen bedeutet, C Carboxy, Niederalkoxycarbonyl oder Carbamoyl darstellt, und ihre Salze, insbesondere ihre therapeutisch verwendbaren Salze.

Die vor- und nachstehend verwendeten allgemeinen Definitionen haben im Rahmen der vorliegenden Beschreibung die folgenden Bedeutungen:

Ein Alkylenrest mit 1 bis 12 Kohlenstoffatomen kann geradkettig oder verzweigt sein und steht vorzugsweise für Propylen, Butylen, Pentylen oder Hexylen, wobei die genannten Reste unsubstituiert oder durch eine oder mehrere Niederalkylgruppen substituiert sind, mit der Massgabe, dass die Summe der Kohlenstoffatome nicht grösser als 12 ist.

Ein Alkenylenrest mit 2 bis 12 Kohlenstoffatomen kann geradkettig oder verzweigt sein und steht vorzugsweise für Äthenylen (Vinylen), Propenylen, 1- oder 2-Butenylen, 1- oder 2 Pentenylen, 1-, 2- oder 3-Hexenylen. Die genannten Reste sind unsubstituiert oder durch eine oder mehrere Niederalkylgruppen substituiert, mit der Massgabe, dass die Summe der Kohlenstoffatome 12 nicht übersteigt.

Ein Alkinylrest mit 2 bis 12 Kohlenstoffatomen, der geradkettig oder verzweigt ist, steht vorzugsweise für Äthinylen, Propinylen, 1- oder 2-Butinylen, 1- oder 2-Pentinylen, 1-, 2- oder 3-Hexinylen. Diese Reste sind unsubstituiert oder durch eine oder mehrere Niederalkylgruppen substituiert, wobei die Summe der Kohlenstoffatome 12 nicht übersteigt.

Der Ausdruck Alkadienylen mit 5 bis 12 Kohlenstoffatomen ist vorzugsweise Niederalkylen-butadienyl mit 1 bis 7 und insbesondere 2 bis 5 Kohlenstoffatomen im Niederalkylenteil.

Der Ausdruck «nieder» definiert in den oben oder nachfolgend genannten organischen Resten oder Verbindungen solche mit höchstens 7, vorzugsweise 4 und insbesondere 1 oder 2 Kohlenstoffatomen.

Eine Niederalkylgruppe enthält vorzugsweise 1 bis 4 Kohlenstoffatome und steht z.B. für Äthyl, Propyl oder Butyl, und insbesondere für Methyl.

Eine Niederalkoxycarbonylgruppe enthält vorzugsweise 1 bis 4 Kohlenstoffatome im Alkoxyteil und bedeutet z.B. Methoxycarbonyl, Propoxycarbonyl oder Isopropoxycarbonyl, und insbesondere Äthoxycarbonyl. Eine Mono-(niederalkyl)-carbamoylgruppe hat vorzugsweise 1 bis 4 Kohlenstoffatome im Alkylteil und ist z.B. N-Methylcarbamoyl, N-Propylcarbamoyl oder insbesondere N-Äthylcarbamoyl. Eine Di-(niederalkyl)-carbamoylgruppe enthält vorzugsweise 1 bis 4 Kohlenstoffatome in jeder Niederalkylgruppe und steht z.B. für N,N-Dimethylcarbamoyl, N-Methyl-N-äthylcarbamoyl und insbesondere für N,N-Diäthylcarbamoyl.

Phenylen bedeutet 1,2-, 1,3- und vorzugsweise 1,4-Phenylen. Niederalkylen-phenylen, Phenylen-niederalkylen, Niederalkylen-phenylen-niederalkylen, Niederalkylen-(thio oder oxy)-phenylen, Phenylen-(thio oder oxy)-niederalkylen, Phenylen-niederalkenylen oder Niederalkylenphenylen-niederalkenylen enthält in jedem Niederalkylenteil vorzugsweise 1 bis 4 Kohlenstoffatome und in jedem Niederalkenylenteil 2 bis 4 Kohlenstoffatome. Die Niederalkylen- und Niederalkenylenteile können unverzweigt oder verzweigt sein.

Eine Niederalkylen-(thio oder oxy)-niederalkylen-Gruppe kann unverzweigt oder verzweigt sein und enthält insgesamt 2 bis 12 Kohlenstoffatome, vorzugsweise 4 bis 10 und insbesondere 3 bis 6 Kohlenstoffatome.

Ein Arylrest in einer Arylniederalkoxy-Gruppe ist insbesondere Phenyl oder Phenyl, das durch Niederalkyl, Halogen oder Niederalkoxy mono- oder disubstituiert ist.

Ein Arylniederalkoxyrest ist insbesondere Benzyloxy.

Niederalkoxy enthält vorzugsweise 1 bis 4 Kohlenstoffatome und steht z.B. für Äthoxy, Propoxy und insbesondere für Methoxy.

Halogen ist vorzugsweise Fluor und Chlor, kann aber auch Brom oder Jod sein.

Salze sind vorzugsweise therapeutisch verwendbare Salze, z. B. Metall- oder Ammoniumsalze der genannten Verbindungen der Formel I, worin C Carboxy, 5-Tetrazolyl oder Hydroxycarbamoyl bedeutet, insbesondere Alkalimetall- oder Erdalkalimetallsalze, z. B. Natrium-, Kalium-, Magnesium- oder Calciumsalze; in erster Linie leicht kristallisierende Ammoniumsalze. Diese werden abgeleitet von Ammoniak oder organischen Aminen, z. B. Mono-, Di- oder Tri-nieder-(alkyl, cycloalkyl oder hydroxyalkyl)-aminen, Niederalkylendiaminen oder (Hydroxy-niederalkyl oder Arylniederalkyl)-niederalkylammonium-Basen, z. B. Methylamin, Diäthylamin, Triäthylamin, Dicyclohexylamin, Triäthanolamin, Äthylendiamin, Tris-(hydroxymethyl)-aminomethan oder Benzyltrimethylammoniumhydroxid. Die genannten Verbindungen der Formel I bilden Säureadditionssalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z. B. starke Mineralsäuren, wie Halogenwasserstoffsäuren, z. B. Chlorwasserstoff- oder Bromwasserstoffsäure, Schwefel-, Phosphor-, Salpeter- oder Perchlorsäure; oder organische Säuren, wie aliphatische oder aromatische Carbon- oder Sulfonsäuren, z. B. Ameisen-, Essig-, Propion-, Bernstein-, Glykol-, Milch-, Äpfel-, Wein-, Glucon-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Äthansulfon-, Hydroxyäthansulfon-, Benzolsulfon-, p-Toluolsulfon-, Naphthalinsulfon-, Sulfanil- oder Cyclohexylsulfaminsäure; oder Ascorbinsäure.

Die Verbindungen der Erfindung zeigen wertvolle pharmakologische Eigenschaften, z. B. kardiovaskulare Effekte, durch selektive Hemmung der Thromboxan-Ausschüttung in Säugern. Diese Hemmung kommt durch selektive Verminderung der Thromboxan-Synthetase zustande. Die Verbindungen sind daher nützlich in der Behandlung von Krankheiten, welche auf Thromboxan-Synthetase-Hemmung in Säugern, einschliesslich Menschen, ansprechen.

Diese Wirkungen können durch in vitro-Versuche oder in vivo, vorzugsweise an Säugetieren, z. B. Meerschweinchen, Mäusen, Ratten, Katzen, Hunden oder Affen nachgewiesen werden. Die genannten Verbindungen können ihnen enteral oder parenteral, vorzugsweise oral oder subkutan, intravenös oder intraperitoneal, z. B. durch Gelatine-Kapseln oder in Form von Stärke enthaltenden Suspensionen oder wässerigen Lösungen, verabreicht werden. Die verwendete Dosis kann in einem Bereich von ungefähr zwischen 0,01 und 100 mg/kg/Tag, vorzugsweise ungefähr 0,10 und 50 mg/kg/Tag, insbesondere ungefähr 1 und 25 mg/kg/Tag liegen.

Die in vitro-Hemmung des Thromboxan-Synthetase-Enzyms kann analog zu der Methode von Sun, Biochem. Biophys. Res. Comm. 74, 1432 (1977) nachgewiesen werden. Das Testverfahren wird wie folgt durchgeführt:

$^{14}C$-Arachidonsäure wird mit einem Enzymgemisch-Präparat, bestehend aus solubilisierter und partiell gereinigter Prostaglandin-cyclooxygenase von Schaf-Samenblasen und aus einem rohen Mikrosomen-Präparat von Thromboxan-Synthetase von lysierten menschlichen Blutplättchen, inkubiert. Die Testverbindung (gelöst in einem Puffer oder, falls nötig, in wenig Äthanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch von Prostaglandin $F_2\alpha$ und $F_2\beta$ [$PGF_2(\alpha+\beta)$] reduziert. Die radioaktiven Produkte und das überschüssige Substrat werden mit Essigsäureäthylester extrahiert und der Extrakt zur Trockene eingedampft. Der Rückstand wird in Aceton gelöst, auf Dünnschichtplatten tropfenweise aufgetragen und mit einem Lösungsmittelsystem von Toluol:Aceton:Eisessig [100:100:3 (Volumen)] chromatographiert. Die radioaktiven Zonen werden lokalisiert. Die Zonen von Thromboxan $B_2$ ($TxB_2$) und $PGF_2\alpha+\beta$ werden in Szintillationsröhrchen für Flüssigkeiten übertragen und gezählt. Der Quotient der Zahlenwerte von $TxB_2/PGF_2\alpha+\beta$ wird für jede Konzentration der Testverbindung berechnet und die $IC_{50}$-Werte graphisch ermittelt. Dieser Wert ist die Konzentration der Testverbindung, bei welcher der Quotient von $TxB_2/PGF_2\alpha+\beta$ auf 50% des Kontrollwertes reduziert wird.

Die in vitro Wirkung auf Prostaglandin-cyclooxygenase wird gemäss einer Modifikation der Methode von Takeguchi et al., welche in Biochemistry 10, 2372 (1971) beschrieben ist, gemessen. Das Testverfahren sieht wie folgt aus:

Lyophilisierte Samenblasen-Mikrosomen von Schafen werden als Enzympräparat für die Prostaglandin-Synthese verwendet. Es wird die Umwandlung von $^{14}C$-Arachidonsäure in $PGE_2$ gemessen. Die Testverbindungen (gelöst in einem Puffer oder, wenn nötig, in wenig Äthanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen in Szintillationsröhrchen für Flüssigkeiten übertragen und ihre Radioaktivität gezählt. Die $IC_{50}$-Werte der Hemmung werden graphisch ermittelt. Dieser Wert bedeutet die Konzentration der Testverbindung, welche die Menge des synthetisierten $PGE_2$ um 50% reduziert.

Die in vitro-Wirkung auf Prostacyclin-($PGI_2$)-Synthetase wird analog zu der Methode von Sun et al., Prostaglandins 14, 1055 (1977) gemessen. Das Testverfahren wird wie folgt durchgeführt:

$^{14}C$-Arachidonsäure wird mit einem Enzymgemisch, bestehend aus solubilisierter und partiell gereinigter Prostaglandin-cyclooxygenase von Schaf-Samenblasen und aus roher $PGI_2$-Synthetase in Form einer Mikrosomen-Fraktion von Aorten von Rindern, inkubiert.

Die Testverbindung (gelöst in einem Puffer oder, wenn nötig, in wenig Äthanol) wird in das

Inkubationsmedium gegeben. Das Reaktionsgemisch wird in 100 mM Tris HCl (pH 7,5) 30 Minuten bei 37° inkubiert, auf den pH-Wert 3 angesäuert und mit Essigsäureäthylester extrahiert. Der Extrakt wird zur Trockene eingedampft, der Rückstand in Aceton gelöst, auf Dünnschicht-Platten aufgetragen und mit dem von Sun et al. beschriebenen Lösungsmittelsystem chromatographiert. Die radioaktiven Zonen werden mit einem Detektor lokalisiert. Die dem 6-Keto-$PGF_1\alpha$ (einem stabilen Endprodukt der Prostacyclin-Biotransformation) und $PGE_2$ entsprechenden Zonen werden in Szintillationsröhrchen für Flüssigkeiten übertragen und gezählt. Der Quotient der Zahlenwerte von 6-Keto-$PGF_1\alpha/PGE_2$ wird für jede Konzentration der verwendeten Testverbindung berechnet. Die $IC_{50}$-Werte der Hemmung werden graphisch ermittelt. Dieser Wert ist die Konzentration der Testverbindung, bei welcher der Quotient von 6-Keto-$PGF_1\alpha/PGE_2$ auf 50% des Kontrollwertes reduziert wird.

Die Hemmung der Synthese und die Reduzierung des Plasmaspiegels von Thromboxan wird in vivo dadurch bestimmt, dass man Ratten eine Testverbindung in folgender Weise verabreicht [vgl. auch die von Tai et al., Anal. Biochem. 87, 343 (1978), und von Salmon, Prostaglandins 15, 383 (1978) beschriebenen Verfahren]:

Ratten werden mit einem Trägermaterial bzw. der Testverbindung behandelt; zwei Stunden später wird Ionophor A 23187 (0,5 mg/kg) i.v. injiziert. 2 Minuten nach der Ionophor-Injektion wird das Blut für die Analyse gesammelt. Eine Probe von jedem Plasma wird auf Thromboxan $B_2$ und eine andere Probe auf 6-Keto-$PGF_1\alpha$, die stabilen Metaboliten von Thromboxan $A_2$ und Prostacyclin ($PGI_2$), mittels Radioimmunotest geprüft.

Die Verbindungen der Formel I sind sehr wirksame Thromboxan-Synthetase-Inhibitoren. Bei wirksamen Dosis-Spiegeln und darüber wird weder das vorteilhafte Prostacyclin-Synthetasenoch das Prostaglandin-cyclooxygenase-Enzymsystem gehemmt. Überraschenderweise wird der Prostacyclin-Spiegel signifikant erhöht.

Der $IC_{50}$-Wert für eine Verbindung der Erfindung, z.B. für das 5-[p-(2-Carboxyprop-1-enyl)-phenyl]imidazo[1,5-a]pyridin beträgt $1 \times 10^{-7}$ Mol für die Thromboxan-Synthetase-Hemmung. Die genannte Verbindung reduziert ferner die Plasmakonzentration von Thromboxan $B_2$ um über 50% bei einer oralen Dosis von 5 mg/kg oder weniger bei der Ratte. Ein signifikanter Anstieg beim Plasmaspiegel des Prostacyclin-Metaboliten 6-Keto-$PGF_1\alpha$ wird bei einer ähnlichen Dosierung beobachtet.

Weiterhin besitzt z.B. das 5-(4-Carboxybuta-1,3-dienyl)-imidazo[1,5-a]pyridin einen $IC_{50}$-Wert von $3 \times 10^{-8}$ Mol für die Thromboxan-Synthetase-Hemmung. Die genannte Verbindung reduziert die Plasmakonzentration von Thromboxan $B_2$ um über 50% bei der Ratte bei einer oralen Dosis von 5 mg/kg p.o. oder weniger. Ein signifikanter Anstieg beim Plasmaspiegel des Prostacyclin-Metaboliten 6-Keto-$PGF_1\alpha$ wird ebenfalls beobachtet.

Aufgrund der vorher genannten vorteilhaften Eigenschaften sind die Verbindungen der Erfindung für Säuger als spezifische therapeutische Mittel, z.B. zur Behandlung von cardiovaskulären Krankheiten wie etwa Thrombo-Embolie, sehr wertvoll.

Die Verbindungen der vorliegenden Erfindung können in an sich bekannter Weise hergestellt werden. Solche, worin $R_2$ Wasserstoff oder Niederalkyl bedeutet, erhält man z.B., indem man

a) eine Verbindung der Formel IV

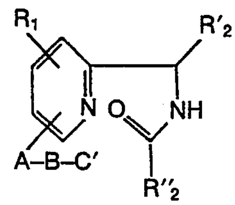

(IV),

worin $R_2'$ und $R_2''$ Wasserstoff oder einer der Reste $R_2'$ und $R_2''$ Niederalkyl bedeuten, $R_1$, A und B die unter Formel I angegebene Bedeutung haben und C' Carboxy, Niederalkoxycarbonyl, unsubstituiertes, mono- oder di-niederalkyl-substituiertes Carbamoyl, Cyan, Hydroxymethyl, Niederalkanoyloxymethyl, veräthertes Hydroxymethyl, Halogenmethyl, 5-Tetrazolyl, unsubstituiertes oder durch Niederalkyl substituiertes 4,5-Dihydro-2-oxazolyl, Hydroxycarbamoyl, Halogen oder Niederalkylendioxymethyl bedeutet, ringschliesst, und eine erhaltene Verbindung der Formel Ia

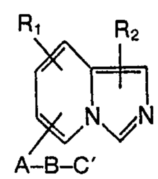

(Ia),

worin sich C' von C unterscheidet, in eine Verbindung der Formel I umwandelt und, wenn erwünscht, eine erhaltene Verbindung der Formel I in eine andere Verbindung der Erfindung umwandelt.

Die Cyclisierung eines Amids der Formel IV wird vorteilhaft unter Bedingungen, wie sie für die Cyclisierung des 6-Methyl-2-methylaminopyridins zu 5-Methylimidazo[1,5-a]pyridin in J. Org. Chem. 40, 1210 (1975) beschrieben ist, durchgeführt. Dieser Ringschluss wird vorzugsweise mit einer Lewissäure, z.B. Polyphosphorsäure, Phosphoroxychlorid oder Polyphosphatester, gegebenenfalls in einem inerten Lösungsmittel, z.B. Toluol, in einem Temperaturbereich zwischen 25° bis 150°, vorzugsweise 50° bis 120° durchgeführt.

Die Amide der Formel IV werden z.B. durch Acylierung einer Verbindung der Formel V

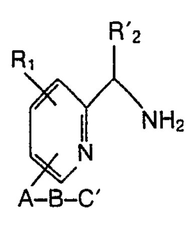

(V),

worin $R_1$, $R_2'$, A, B und C′ die oben angegebene Bedeutung haben, mit einer Carbonsäure der Formel VI

$$R_2''\text{–COOH} \qquad \text{(VI)},$$

worin $R_2''$ die oben angegebene Bedeutung hat, oder mit einem reaktionsfähigen funktionellen Derivat davon, hergestellt.

Reaktionsfähige funktionelle Derivate von Verbindungen der Formel VI sind vorzugsweise Säurehalogenide, einfache oder gemischte Anhydride, z.B. das Säurechlorid, das Säureanhydrid $(R_2''CO)_2O$, oder ein gemischtes Anhydrid. Letzteres kann von einem Niederalkoxycarbonylhalogenid, z.B. Chlorameisensäureäthylester, oder von einem gehinderten Niederalkanoylhalogenid, z.B. vom Pivaloylchlorid, nach an sich bekannter Methode, hergestellt werden.

Die Kondensation von Verbindungen der Formeln V und VI, d.h. die Acylierung von Verbindungen der Formel V, verläuft entweder spontan, z.B. durch Erhitzen mit Ameisensäure, oder in Gegenwart von Kondensationsmitteln, wie disubstituierten Carbodiimiden, z.B. Dicyclohexylcarbodiimid.

Die Acylierung von Verbindungen der Formel V mit einem reaktionsfähigen funktionellen Derivat von einer Verbindung der Formel VI, z.B. mit Acetylchlorid oder Essigsäureanhydrid, wird vorzugsweise in Gegenwart einer organischen oder anorganischen Base, z.B. Kaliumcarbonat oder Triäthylamin, durchgeführt.

Die Amine der Formel V können z.B. aus entsprechend substituierten 2-(Cyan oder Hydroxyimino-niederalkyl)-pyridinen durch Reduktion, z.B. durch Hydrierung in Gegenwart eines Katalysators wie Palladium auf Kohle, oder durch Behandlung mit einem chemischen Reduktionsmittel, z.B. Boran oder Natriumcyanborhydrid, erhalten werden. Das Reduktionsmittel wird je nach der Art der anderen im Molekül vorhandenen funktionellen Gruppen gewählt. Die Verbindungen der Formel V können auch durch die Aminierung der entsprechend substituierten und reaktionsfähig veresterten 2-(Hydroxymethyl)-pyridine erhalten werden.

Erfindungsgemässe Verbindungen, worin A unsubstituiertes oder durch Niederalkyl substituiertes Äthenylen bedeutet, können hergestellt werden, indem man

b) eine Verbindung der Formel VII

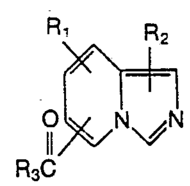

(VII),

worin $R_1$ und $R_2$ die oben angegebene Bedeutung haben und $R_3$ Wasserstoff oder Niederalkyl darstellt, mit einer Verbindung der Formel VIII

$$R_4\text{–}\underset{\displaystyle |}{\overset{\displaystyle R_5}{\text{CH}}}\text{–B–C}' \qquad \text{(VIII)},$$

worin $R_4$ Wasserstoff oder Niederalkyl bedeutet, $R_5$ Di-niederalkylphosphono oder Triarylphosphoranyl bedeutet, C′ Carboxy, Niederalkoxycarbonyl, Carbamoyl, mono- oder di-niederalkylsubstituiertes Carbamoyl, Cyan, Halogenmethyl, Niederalkanoyloxymethyl, veräthertes Hydroxymethyl, 5-Tetrazolyl, unsubstituiertes oder durch Niederalkyl substituiertes 4,5-Dihydro-2-oxazolyl, oder Hydroxycarbamoyl bedeutet und B wie oben unter Formel I definiert ist, kondensiert, und eine erhaltene Verbindung, worin sich C′ von C unterscheidet, in eine Verbindung der Formel I umwandelt und, wenn erwünscht, eine erhaltene Verbindung der Formel I in eine andere Verbindung der Erfindung umwandelt.

In den Ausgangsstoffen der Formel VIII bedeutet Di-niederalkylphosphono z.B. Diäthyl-phosphono und Triarylphosphoranyl z.B. Triphenylphosphoranyl.

Die Kondensation wird z.B. unter Bedingungen wie sie üblicherweise für Wittig-Reaktionen gewählt werden, z.B. wie in J. Am. Chem. Soc. 83, 1733 (1961) angegeben, nämlich unter Bildung eines Ylids, z.B. in Gegenwart einer starken Base wie etwa Natriumhydrid, in einem Lösungsmittel, z.B. Methylenchlorid oder Toluol, in einem Temperaturbereich von −20° bis +100°, vorzugsweise von −10° bis +50°, durchgeführt.

Bestimmte Verbindungen der Formel I, z.B. solche, worin A eine einfache Bindung bedeutet, B Niederalkylenphenylen-niederalkylen, Niederalkylenphenylen, Niederalkylen-(thio oder oxy)-niederalkylen, Niederalkylen-(thio oder oxy)-phenylen, Niederalkylenphenylen-niederalkenylen oder Niederalkadienylen bedeutet, können dadurch hergestellt werden, dass man

c) eine Verbindung der Formel IX

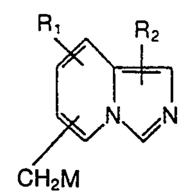

(IX),

worin M ein Alkalimetall bedeutet, $R_1$ und $R_2$ Wasserstoff oder Niederalkyl darstellen, mit einem reaktionsfähigen funktionellen Derivat einer Verbindung der Formel X

$$\text{HO} - \text{B}' - \text{C}' \qquad \text{(X)},$$

worin B′ Niederalkylenphenylen-niederalkylen, Niederalkylenphenylen, Niederalkylen-(thio oder oxy)-niederalkylen, Niederalkylen-(thio oder oxy)-phenylen, Niederalkylenphenylen-niederalkenylen oder Niederalkadienylen bedeutet, C′ Carboxy, Trialkoxymethyl, unsubstituiertes, mono- oder di-Niederalkyl-substituiertes Carbamoyl, Cyan, Niederalkanoyloxymethyl, veräthertes Hydroxymethyl, Halogenmethyl, unsubstituiertes oder durch Niederalkyl substituiertes 4,5-Dihydro-2-oxazolyl, 5-Tetrazolyl oder Hydroxycarbamoyl bedeutet, umsetzt und eine erhaltene Verbindung der Formel Ib

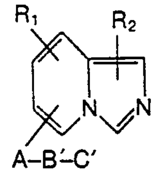

(Ib),

worin A, B′, C′, $R_1$ und $R_2$ die oben angegebene Bedeutung haben, und C′ sich von C unterscheidet, in eine Verbindung der Formel I umwandelt, und wenn erwünscht, eine erhaltene Verbindung der Formel I in eine andere Verbindung der Erfindung umwandelt.

Reaktionsfähige organometallische Verbindungen der Formel IX, worin M ein Alkalimetallatom bedeutet, können durch Metallierung von geeigneten methyl-substituierten Imidazo[1,5-a]pyridinen erhalten werden. So wird z.B. das gemäss J. Org. Chem. 40, 1210 (1975) hergestellte 5-Methylimidazo[1,5-a]pyridin mit einem reaktionsfähigen Metallierungsmittel, z.B. mit Butyllithium oder Lithium-diisopropylamid, in einem inerten Lösungsmittel, z.B. Tetrahydrofuran, bei einer Temperatur unter Zimmertemperatur, vorzugsweise bei ungefähr −50°, umgesetzt.

Die Kondensation eines Zwischenproduktes der Formel IX mit einem reaktionsfähigen funktionellen Derivat einer Verbindung der Formel X wird vorzugsweise in einem Temperaturbereich von ungefähr −75° bis +50° vorgenommen. Wenn C′ Carboxy, 5-Tetrazolyl, Hydroxycarbamoyl oder Mono-Niederalkyl-carbamoyl bedeutet, wird zuerst das geeignete Metallsalz, z.B. das Lithiumsalz des reaktionsfähigen funktionellen Derivats der entsprechenden Verbindung der Formel X hergestellt und dieses mit dem Zwischenprodukt der Formel IX umgesetzt.

Die in den vorher beschriebenen Verfahren verwendeten Begriffe haben die folgenden Bedeutungen:

In einem reaktionsfähigen funktionellen Derivat eines Alkohols der Formel X ist die Hydroxygruppe z.B. mit einer starken anorganischen Säure oder einer organischen Sulfonsäure, vor allem mit einer Halogenwasserstoffsäure, z.B. Chlorwasserstoff-, Bromwasserstoff- oder Jodwasserstoffsäure, einer aliphatischen oder aromatischen Sulfonsäure, z.B. Methansulfon- oder p-Toluolsulfonsäure, verestert. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Veräthertes Hydroxymethyl bedeutet vorzugsweise tertiäres Niederalkoxymethyl, niederes Alkoxy-alkoxymethyl, z.B. Methoxymethoxymethyl, 2-Oxa- oder 2-Thiacycloalkoxymethyl, insbesondere 2-Tetrahydropyranyloxymethyl.

Niederalkylendioxy ist vorzugsweise Äthylendioxy.

Halogenmethyl ist insbesondere Chlormethyl, kann jedoch auch Brommethyl oder Jodmethyl sein.

Niederalkanoyloxymethyl ist vorzugsweise Acetoxymethyl.

Ein Alkalimetall ist vorzugsweise Lithium, es kann aber auch Kalium oder Natrium sein.

Die Umwandlung einer zuerst erhaltenen Verbindung, worin sich C′ von C unterscheidet, in eine Verbindung der Formel I bzw. die fakultativen Umwandlungen eines erhaltenen Produkts der Formel I in eine andere Verbindung der Erfindung werden nach an sich bekannten chemischen Methoden durchgeführt.

Die Hydrolyse von Zwischenprodukten, in welchen C′ Trialkoxymethyl bedeutet, zu Verbindungen der Formel I, in welchen C Carboxy ist, wird vorzugsweise mit anorganischen Säuren, wie Halogenwasserstoff- oder Schwefelsäure, vorgenommen. Die Hydrolyse von Zwischenprodukten, in welchen C′ veräthertes Hydroxymethyl bedeutet, zu Verbindungen der Formel I, in welchen C Hydroxymethyl ist, wird vorzugsweise mit wässerigen Lösungen von anorganischen Säuren, z.B. einer Halogenwasserstoffsäure, durchgeführt.

Zwischenprodukte der Formel Ia oder Ib, in welchen C′ Halogenmethyl bedeutet, können vorzugsweise mit einem Metallcyanid, z.B. Kaliumcyanid, in an sich bekannter Weise umgesetzt werden. Man erhält dabei Verbindungen der Formel I, in welchen die Kette um ein Kohlenstoffatom verlängert ist und C für Cyan steht. Diese können ihrerseits nach an sich bekannten Methoden in Verbindungen der Formel I, worin C Carboxy, Alkoxycarbonyl oder Carbamoyl bedeutet, überführt werden.

So können Verbindungen der Formel I, worin C Cyan bedeutet (Nitrile), in Verbindungen der Formel I, in welchen C für Carboxy steht, durch Hydrolyse mit anorganischen Säuren, z.B. Halogenwasserstoffsäure, wie Chlorwasserstoffsäure oder Schwefelsäure, in wässerigen Lösungen oder vorzugsweise durch Hydrolyse mit wässerigen Alkalimetallhydroxiden, z.B. Kaliumhydroxid, vorzugsweise bei Rückflusstemperatur, umgewandelt werden.

Die Umwandlung der genannten Nitrile in Verbindungen der Formel I, worin C Niederalkoxycarbonyl bedeutet, wird vorzugsweise durch Behandlung mit einem Niederalkanol, z.B. wasserfreiem Äthanol, in Gegenwart einer starken Säure, z.B. Chlorwasserstoffsäure, vorzugsweise bei Rückflusstemperatur, und nachfolgende vorsichtige Hydrolyse mit Wasser durchgeführt.

Weiter wird die Umwandlung der genannten Nitrile in Verbindungen der Formel I, worin C Carbamoyl bedeutet, vorzugsweise durch Behandlung mit einem Alkalimetallhydroxid, z.B. verdünntem Natriumhydroxid, und Wasserstoffperoxid, vorzugsweise bei Raumtemperatur, durchgeführt.

Die genannten Nitrile können ferner in Verbindungen der Formel I umgewandelt werden, worin C 5-Tetrazolyl bedeutet, indem man sie mit einer Verbindung umsetzt, die geeignet ist, Stickstoffwasserstoffsäure in situ zu generieren, z.B. Natriumazid oder Ammoniumazid, vorzugsweise in einem polaren Lösungsmittel, z.B. Dimethylformamid, in einem Temperaturbereich von 75° bis 150°.

Ferner können die Zwischenprodukte der Formel Ia oder Ib, in welchen C′ Halomethyl, z.B. Chlormethyl ist, in Verbindungen der Formel I, worin C Carboxy ist und die Kettenlänge um zwei Kohlenstoffatome verlängert ist, wie folgt umgewandelt werden:

Zunächst werden sie durch Behandlung mit z.B. einem Malonsäure-di-niederalkylester, etwa Malonsäure-diäthylester, in Gegenwart einer Base, z.B. Kaliumcarbonat oder Natriumäthoxid, in einem Lösungsmittel, z.B. Dimethylformamid, vorzugsweise in einem Temperaturbereich zwischen 50 und 100°, in einen substituierten Malonsäure-di-niederalkylester überführt. Dieser wird vorzugsweise mit einer wässerigen Base, z.B. verdünntem Natriumhydroxid, zu der entsprechenden Malonsäure hydrolysiert, welche nach Standardmethoden, z.B. durch Erhitzen in Xylol, decarboxyliert werden kann. Man erhält eine Verbindung der Formel I, worin C Carboxy bedeutet. Ersetzt man den Malonsäure-di-niederalkylester durch einen Cyanessigsäure-niederalkylester, so erhält man die entsprechende Verbindung, worin C Cyan bedeutet.

Verbindungen der Erfindung, worin B einen Alkenylenrest bedeutet und eine terminale Doppelbindung aufweist, können auch aus Zwischenprodukten der Formel Ia oder Ib, worin C′ Halogenmethyl bedeutet, hergestellt werden. So werden z.B. diese Zwischenprodukte zuerst mit z.B. einem α-(Aryl oder Alkyl)thioessigsäure-niederalkylester, wie α-(Phenylthio)-essigsäure-äthylester, behandelt. Die Reaktion wird in Gegenwart einer starken Base, z.B. Natriumhydrid, durchgeführt. Die nachfolgende Oxidation des erhaltenen α-Arylthio- oder α-Alkylthio-substituierten Esters zu dem entsprechenden α-Arylsulfinyl- oder α-Alkylsulfinylester mit z.B. Natriumperjodat und anschliessend eine durch Hitze ausgelöste Eliminierung, z.B. durch Kochen in Xylol unter Rückfluss, ergibt eine Verbindung der allgemeinen Formel I (einen α,β-ungesättigten Ester), worin B z.B. Alkenylen bedeutet, C z.B. für Niederalkoxycarbonyl steht und die Länge der Kette um 2 Kohlenstoffatome verlängert ist. Ähnlich können Verbindungen der Formel Ia, worin C Halogenmethyl bedeutet, zuerst zu den entsprechenden Carboxaldehyden, z.B. mit Dimethylsulfoxid in Gegenwart von Triäthylamin und Silber-tetrafluorborat, umgewandelt werden. Die nachfolgende Wittig-Kondensation, z.B. mit (Triphenylphosphoranyliden)-essigsäureäthylester, ergibt ebenfalls die vorher genannten α,β- ungesättigten Ester. Führt man die Umsetzung in ähnlicher Weise z.B. mit 4-Phosphonocrotonsäuretriäthylester durch, so erhält man die entsprechenden Verbindungen der Formel I, in denen B Niederalkadienylen und C Niederalkoxycarbonyl bedeutet.

Verbindungen der Formel I, worin C Niederalkoxycarbonyl bedeutet, können mit Ammoniak, Mono- oder Di-niederalkylaminen, z.B. Methylamin oder Dimethylamin, Hydroxylamin oder 2-Hydroxyäthylamin, welches gegebenenfalls durch Niederalkyl substituiert ist, in einem inerten Lösungsmittel, z.B. Niederalkanol, wie Butanol, gegebenenfalls bei erhöhten Temperaturen, zu Verbindungen der Formel I, in welchen C unsubstituiertes, mono- oder di-(niederalkyl)-substituiertes Carbamoyl, Hydroxycarbamoyl oder gegebenenfalls niederalkyl-substituiertes Dihydro-2-oxazolyl bedeutet, amidiert werden.

Verbindungen der Formel I, in welchen C für unsubstituiertes Carbamoyl steht, können zu den entsprechenden Nitrilen in an sich bekannter Weise, z.B. durch Behandlung mit Phosphoroxychlorid oder Thionylchlorid, in einem inerten Lösungsmittel, z.B. Toluol, dehydratisiert werden.

Die Umwandlung von Verbindungen der Formel I, in welchen C Niederalkoxycarbonyl, Cyan, unsubstituiertes, mono- oder di-(niederalkyl)-substituiertes Carbamoyl oder gegebenenfalls niederalkyl-substituiertes Dihydro-2-oxazolyl bedeutet, zu Verbindungen der Formel I, in welchen C für Carboxy steht, wird vorzugsweise durch Hydrolyse mit anorganischen Säuren, z.B. mit Halogenwasserstoffsäuren oder Schwefelsäure, oder mit wässerigem Alkali, vorzugsweise mit Alkalimetallhydroxiden, z.B. Lithium- oder Natriumhydroxid, durchgeführt.

Verbindungen der Formel I, in welchen C Carboxy oder Niederalkoxycarbonyl bedeutet, können mit einfachen oder komplexen Leichtmetallhydriden, z.B. mit Lithiumaluminiumhydrid, Alan oder Diboran, zu Verbindungen der Formel I, in welchen C für Hydroxymethyl steht, reduziert werden. Die Alkohole können auch durch geeignete Solvolyse von Zwischenprodukten der Formel Ia oder Ib, worin C′ Halogenmethyl bedeutet, durch Behandlung z.B. mit einem Alkalimetallhydroxid, z.B. Lithium- oder Natriumhydroxid, erhalten werden.

Die vorher genannten Alkohole können ihrerseits in Verbindungen der Formel I, worin C Carboxy bedeutet, mit konventionellen Oxidationsmitteln, vorzugsweise mit Pyridin-dichromat in Dimethylformamid bei Raumtemperatur, umgewandelt werden.

Freie Carbonsäuren können mit Niederalkanolen, z.B. Äthanol, in Gegenwart einer starken Säure, z.B. Schwefelsäure, vorzugsweise bei erhöhter Temperatur, oder mit Diazo-niederalkanen, z.B. Diazomethan, in einem Lösungsmittel, z.B. Diäthyläther, vorzugsweise bei Raumtemperatur, zu den entsprechenden Estern, nämlich zu solchen Verbindungen der Formel I, worin C Niederalkoxycarbonyl bedeutet, verestert werden.

Weiter können die freien Carbonsäuren durch Behandlung eines ihrer reaktionsfähigen Derivate, z.B. eines Acylhalogenids, wie eines Säurechlorids, oder eines gemischten Anhydrids, z.B. eines solchen, das von einem Halogenkohlensäure-niederalkylester, z.B. Chlorameisensäureäthylester, abgeleitet ist, mit Ammoniak, Mono- oder Di-niederalkylaminen, Hydroxylamin oder gegebenenfalls niederalkyl-substituiertem 2-Hydroxyäthylamin, in einem inerten Lösungsmittel, z.B. Methylenchlorid, vorzugsweise in Gegenwart eines basischen Katalysators, z.B. Pyridin, in Verbindungen der Formel I, in welchen C für unsubstituiertes, mono- oder di-(niederalkyl)-substituiertes Carbamoyl, Hydroxycarbamoyl oder unsubstituiertes oder niederalkyl-substituiertes 4,5-Dihydro-2-oxazolyl steht, umgewandelt werden.

Verbindungen der Formel I, worin C Mononiederalkyl-carbamoyl bedeutet, können in Verbindungen der Formel I, in welchen C für Di-niederalkyl-carbamoyl steht, durch Behandlung der ersteren mit einer starken Base, z.B. Natriumhydrid, und dann mit einem Alkylierungsmittel, z.B. einem Niederalkylhalogenid, in einem inerten Lösungsmittel, z.B. Dimethylformamid, überführt werden.

Zwischenprodukte der Formel Ia, worin C′ Halogen bedeutet, welches mit einem Phenylrest der Gruppe B verbunden ist, können in Verbindungen der Formel I umgewandelt werden, worin B eine Phenylen-niederalkenylen-Einheit enthält und C Niederalkoxycarbonyl bedeutet, indem erstere mit einem $\alpha,\beta$-ungesättigten Ester, z.B. Methacrylsäuremethylester, in Gegenwart eines Palladiumsalzes, z.B. Palladiumacetat, und eines Triarylphosphins, z.B. Tri-o-tolylphosphin, behandelt werden.

Verbindungen der Formel I, worin B Niederalkylen-(thio oder oxy)-phenylen, Phenylen- (thio oder oxy)-niederalkylen oder Niederalkylen-(thio oder oxy)-niederalkylen bedeutet, können

d) durch die Umsetzung eines Zwischenproduktes der Formel Ia, worin B Niederalkylen oder Phenylen und C′ reaktionsfähiges, funktionell modifiziertes Hydroxymethyl, z.B. Halogenmethyl, bedeuten, mit einem Niederalkanol bzw. einem Niederalkanthiol oder einem Phenol bzw. einem Thiophenol, welche jeweils in geeigneter Weise durch C (oder vorübergehend geschützt als C′) substituiert sind, vorzugsweise in Gegenwart einer starken Base, z.B. Natriumhydroxid, erhalten werden.

Weiterhin können die Verbindungen der Formel I, die im vorigen Absatz bezeichnet sind, in ähnlicher Weise auch dadurch erhalten werden, dass man ein Zwischenprodukt der Formel Ia, worin B Niederalkylen oder Phenylen bedeutet, C′ Hydroxy oder Mercapto darstellt und $R_1$, $R_2$ und A die oben angegebene Bedeutung haben, mit einem reaktionsfähigen, funktionellen Derivat einer Verbindung der Formel X, z.B. einem Halogenderivat, worin B′ Niederalkylen bedeutet und C′ wie oben angegeben definiert ist, umsetzt. Falls erforderlich, wird das erhaltene Produkt noch in gleicher Weise weiterbehandelt, wie oben für die Freisetzung von Verbindungen der Formel I aus z.B. Verbindungen der Formel Ia beschrieben.

Verbindungen der Formel I oder geeignete Zwischenprodukte können in die entsprechenden 5,6,7,8-Tetrahydro-imidazo[1,5-a]pyridin-Verbindungen durch Reduktion mit Wasserstoff in Gegenwart von Hydrierungskatalysatoren, z.B. Palladium, und einer Säure, z.B. einer Mineralsäure, wie Chlorwasserstoffsäure, in einem inerten Lösungsmittel, z.B. Äthanol, umgewandelt werden.

Weiter können Verbindungen der Formel I, in welchen B z.B. Phenylenniederalkenylen bedeutet, durch katalytische Hydrierung, vorzugsweise unter neutralen Bedingungen, z.B. mit einem Palladium-Katalysator bei atmosphärischem Druck in einem inerten Lösungsmittel, z.B. Äthanol, in Verbindungen der Formel I, worin B z.B. für Phenylenniederalkylen steht, umgewandelt werden.

Überdies können Verbindungen der Formel I, worin $R_1$ und $R_2$ Wasserstoff bedeuten, in die entsprechenden Halogenderivate durch direkte Halogenierung mit Chlor, Brom oder Jod überführt werden.

Verbindungen der Formel I und solche Zwischenprodukte, worin $R_1$ Aryl-niederalkoxy, z.B. Benzyloxy, oder Niederalkoxy, z.B. Methoxy, ist, können in Verbindungen der Formel I umgewandelt werden, worin $R_1$ Hydroxy ist, indem erstere in an sich bekannter Weise hydriert oder hydrolysiert werden.

Die oben genannten Reaktionen können in gleicher Weise, wenn geeignet, auf die entsprechenden 5,6,7,8-Tetrahydro-imidazo[1,5-a]pyridin-Zwischenprodukte angewendet werden, wobei man die 5,6,7,8-Tetrahydroderivate der Verbindungen der Formel I enthält.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, und, wenn erforderlich, mit vorübergehendem Schutz von reaktiven funktionellen Gruppen, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im Vorstehenden als besonders wertvoll beschriebenen Verbindungen führen.

Die Erfindung betrifft auch die neuen Ausgangsstoffe und Verfahren zu ihrer Herstellung.

Je nach der Wahl von Ausgangsstoffen und Verfahren können die neuen Verbindungen in Form eines der möglichen Isomeren oder als Gemische von solchen vorliegen. So können sie z.B. je nach Anwesenheit einer Doppelbindung und nach der Anzahl der asymmetrischen Kohlenstoffatome als reine optische Isomeren, z.B. als Antipoden, oder als Gemische von Isomeren, z.B. als Racemate, Gemische von Diastereomeren, als Gemische von Racematen oder Gemische von geometrischen Isomeren vorliegen.

Erhaltene Gemische von Diastereomeren, Gemische von Racematen oder geometrischen Isomeren können auf der Basis der physikochemischen Unterschiede der Komponenten, in an sich bekannter Weise, in die reinen Isomeren, Diastereomeren, Racemate oder geometrischen Isomeren, z.B. durch Chromatographie und/oder fraktionierte Kristallisation, getrennt werden.

Erhaltene Racemate können weiter in die optischen Antipoden in an sich bekannter Weise, z.B. durch Umkristallisation aus optisch aktiven Lösungsmitteln, durch Mikroorganismen, oder durch Umsetzung des sauren Endprodukts mit einer optisch aktiven Base, welche mit der racemischen Säure Salze bildet, aufgetrennt werden. Diese Salze können z.B. aufgrund ihrer unterschiedlichen Löslichkeit in die Diastereomeren getrennt werden. Aus letzteren können die Antipoden durch Einwirkung von geeigneten Mitteln freigesetzt werden. Basische racemische Produkte können in analoger Weise durch Trennung ihrer diastereomeren Salze, z.B. durch fraktionierte Kristallisation der d- oder l-Tartrate, in die Antipoden aufgetrennt werden.

Vorzugsweise wird von den zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz vorzugsweise mit Säuren, die therapeutisch verwendbare Säureadditionssalze ergeben, oder mit Anionenaustauschern überführt werden. Erhaltene Salze können in die entsprechenden freien Basen z.B. durch Behandlung mit einer stärkeren Base, wie einem Metallhydroxid, Ammoniumhydroxid oder einem basischen Salz, z.B. einem Alkalimetallhydroxid oder -carbonat, oder einem Kationenaustauscher umgewandelt werden. Eine Verbindung der Formel I, in welcher C Carboxy, 5-Tetrazolyl oder Hydroxycarbamoyl bedeutet, kann auch in die entsprechenden Metall- oder Ammoniumsalze überführt werden. Diese oder andere Salze, z.B. die Pikrate, können auch bei der Reinigung von freien Basen verwendet werden. Die Basen werden in ihre Salze übergeführt, die Salze abgetrennt und die Basen aus den Salzen freigesetzt.

Infolge der engen Beziehung zwischen den neuen Verbindungen in freier Form und in Form ihrer Salze sind im Vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere, für die Kristallisation verwendete Lösungsmittel einschliessen.

Die erfindungsgemässen pharmazeutische Präparate sind solche, die sich für enterale, z.B. orale oder rektale, und parenterale Verabreichung an Säugern, inklusive Menschen, eignen. Die Präparate werden für die Behandlung oder Vorbeugung von Krankheiten, welche auf die Hemmung der Thromboxan-Synthetase reagieren, z.B. periphäre vaskuläre Krankheiten, verwendet. Diese Präparate enthalten eine wirksame Dosis einer pharmakologisch aktiven Verbindung der Formel I oder eines pharmazeutisch verwendbaren Salzes davon, allein oder in Kombination mit einem oder mehreren pharmazeutisch annehmbaren Trägermaterialien.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder ihren Salzen, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, und/oder Absorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservierungs-, Stabilisierungs-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren hergestellt und enthalten von 0,1% bis 75%, insbesondere von 1% bis 50%, des Aktivstoffes. Einzeldosen für Säuger mit einem Gewicht von ungefähr 50–70 kg können zwischen 10–200 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nichts anderes angegeben ist, wird das Eindampfen von Lösungsmitteln unter vermindertem Druck, z.B. zwischen 20 und 130 mbar, durchgeführt.

Beispiel 1:

Eine gerührte Suspension von 150 mg Natriumhydrid in 25 ml Toluol wird mit 550 mg 4-Diäthylphosphono-crotonsäureäthylester innerhalb 10 Minuten tropfenweise versetzt. Das Reaktionsgemisch wird durch Kühlung mit einem Eisbad auf 5° gehalten. Dann wird das Gemisch mit 300 mg 5-Formylimidazo[1,5-a]pyridin versetzt und bei Raumtemperatur 1 Stunde gerührt. Das Reaktionsgemisch wird in 100 ml Eiswasser gegossen und mit 2 × 100 ml Essigsäureäthylester extra-

hiert. Die Extrakte werden vereinigt, über Magnesiumsulfat getrocknet, filtriert und zur Trockne eingedampft. Der erhaltene ölige Rückstand wird durch Säulenchromatographie auf Kieselgel gereinigt und mit einem Gemisch von Diäthyläther-Essigsäureäthylester eluiert. Das Lösungsmittel wird unter vermindertem Druck abgedampft. Man erhält das 5-(4-Äthoxycarbonylbuta-1,3-dienyl)-imidazo[1,5-a]pyridin, welches bei 101–103° schmilzt.

Der Ausgangsstoff wird wie folgt hergestellt:

Eine Lösung von 18 g 3-Äthylthioimidazo[1,5-a]pyridin (Blatcher und Middlemiss, Tetrahedron Lett. 21, 2195 (1980)) in 200 ml Tetrahydrofuran wird bei −50° mit 80 ml einer 1,6-molaren Lösung von n-Butyllithium in Hexan innerhalb 30 Minuten tropfenweise versetzt. Nach beendeter Zugabe wird das Reaktionsgemisch bei −50° weitere 45 Minuten gerührt und dann innerhalb von 10 Minuten tropfenweise mit 10 ml Dimethylformamid versetzt. Man lässt das Reaktionsgemisch auf Raumtemperatur erwärmen und giesst es in 500 ml Eiswasser. Es wird mit 500 ml Diäthyläther extrahiert. Der Ätherextrakt wird über wasserfreiem Magnesiumsulfat getrocknet, filtriert und unter vermindertem Druck eingedampft. Der ölige Rückstand wird durch Säulenchromatographie auf Kieselgel gereinigt und mit einem Gemisch von Diäthyläther-Hexan (1:2) eluiert. Das Lösungsmittel wird abgedampft. Man erhält 5-Formyl-3-äthylthioimidazo[1,5-a]pyridin; Smp. 41–42°.

Eine Lösung von 20 g 5-Formyl-3-äthylthioimidazo[1,5-a]pyridin in 200 ml Isopropanol wird mit 15 g Raney-Nickel versetzt. Das Reaktionsgemisch wird gerührt und 16 Stunden unter Rückfluss gekocht. Der Katalysator wird durch Kieselgur abfiltriert. Das Filtrat wird unter vermindertem Druck eingedampft. Der erhaltene ölige Rückstand wird durch Säulenchromatographie auf Kieselgel gereinigt und mit einem Gemisch von Diäthyläther-Essigsäureäthylester (2:1) eluiert. Das Lösungsmittel wird unter vermindertem Druck abgedampft. Man erhält 5-Formylimidazo[1,5-a]pyridin, welches bei 138–140° schmilzt.

Beispiel 2:

Eine Lösung von 200 mg 5-(4-Äthoxycarbonylbuta-1,3-dienyl)-imidazo[1,5-a]pyridin in 20 ml Methanol wird mit 4 ml 1-n. Natriumhydroxidlösung versetzt. Das Reaktionsgemisch wird 18 Stunden bei Raumtemperatur gerührt. Das Methanol wird unter vermindertem Druck abgedampft, der Rückstand in 20 ml Wasser verdünnt und die Lösung mit Chlorwasserstoffsäure auf den pH-Wert 5 eingestellt. Der Niederschlag wird abgetrennt. Man erhält 5-(4-Carboxybuta-1,3-dienyl)-imidazo[1,5-a]pyridin, welches bei 243–345° schmilzt.

Beispiel 3:

Eine Lösung von 0,34 g 5-(p-Bromphenyl)-imidazo[1,5-a]pyridin, 2,7 mg Palladiumacetat, 7,5-mg Trio-o-tolylphosphin und 0,22 g Methacrylsäuremethylester in 3 ml Triäthylamin wird 7 Tage unter Rückfluss gekocht, dann mit 50 ml Methylenchlorid verdünnt und mit Wasser gewaschen. Das Methylenchlorid wird abgedampft und der Rückstand in 50 ml Äther aufgenommen, filtriert und mit 2 × 20 ml eiskalter 0,5-n. Salzsäure gewaschen. Die wässerige Phase wird auf pH 8 eingestellt und mit Methylenchlorid extrahiert. Der Extrakt wird über Magnesiumsulfat getrocknet und eingedampft. Durch präparative Dünnschichtchromatographie (Kieselgel, Äther) erhält man 5-[p-(2-Methoxycarbonylprop-2-enyl)-phenyl]-imidazo-[1,5-a]pyridin; Rf = 0,37; NMR ($CDCl_3$): 5,62 (s,1H), 6,32 (s,1H); IR ($CH_2Cl_2$): 1720 $cm^{-1}$.

Das Ausgangsmaterial wird wie folgt hergestellt:

Zu einer Lösung von 2,54 g 2-(p-Bromphenyl)-pyridin (hergestellt wie in J. Chem. Soc. 1940, 349,355 beschrieben) in 25 ml Essigsäure werden langsam 1,6 ml 40% Peressigsäure gegeben. Die Mischung wird 2 Stunden bei 80–85° gehalten, und dann überschüssige Persäure bei 25° mit gesättigter Natriumsulfitlösung zerstört. Die Essigsäure wird abgedampft, und der Rückstand in 30 ml Methylenchlorid aufgenommen, filtriert und das Methylenchlorid abgedampft. Das zurückbleibende 2-(p-Bromphenyl)pyridin-N-oxid wird mit 1,47 g Dimethylsulfat in 15 ml Toluol 2 Stunden bei 80–90° behandelt. Das Lösungsmittel wird abgedampft, und man erhält das Methylsulfat-Salz, das in 3,5 ml eiskaltem Wasser gelöst wird. Eine Lösung von 2,17 g Kaliumcyanid und 1,6 ml einer 1-n. Natriumhydroxid-Lösung in 3,5 ml eiskaltem Wasser wird so hinzugegeben, dass die Temperatur 0° nicht überschreitet. Man hält das Gemisch 16,5 Stunden bei 0° und extrahiert dann das Produkt mit 3 × 50 ml Methylenchlorid. Der Extrakt wird über Natriumsulfat getrocknet, das Lösungsmittel abgedampft, wobei man ein Öl erhält, das auf 45 g Kieselgel mit Äther als Elutionsmittel chromatographiert wird. Man erhält 2-(p-Bromphenyl)-6-cyanpyridin, das bei 76–78° schmilzt.

Zu einer unter Rückfluss kochenden Lösung von 1,6 g 2-(p-Bromphenyl)-6-cyanpyridin in 8 ml Tetrahydrofuran wird langsam eine Lösung von 0,92 ml Boran-dimethylsulfid in 6 ml Tetrahydrofuran hinzugegeben. Gleichzeitig wird Dimethylsulfid durch Destillation über eine 10 cm Vigreux-Kolonne entfernt. Nach dem Ende der Zugabe wird die Mischung noch 15 Minuten unter Rückfluss gekocht, dann auf 30° abgekühlt und 6 ml 6-n. Salzsäure hinzugegeben. Nachdem die Wasserstoffentwicklung aufgehört hat, wird das Gemisch 30 Minuten unter Rückfluss gekocht, dann auf 0° abgekühlt, mit festem Natriumcarbonat gesättigt und mit 4 × 50 ml Methylenchlorid extrahiert. Die Methylenchlorid-Lösung wird eingedampft und der Rückstand über 10,0 g Kieselgel chromatographiert, wobei ein 1:1-Methanol-Essigsäureäthylester-Gemisch als Elutionsmittel dient. Man erhält 2-Aminomethyl-6-(p-bromphenyl)-pyridin, das bei 71–75° schmilzt.

Eine Lösung von 0,4 g 2-Aminomethyl-6-(p-bromphenyl)-pyridin in 1,0 ml Ameisensäure wird 18 Stunden auf 90° erhitzt, dann auf 0° abgekühlt, mit gesättigter Ammoniaklösung basisch gemacht und mit 4 × 10 ml Methylenchlorid extrahiert. Die

vereinigten Extrakte werden getrocknet und eingedampft, wobei rohes 2-Formylamino-6-(p-bromphenyl)-pyridin, Smp. 115–119°, erhalten wird, das in 1,4 ml Toluol gelöst und mit 0,65 g Phosphoroxychlorid 18 Stunden lang auf 90° erhitzt wird. Das überschüssige Phosphoroxychlorid wird zusammen mit dem Toluol abgedampft, der Rückstand mit gesättigter Ammoniaklösung basisch gemacht und das Produkt mit 4 × 15 ml Methylenchlorid extrahiert. Nach Verdampfen des Lösungsmittels erhält man ein rohes Produkt, das mit Essigsäureäthylester als Elutionsmittel auf Kieselgel chromatographiert wird. Nach Abdampfen des Lösungsmittels erhält man 5-(p-Bromphenyl)-imidazo[1,5-a]pyridin, das bei 55–57° schmilzt.

Beispiel 4:

Eine Lösung von 20 mg 5-[p-(2-Methoxycarbonylprop-2-enyl)phenyl]imidazo[1,5-a]pyridin und 20 mg Natriummethoxid in 2 ml Methanol wird unter Stickstoff 1 Stunde bei 25° gerührt. Es werden 2 ml Wasser hinzugefügt, und die homogene Lösung 18 Stunden bei Raumtemperatur gerührt. Das Lösungsmittel wird abgedampft, der Rückstand in 5 ml Wasser gelöst und mit 3 ml Äther gewaschen. Die wässerige Lösung wird auf pH 5 eingestellt, mit 10 ml Methylenchlorid extrahiert, über Magnesiumsulfat getrocknet und im Vakuum eingedampft. Man erhält 5-[p-(2-Carboxyprop-2-enyl)-phenyl]imidazo[1,5-a]pyridin, Smp. 171–172°.

Beispiel 5:

Zu einer Lösung von Lithiumdiisopropylamid (hergestellt aus 3,8 ml n-Butyllithium in Hexan und 0,84 ml Diisopropylamin) in 10 ml Tetrahydrofuran wird tropfenweise innerhalb von 15 Minuten bei −70° 1,35 g 4-Diäthylphosphono-crotonsäureäthylester hinzugegeben. Nach Ende der Zugabe werden 6,0 g 5-(5-Formylpentyl)imidazo[1,5-a]pyridin, ebenfalls tropfenweise, hinzugefügt. Das Gemisch wird 15 Minuten bei Raumtemperatur gerührt. Es wird mit Methanol behandelt und das Lösungsmittel im Vakuum abgedampft, wobei man 5-(9-Äthoxycarbonyl-nona-6,8-dienyl)-imidazo[1,5-a]pyridin als Öl erhält.

Der Ausgangsstoff wird wie folgt hergestellt:

Eine Lösung von 50 g 5-Methylimidazo[1,5-a]pyridin [J. Org. Chem. 40, 1210 (1975)] in 625 ml Tetrahydrofuran wird auf −75° vorgekühlt und unter Stickstoff mit 175 ml 2,4-n. n-Butyllithium in Hexan versetzt, wobei man die Temperatur unter −53° hält. Die Lösung von 5-(Lithiomethyl)-imidazo[1,5-a]pyridin wird wieder auf −75° gekühlt und mit einer Lösung von 121,8 g 5-Brom-1,1,1-triäthoxypentan in 125 ml Tetrahydrofuran rasch versetzt, wobei die Temperatur auf −60° steigt. Man lässt das Reaktionsgemisch innerhalb 45 Minuten auf −4° erwärmen und dampft es praktisch bis zur Trockene ein. Der Rückstand wird mit 500 ml Diäthyläther und 240 ml 3-n. Salzsäure versetzt und die Phasen getrennt. Die ätherische Phase wird noch zweimal mit 60 ml 3-n. Salzsäure extrahiert. Die vereinigten wässerigen Extrakte werden mit 100 ml konz. Ammoniaklösung basisch gemacht und zweimal mit 200 ml Diäthyläther extrahiert. Der Ätherextrakt wird über Magnesiumsulfat getrocknet und zur Trockene eingedampft. Das erhaltene Öl wird im Hochvakuum destilliert. Man erhält 5-(5-Äthoxycarbonylpentyl)-imidazo[1,5-a]pyridin, welches bei 180–185°/0,16 mbar siedet.

Eine Suspension von 26 g 5-(5-Äthoxycarbonylpentyl)-imidazo[1,5-a]pyridin in 100 ml 1-n. wässeriger Natriumhydroxid-Lösung wird zwei Stunden auf dem Dampfbad erhitzt, mit 10 ml Äthanol versetzt und 45 Minuten weiter erhitzt. Das Reaktionsgemisch wird abgekühlt, mit 300 ml Äther gewaschen und die Lösung mit konz. Salzsäure auf den pH-Wert 5,5 eingestellt. Man filtriert die ausgefallenen Kristalle ab und erhält 5-(5-Carboxypentyl)-imidazo[1,5-a]pyridin, welches bei 144–147° schmilzt.

Eine auf −60° gekühlte Lösung von 4,9 g 5-(5-Methoxycarbonyl-pentyl)-imidazo[1,5-a]pyridin (welches durch Veresterung des oben beschriebenen 5-(5-Carboxypentyl)-imidazo[1,5-a]pyridins mit Diazomethan in Methylenchlorid erhalten wird) in 140 ml Methylenchlorid wird innerhalb von 20 Minuten mit 40 ml einer 1,75-m. Lösung von Di-isobutyl-aluminiumhydrid in Hexan tropfenweise versetzt. Dann wird das Reaktionsgemisch 20 Minuten bei −60° gerührt und nachfolgend mit 10 ml Methanol und 100 ml Wasser zur Unterbrechung der Reaktion versetzt. Das Gemisch wird bei Raumtemperatur 15 Minuten gerührt, die Methylenchlorid-Phase abgetrennt und das Lösungsmittel bei vermindertem Druck abgedampft. Man erhält 5-(5-Formylpentyl)-imidazo[1,5-a]pyridin als Öl. NMR ($CDCl_3$): 9,7 (m, 1H); IR (Methylenchlorid): 1710 $cm^{-1}$.

Beispiel 6:

Zu einer Lösung von 6 g 5-(9-Äthoxycarbonylnona-6,8-dienyl)imidazo[1,5-a]pyridin in 30 ml Methanol werden 6 ml 1-n. Natriumhydroxid-Lösung hinzugegeben. Das Gemisch wird 3 Stunden bei Raumtemperatur gerührt. Das Methanol wird im Vakuum abgedampft und der Rückstand mit 15 ml Wasser verdünnt. Nach Einstellung der Lösung auf pH 7 mit 1-n. Salzsäure wird sie mit 2 × 150 ml Essigsäureäthylester extrahiert. Die vereinigten Extrakte werden über Magnesiumsulfat getrocknet, filtriert und das Lösungsmittel im Vakuum verdampft, wobei man 5-(9-Carboxynona-6,8-dienyl)-imidazo[1,5-a]pyridin erhält, das bei 120–122° schmilzt.

Beispiel 7:

Eine Lösung von 2-(p-Äthoxycarbonylphenyl)-6-formylaminomethylpyridin (9,89 g) und Phosphoroxychlorid (11,15 g) in 26 ml Toluol wird 15 Stunden auf 90° erhitzt. Das Lösungsmittel wird verdampft und der Rückstand in 50 ml Methylenchlorid aufgenommen, auf 0° abgekühlt und mit einem Überschuss eiskalter gesättigter Ammoniaklösung basisch gemacht. Die organische Phase wird abgetrennt, getrocknet und das Lösungsmittel verdampft. Der Rückstand wird auf 100 g Kieselgel mit Essigsäureäthylester als Elutionsmittel chromatographiert, wobei man nach Kristallisation aus Essigsäureäthylester 5-(p-Äthoxycarbonylphenyl)-imidazo[1,5-a]pyridin vom Smp. 118–119° erhält.

Der Ausgangsstoff wird wie folgt hergestellt:

Peressigsäure (40%, 8,9 ml) wird tropfenweise zu 14,08 g 2-(p-Äthoxycarbonylphenyl)-pyridin gegeben, so dass das Reaktionsgemisch zwischen 80–85° gehalten wird. Nach beendeter Zugabe wird das Reaktionsgemisch 3 Stunden auf 90° erhitzt, dann lässt man auf Raumtemperatur abkühlen, und überschüssige Peressigsäure wird mit wässeriger Natriumsulfitlösung zerstört. Das Lösungsmittel wird abgedampft, der Rückstand in Methylenchlorid aufgenommen und durch Celit filtriert. Nach Eindampfen der Lösung erhält man 2-(p-Äthoxycarbonylphenyl)-pyridin-N-oxid, das mit 8,66 g Dimethylsulfat in 62 ml Toluol 3 Stunden bei 90° behandelt wird. Das Lösungsmittel wird abgedampft und der Rückstand in einer eiskalten Mischung aus 8 ml Wasser und 9,3 ml 1-n. Natriumhydroxidlösung aufgenommen. Es wird eine Lösung von 13,64 g Kaliumcyanid in 10 ml Wasser langsam hinzugegeben und das Reaktionsgemisch 24 Stunden bei 0° gehalten. Es wird mit 2 × 30 ml Methylenchlorid extrahiert, der Extrakt über Natriumsulfat getrocknet und das Lösungsmittel abgedampft. Man erhält 2-(p-Äthoxycarbonylphenyl)-6-cyanpyridin; IR ($CH_2Cl_2$) 2200 $cm^{-1}$.

2-(p-Äthoxycarbonylphenyl)-6-cyanpyridin (16,23 g) wird bei atmosphärischem Druck in 254 ml Methanol mit 12,9 ml konz. Salzsäure und 2,63 g 10% Palladium-auf-Kohle hydriert, bis 2 Mol Wasserstoff aufgenommen sind. 6,9 g Natriummethoxid werden hinzugegeben und der Katalysator abfiltriert. Das Lösungsmittel wird verdampft, der Rückstand in 20 ml Methylenchlorid gelöst und Salze durch Filtration entfernt. Nach Abdampfen des Lösungsmittels erhält man einen Feststoff, der aus Chloroform kristallisiert wird. Es handelt sich um 6-Aminomethyl-2-(p-äthoxycarbonylphenyl)pyridin, Smp. 141–143°.

Eine Lösung von 6-Aminomethyl-2-(p-äthoxycarbonylphenyl)-pyridin (10,76 g) in 10 ml Ameisensäure wird 15 Stunden auf 90° erhitzt. Das Gemisch wird auf 0° abgekühlt, mit überschüssiger gesättigter Ammoniaklösung basisch gemacht und mit Chloroform extrahiert (4 × 30 ml). Die organischen Extrakte werden getrocknet, das Lösungsmittel verdampft, und man erhält 2-(p-Äthoxycarbonylphenyl)-6-formylaminomethylpyridin, das aus Toluol kristallisiert wird; Smp. 119,5–120,5°

Beispiel 8:

Eine Lösung von 5-(p-Äthoxycarbonylphenyl)-imidazo[1,5-a]pyridin (1,0 g) in 10 ml Äthanol und 10 ml 1-n. Natriumhydroxidlösung wird 3 Stunden unter Rückfluss gekocht. Das Äthanol wird abgedampft, die wässerige Phase mit Äther gewaschen, auf pH 6 eingestellt und der Feststoff, der 5-(p-Carboxyphenyl)-imidazo-[1,5-a]pyridin entspricht, gesammelt.

Beispiel 9:

1,0 g 5-(p-Äthoxycarbonylphenyl)-imidazo[1,5-a]pyridin werden in 26 ml Methylenchlorid bei −78° unter Stickstoff gelöst und 6,6 ml Diisobutylaluminiumhydrid in Toluol (11,4 mMol) tropfenweise hinzugefügt. Nach 1 Stunde Rühren werden 1,5 ml Methanol zugegeben, das Kühlbad entfernt und 15 ml Wasser hinzugefügt. Die Salze werden abfiltriert, die organische Phase über Natriumsulfat getrocknet und das Lösungsmittel verdampft, wobei man 5-(p-Hydroxymethylphenyl)-imidazo[1,5-a]pyridin, Smp. 137–138°, erhält.

Beispiel 10:

Eine Lösung von 0,52 g 5-(p-Hydroxymethylphenyl)-imidazo[1,5-a]pyridin in 10 ml Methylenchlorid wird mit 5,2 g aktiviertem Mangandioxid 24 Stunden unter Rückfluss gekocht. Es werden nochmals 5,2 g Mangandioxid hinzugegeben und die Reaktion weitere 6 Stunden unter Rückfluss gehalten. Man filtriert, verdampft das Lösungsmittel und erhält 5-(p-Formylphenyl)-imidazo[1,5-a]pyridin, Smp. 144–146°.

Beispiel 11:

a) 0,61 g 2-Diäthylphosphono-propionsäureäthylester werden zu einer Lösung von Lithiumdiisopropylamid (aus 0,32 g Diisopropylamin und 1,7 ml 1,64-m. n-Butyllithium) in 20 ml Tetrahydrofuran bei 0° unter Stickstoff gegeben. Es wird 10 Minuten gerührt, und dann eine Lösung von 0,50 g 5-(p-Formylphenyl)-imidazo[1,5-a]pyridin in 5 ml Tetrahydrofuran tropfenweise hinzugegeben. Das Reaktionsgemisch wird 2 Stunden bei 0° gerührt und mit 25 ml Wasser versetzt. Die Phasen werden getrennt, und die wässerige Phase wird mit 2 × 10 ml Essigsäureäthylester extrahiert. Die vereinigten organischen Extrakte werden über Natriumsulfat getrocknet, filtriert, und das Lösungsmittel abgedampft, wobei man ein Öl erhält, das auf Kieselgel mit Essigsäureäthylester als Elutionsmittel chromatographiert wird. Man erhält 5-[p-(2-Äthoxycarbonylprop-1-enyl)phenyl]imidazo[1,5-a]pyridin; IR ($CH_2Cl_2$) 1700 $cm^{-1}$.

b) 5-[p-(2-Äthoxycarbonyläth-1-enyl)phenyl]-imidazo[1,5-a]pyridin kann in ähnlicher Weise aus 2-Diäthylphosphono-essigsäureäthylester und 5-(p-Formylphenyl)-imidazo[1,5-a]pyridin erhalten werden.

Beispiel 12:

a) Eine Lösung von 0,60 g 5-[p-(2-Äthoxycarbonylprop-1-enyl)phenyl]-imidazo[1,5-a]pyridin in 2 ml Äthanol und 6,2 ml 1-n. Natriumhydroxid-Lösung werden 3 Stunden unter Rückfluss gekocht, dann wird abgekühlt und das Gemisch eingedampft. Zum Rückstand werden 5 ml Wasser und 1 ml Essigsäureäthylester gegeben und die Phasen getrennt. Die wässerige Phase wird mit konz. Salzsäure angesäuert (pH = 2) und der erhaltene Feststoff abfiltriert, der 5-[p-(2-Carboxyprop-1-enyl)phenyl]-imidazo[1,5-a]pyridin-Hydrochlorid, Smp. 280–282°, entspricht.

b) 5-[p-(2-Carboxyäth-1-enyl)phenyl]-imidazo-[1,5-a]pyridin kann in ähnlicher Weise durch Hydrolyse des 5-[p-(2-Äthoxycarbonyläth-1-enyl)-phenyl]-imidazo[1,5-a]pyridins erhalten werden.

Beispiel 13:

2,66 g 5-(p-Carboxyphenäthyl)-imidazo[1,5-a]pyridin werden in 100 ml trockenem Tetrahydrofuran bei 0° gelöst, und 20 ml eines 1-m. Boran-Tetrahydrofuran-Komplexes in Tetrahydrofuran (20 ml) tropfenweise hinzugefügt. Das Reaktionsgemisch wird 2 Stunden bei Raumtemperatur gerührt, und 20 ml Essigsäure und 20 ml Methanol werden vorsichtig hinzugegeben. Das Gemisch wird 1 Stunde unter Rückfluss gekocht, dann abgekühlt und eingedampft. Zum erhaltenen Öl wird Äther und 1-n. Schwefelsäure hinzugegeben. Die wässerige Phase wird abgetrennt, auf pH 8 eingestellt und mit Essigsäureäthylester extrahiert. Der Extrakt wird über Natriumsulfat getrocknet und das Lösungsmittel eingedampft, wobei man 5-[p-(Hydroxymethyl)phenäthyl]-imidazo[1,5-a]pyridin erhält.

Beispiel 14:

11,18 g Kaliumcyanid und 1,0 g Dibenzo-18-Krone-6-Äther werden unter Stickstoff zu einer Lösung von 9,5 g 5-[p-(Chlormethyl)phenäthyl]-imidazo[1,5-a]pyridin in 300 ml trockenem Acetonitril gegeben. Das Gemisch wird 24 Stunden unter Rückfluss gekocht, das Lösungsmittel abgedampft und der Rückstand mit Methylenchlorid und Wasser versetzt. Die organische Phase wird abgetrennt, getrocknet und das Lösungsmittel abgedampft, wobei man 5-[p-(Cyanmethyl)phenäthyl]-imidazo[1,5-a]pyridin erhält.

Der Ausgangsstoff wird wie folgt hergestellt:

5-[p-(Hydroxymethyl)phenäthyl]-imidazo[1,5-a]pyridin (2,52 g) wird in 20 ml Thionylchlorid 2 Stunden unter Rückfluss gekocht. Überschüssiges Thionylchlorid wird abgedampft und der Rückstand mit Essigsäureäthylester und verdünnter Natriumbicarbonat-Lösung versetzt. Die organische Phase wird über Natriumsulfat getrocknet, das Lösungsmittel verdampft, und man erhält 5-[p-(Chlormethyl)phenäthyl]-imidazo[1,5-a]pyridin.

Beispiel 15:

Eine Lösung von 1,0 g 5-[p-(Cyanomethyl)-phenäthyl]-imidazo[1,5-a]pyridin in 2-n. Natriumhydroxid-Lösung (30 ml) wird 15 Stunden unter Rückfluss gekocht und dann mit konz. Schwefelsäure angesäuert. Man filtriert das ausgefallene 5-[p-(Carboxymethyl)-phenäthyl]-imidazo[1,5-a]pyridin ab.

Beispiel 16:

2,52 g 5-[p-(Hydroxymethyl)phenäthyl]-imidazo-[1,5-a]pyridin und 25,2 g aktiviertes Mangandioxid werden 24 Stunden in 100 ml Methylenchlorid unter Rückfluss gekocht. Das Gemisch wird filtriert und das Lösungsmittel abgedampft, wobei man 5-(p-Formylphenäthyl)-imidazo[1,5-a]pyridin erhält.

Beispiel 17:

0,61 g 2-Diäthylphosphono-propionsäureäthylester werden zu einer Lösung von Lithiumdiisopropylamid (aus 0,32 g Diisopropylamin und 1,7 ml einer 1,64-m. n-Butyllithium-Lösung in Hexan hergestellt) in 20 ml trockenem Tetrahydrofuran unter Stickstoff bei 0° gegeben. Man rührt 15 Minuten bei 0°, gibt dann eine Lösung von 0,55 g 5-(p-Formylphenäthyl)-imidazo[1,5-a]pyridin in 5 ml Tetrahydrofuran hinzu und rührt das Reaktionsgemisch zunächst 1 Stunde bei 0°, dann 1 Stunde bei 25° und gibt schliesslich 25 ml Wasser hinzu. Man trennt die organische Phase ab, trocknet sie und dampft das Lösungsmittel ab. Das erhaltene Öl wird in Essigsäureäthylester gelöst und auf eine Kieselgel-Säule gegeben, um überschüssigen 2-Diäthylphosphono-propionsäureäthylester zu entfernen, und man erhält 5-[p-(2-Äthoxycarbonylprop-1-enyl)phenäthyl]-imidazo[1,5-a]pyridin.

Beispiel 18:

0,33 g 5-[p-(2-Äthoxycarbonylprop-1-enyl)phenäthyl]-imidazo[1,5-a]pyridin in 10 ml einer 0,5-n. Natriumhydroxid-Lösung werden 3 Stunden unter Rückfluss gekocht; dann kühlt man die Lösung und extrahiert sie mit Äther. Die wässerige Phase wird auf pH 6 eingestellt und der erhaltene Feststoff abfiltriert, der 5-[p-(2-Carboxyprop-1-enyl)-phenäthyl]-imidazo[1,5-a]pyridin entspricht.

Beispiel 19:

2,19 g Mercaptoessigsäureäthylester werden langsam zu Natriumhydrid (0,87 g, 50% Dispersion in Mineralöl) in 30 ml trockenem Dimethylformamid bei 5° unter Stickstoff gegeben. Das Kühlbad wird entfernt, man rührt 30 Minuten bei Raumtemperatur und gibt dann eine Lösung von 3,47 g 5-(4-Chlorbutyl)-imidazo[1,5-a]pyridin in 15 ml Dimethylformamid hinzu. Nach 15 Stunden wird das Gemisch in 150 ml Wasser gegossen, angesäuert (pH = 2) und mit 100 ml Äther verdünnt. Die wässerige Phase wird abgetrennt, auf pH 8 eingestellt und mit Methylenchlorid extrahiert. Der Extrakt wird getrocknet, das Lösungsmittel abgedampft, und man erhält 5-[4-(Äthoxycarbonylmethylthio)butyl]-imidazo[1,5-a]pyridin; IR 1730, 1640 $cm^{-1}$.

Beispiel 20:

4,4 g 5-[4-(Äthoxycarbonylmethylthio)butyl]-imidazo[1,5-a]pyridin, gelöst in 30 ml Äthanol, und 61 ml einer 1-n. Natriumhydroxid-Lösung werden 3 Stunden unter Rückfluss gekocht. Das Äthanol wird abgedampft, die wässerige Phase mit Essigsäureäthylester (20 ml) extrahiert und der pH-Wert auf 5 eingestellt. Das erhaltene Öl wird aus Äthanol kristallisiert, wobei man 5-[4-(Carboxymethylthio)butyl]-imidazo[1,5-a]pyridin, Smp. 124–126°, erhält.

Beispiel 21:

Eine Lösung von 5,20 g 5-(4-Chlorbutyl)imidazo[1,5-a]pyridin in 80 ml Dimethylformamid wird zusammen mit dem Natriumsalz des p-Hydroxybenzoesäureäthylesters, das aus 4,55 g p-Hydroxybenzoesäureäthylester und 1,31 g 50% Natriumhydrid-Suspension erhältlich ist, 15 Stunden

auf 50° erhitzt. Man lässt abkühlen, giesst das Reaktionsgemisch auf 100 g Eis, säuert es an und extrahiert es mit 50 ml Äther. Die wässerige Phase wird auf pH 8 eingestellt und mit 3 × 100 ml Äther extrahiert. Die vereinigten Extrakte werden getrocknet, das Lösungsmittel abgedampft, und man erhält 5-[4-(p-Äthoxycarbonylphenoxy)butyl]-imidazo[1,5-a]pyridin vom Smp. 85–87° (kristallisiert aus Äther).

Das Ausgangsmaterial wird wie folgt hergestellt:

Eine Lösung von 27 g 1-Brom-3-chlorpropan in 20 ml trockenem Tetrahydrofuran wird zu einer Lösung von 5-(Lithiomethyl)imidazo[1,5-a]pyridin, hergestellt aus 22 g 5-Methyl-imidazo[1,5-a]pyridin und 90 ml einer 2,3-n. Lösung von n-Butyllithium in Hexan, in Tetrahydrofuran bei einer Temperatur von weniger als −50° gegeben, wobei die Temperatur unter −50° bleiben muss. Das Reaktionsgemisch wird 2 bis 3 Stunden bei −50° gerührt, man lässt auf Raumtemperatur erwärmen, rührt über Nacht und dampft zur Trockene ein.

Der Rückstand wird in Methylenchlorid aufgenommen, mit Wasser gewaschen, über Magnesiumsulfat getrocknet und das Lösungsmittel abgedampft, wobei man 5-(4-Chlorbutyl)imidazo[1,5-a]pyridin erhält, das ohne weitere Reinigung weiterverwendet wird.

Beispiel 22:

Eine Lösung von 6,5 g rohem 5-[4-(p-Äthoxycarbonylphenoxy)butyl]-imidazo[1,5-a]pyridin in 30 ml Äthanol und 30 ml einer 1-n. Natriumhydroxid-Lösung werden 3 Stunden unter Rückfluss erhitzt. Das Äthanol wird abgedampft, die wässerige Phase mit Äther extrahiert, auf pH 2 angesäuert und der erhaltene Feststoff abfiltriert, der dem 5-[4-(p-Carboxyphenoxy)butyl]-imidazo[1,5-a]-pyridin-Hydrochlorid, Smp. 266–268° (Zersetzung), entspricht.

Beispiel 23:

2,64 g 5-[p-(2-Carboxyäth-1-enyl)phenyl]-imidazo[1,5-a]pyridin werden in 50 ml trockenem Äthanol gelöst und bei einem Druck von 3 atm mit 0,5 g 5% Palladium-auf-Kohle hydriert, bis 1 Mol Wasserstoff aufgenommen ist. Man filtriert, dampft das Lösungsmittel ab und erhält 5-[p-(2-Carboxyäthyl)phenyl]imidazo[1,5-a]pyridin.

Beispiel 24:

a) Eine Lösung von 1,46 g 5-Formylimidazo[1,5-a]pyridin in 15 ml trockenem Dimethylsulfoxid wird zu einer Lösung von 4,4 g Natrium-5-(triphenylphosphoranyl)valerat in 50 ml trockenem Dimethylsulfoxid bei Raumtemperatur gegeben [Helv. Chim. Acta 63, 1430 (1980)]. Das Gemisch wird 3 Stunden gerührt, mit Wasser hydrolysiert und mit verdünnter Schwefelsäure neutralisiert. Man extrahiert mit Essigsäureäthylester, chromatographiert über Kieselgel und erhält 5-(5-Carboxypent-1-enyl)-imidazo[1,5-a]pyridin.

b) In ähnlicher Weise kann 5-[2-(p-Carboxyphenyl)äthen-1-yl]-imidazo[1,5-a]pyridin aus Natrium-p-(triphenylphosphoranylmethyl)-benzoat und 5-Formylimidazo[1,5-a]pyridin hergestellt werden.

Beispiel 25:

2,0 g 5-[2-(p-Carboxyphenyl)äthen-1-yl]-imidazo[1,5-a]pyridin in 50 ml Äthanol werden mit 1,0 g 5% Palladium-auf-Kohle bei 3 atm hydriert, bis 1 Mol Wasserstoff aufgenommen ist. Man filtriert durch Celit, verdampft das Lösungsmittel und erhält 5-[2-(p-Carboxyphenäthyl)]imidazo[1,5-a]pyridin.

Beispiel 26:

Eine Lösung von 2,36 g 5-[2-(p-Hydroxyphenyl)äthen-1-yl]-imidazo[1,5-a]pyridin in 10 ml Dimethylformamid wird zu 0,5 g Natriumhydrid in 30 ml trockenem Dimethylformamid bei 0° unter Stickstoff gegeben. Man lässt auf Raumtemperatur erwärmen, gibt 1,67 g Bromessigsäureäthylester hinzu und erhitzt das Reaktionsgemisch 5 Stunden auf 60°. 200 ml Wasser werden hinzugegeben und die Lösung mit Essigsäureäthylester extrahiert. Die organische Phase wird mit Wasser gewaschen, über Natriumsulfat getrocknet, das Lösungsmittel abgedampft, und man erhält 5-[2-(p-[Äthoxycarbonylmethoxy]phenyl)äthen-1-yl]-imidazo[1,5-a]pyridin.

Der Ausgangsstoff wird wie folgt hergestellt:

4,29 g Imidazo[1,5-a]pyridin-5-methyltriphenylphosphoniumchlorid, das aus äquimolaren Mengen von Triphenylphosphin und 5-Chlormethylimidazo[1,5-a]pyridin hergestellt wird, werden zu 0,5 g Natriumhydrid (50% Mineralöl-Dispersion) in 100 ml trockenem Tetrahydrofuran bei 0° unter Stickstoff gegeben. Man hält 1 Stunde bei 0° und gibt dann eine Lösung 0,61 g p-Hydroxybenzaldehyd in 15 ml trockenem Tetrahydrofuran hinzu. Man lässt das Gemisch auf Raumtemperatur erwärmen, kocht 1 Stunde unter Rückfluss, lässt abkühlen und säuert mit verdünnter Schwefelsäure an. Die wässerige Phase wird mit Essigsäureäthylester gewaschen, mit 50% Natriumhydroxid-Lösung auf pH 7 eingestellt und mit Essigsäureäthylester extrahiert. Die organische Phase wird über Natriumsulfat getrocknet, das Lösungsmittel abgedampft und man erhält 5-[2-(p-Hydroxyphenyl)äthen-1-yl]-imidazo[1,5-a]pyridin.

5-Chlormethylimidazo[1,5-a]pyridin wird aus 5-Formylimidazo[1,5-a]pyridin hergestellt, indem letzteres mit Natriumborhydrid zu 5-Hydroxymethylimidazo[1,5-a]pyridin reduziert und nachfolgend mit Thionylchlorid behandelt wird.

Beispiel 27:

Eine Lösung von 3,2 g 5-[2-(p-[Äthoxycarbonylmethoxy]phenyl)äthen-1-yl]imidazo[1,5-a]pyridin in 30 ml Äthanol wird mit 20 ml einer 1-n. Natriumhydroxid-Lösung 3 Stunden unter Rückfluss gekocht. Das Äthanol wird abgedampft und die wässerige Phase auf pH 6 eingestellt. Durch Filtration gewinnt man die substituierte Phenoxyessigsäure 5-[2-(p-[Carboxymethoxy]phenyl)äthen-1-yl]-imidazo[1,5-a]pyridin.

Beispiel 28:

Eine Lösung von 6,0 g 5-[p-(Cyanmethyl)phenäthyl]-imidazo[1,5-a]pyridin in 16 ml trockenem Dimethylformamid wird mit 2,15 g Natriumazid, 0,2 g Lithiumchlorid und 1,80 g Ammoniumchlorid 15 Stunden auf 120° erhitzt. Man kühlt ab, filtriert, verdampft das Lösungsmittel und löst den Rückstand in 50 ml Wasser. Es wird mit 25 ml Essigsäureäthylester extrahiert, dann die wässerige Phase mit konz. Schwefelsäure auf pH 5 gebracht. Der ausgefallene Feststoff wird filtriert, mit Wasser gewaschen, getrocknet, und man erhält 5-[p-(5-Tetrazolylmethyl)phenäthyl]-imidazo[1,5-a]pyridin.

Beispiel 29:

Die folgenden Verbindungen der Formel I, a–e, worin A eine einfache Bindung und $R_2$ Wasserstoff ist, werden in analoger Weise wie in den vorhergehenden Beispielen beschrieben hergestellt.

| Verbindung | B | C | $R_1$ |
|---|---|---|---|
| a | $5-(CH_2)_3OCH_2CH_2$ | COOH | H |
| b | $5-(CH_2)_3SCH_2CH_2$ | COOH | $6-OCH_2C_6H_5$ |
| c | $5-(CH_2)_2p-C_6H_4-CH_2$ | COOH | H |
| d | $5-(CH_2)_2O-p-C_6H_4$ | COOH | H |
| e | $7-(CH_2)_3O-p-C_6H_4$ | COOH | H |

Zur Herstellung der Verbindungen a, b und d werden als Ausgangsmaterialien Halogen- bzw. Bromverbindungen benötigt, die mit 5-Methylimidazo[1,5-a]pyridin umgesetzt werden. Diese sind im US-Patent 3 984 459, in Chem. Abstracts 83, 166177b und im US-Patent 2 790 825 beschrieben.

Der Ausgangsstoff 6-Benzyloxy-5-methylimidazo[1,5-a]pyridin für Verbindung b wird wie folgt hergestellt:

Eine Lösung von 8,54 g 3-Benzyloxy-6-hydroxymethyl-2-methylpyridin in 53 ml Thionylchlorid wird 2 Stunden unter Rückfluss gekocht und dann das Thionylchlorid durch Destillation entfernt. Der Rückstand wird auf 50 g Eis gegossen, mit gesättigter Natriumbicarbonat-Lösung basisch gemacht und mit 3 × 50 ml Essigsäureäthylester extrahiert. Die organischen Extrakte werden über Natriumsulfat getrocknet, das Lösungsmittel abgedampft, und man erhält ein gelbes Öl, das als 3-Benzyloxy-6-chlormethyl-2-methylpyridin identifiziert wird; NMR ($CDCl_3$): 2,47 (3H), 4,54 (2H), 4,97 (2H).

Eine Lösung von 8,02 g 3-Benzyloxy-6-chlormethyl-2-methylpyridin und 5,98 g Natriumazid in 400 ml Äthanol wird 4 Stunden auf 80° erhitzt. Man lässt abkühlen, filtriert, verdampft das Lösungsmittel und erhält einen Rückstand, zu dem 150 ml 0,5-n. Natriumhydroxid-Lösung und 150 ml Äther gegeben werden. Die organische Phase wird über Natriumsulfat getrocknet, das Lösungsmittel abgedampft und man erhält 6-Azidomethyl-3-benzyloxy-2-methylpyridin; NMR ($CDCl_3$): 2,47 (3H), 4,27 (2H), 4,95 (2H).

1,27 g Lithiumaluminiumhydrid werden zu einer Lösung von 7,18 g 6-Azidomethyl-3-benzyloxy-2-methylpyridin in 120 ml trockenem Äther bei Raumtemperatur gegeben. Man rührt 45 Minuten, und gibt dann nacheinander zunächst 1,3 ml Wasser, dann 1,3 ml einer 15% Natriumhydroxid-Lösung und schliesslich 3,8 ml Wasser hinzu. Die Salze werden abfiltriert, das Filtrat eingedampft, und man erhält 6-Aminomethyl-3-benzyloxy-2-methylpyridin; NMR ($CDCl_3$): 1,67 (2H), 2,47 (3H), 3,8 (2H), 4,95 (2H).

Eine Lösung von 6,31 g 6-Aminomethyl-3-benzyloxy-2-methylpyridin in 8,7 ml Ameisensäure wird 15 Stunden auf 90° erhitzt. Man lässt abkühlen, macht mit eiskalter wässeriger Ammoniak-Lösung basisch und extrahiert mit 3 × 25 ml Chloroform. Die vereinigten Extrakte werden über Natriumsulfat getrocknet und das Lösungsmittel abgedampft. Man kristallisiert aus Äther und erhält 3-Benzyloxy-6-formylaminomethyl-2-methylpyridin vom Smp. 67–68°.

Eine Lösung von 5,14 g 3-Benzyloxy-6-formylaminomethyl-2-methylpyridin und 4,0 ml Phosphoroxychlorid in 18 ml Toluol wird 15 Stunden auf 90° erhitzt. Das Lösungsmittel wird abgedampft, der Rückstand in 50 ml Chloroform aufgenommen, auf 0° abgekühlt und mit eiskalter wässeriger Ammoniak-Lösung basisch gemacht. Die wässerige Phase wird nochmals mit 3 × 20 ml Chloroform extrahiert und die Extrakte über Natriumsulfat getrocknet. Nach dem Abdampfen des Lösungsmittels erhält man 3,84 g eines Öls, das, gelöst in Essigsäureäthylester, über 38 g Kieselgel geschickt wird. Man dampft das Lösungsmittel ab, kristallisiert den erhaltenen Feststoff aus Äther und erhält 6-Benzyloxy-5-methylimidazo[1,5-a]pyridin vom Smp. 52–54°.

Der Ausgangsstoff 7-(3-Chlorpropyl)-imidazo-[1,5-a]pyridin für Verbindung e wird wie folgt hergestellt:

5,0 ml 40% Peressigsäure werden zu 5,18 g 4-(3-Chlorpropyl)-pyridin so zugegeben, dass die Reaktionstemperatur bei 80° liegt. Das Gemisch wird gerührt, bis die Temperatur auf 30° fällt. Überschüssige Persäure wird mit Natriumsulfit-Lösung zerstört und das Lösungsmittel im Vakuum destilliert. Der Rückstand wird in 50 ml Methylenchlorid gelöst, filtriert, das Lösungsmittel abgedampft, wobei man rohes 4-(3-Chlorpropyl)-pyridin-N-oxid erhält, das zusammen mit 4,66 g Dimethylsulfat 2 Stunden auf 80° erhitzt wird. Das entstandene 4-(3-Chlorpropyl)-1-methoxypyridinium-methylsulfat-Salz wird in 10 ml Wasser gelöst, auf 0° abgekühlt und mit einer eiskalten Lösung von 6,7 g (100 mMol) Kaliumcyanid in 20 ml einer 0,25-n. Natriumhydroxid-Lösung 22 Stunden lang

umgesetzt. Das Produkt wird mit 3 × 30 ml Methylenchlorid extrahiert und die Extrakte über Natriumsulfat getrocknet. Das Lösungsmittel wird abgedampft, der Rückstand, gelöst in Äther, über 45 g Kieselgel filtriert, wobei man 4-(3-Chlorpropyl)-2-cyanpyridin erhält. NMR ($CDCl_3$): 3,56 (t, 2H), 7,40 (d, 1H), 7,57 (s. 1H), 8,60 (d, 1H).

Eine Lösung von Boran-dimethylsulfid (0,83 ml, 7,7 mMol) in 7 ml Tetrahydrofuran wird langsam zu einer unter Rückfluss kochenden Lösung von 4-(3-Chlorpropyl)-2-cyanpyridin (1,24 g, 6,9 mMol) in 7 ml Tetrahydrofuran gegeben, wobei gleichzeitig Dimethylsulfid abdestilliert wird. Nach beendeter Zugabe wird das Gemisch 15 Minuten unter Rückfluss gekocht, auf 30° gekühlt und mit 6-n. Salzsäure versetzt. Am Ende der Wasserstoffentwicklung wird das Gemisch 30 Minuten unter Rückfluss gekocht, auf 0° gekühlt, mit festem Natriumcarbonat gesättigt und mit Methylenchlorid (4 × 50 ml) extrahiert. Die organische Extrakte werden über Natriumsulfat getrocknet und eingedampft. Man erhält ein Öl, welches durch 10 g Kieselgel (Elutionsmittel: 1:1 Essigsäureäthylester-Methanol) filtriert wird. Man erhält 2-Aminomethyl-4-(3-chlorpropyl)-pyridin als gelbes Öl, NMR ($CDCl_3$): 3,55 (t, 2H), 4,20 (s, 2H).

Eine Lösung von 2-Aminomethyl-4-(3-chlorpropyl)-pyridin (0,47 g) in 1 ml Ameisensäure wird 18 Stunden bei 90° erhitzt, auf 0° gekühlt und mit gesättigter wässeriger Ammoniaklösung basisch gemacht. Extraktion mit Methylenchlorid (4 × 10 ml), Trocknen über Magnesiumsulfat und Eindampfen ergibt 2-(N-Formylaminomethyl)-4-(3-chlorpropyl)-pyridin (IR 1674 $cm^{-1}$). Dieses wird in Phosphoroxychlorid (0,75 g) 15 Stunden bei 90° erhitzt. Das überschüssige Phosphoroxychlorid wird mit Toluol abgedampft, der Rückstand in Methylenchlorid (15 ml) suspendiert, auf 0° gekühlt und mit gesättigter Ammoniaklösung basisch gemacht. Extraktion mit Methylenchlorid (4 × 15 ml), Trocknen über Natriumsulfat und Dünnschichtchromatographie (Kieselgel, Essigsäureäthylester) des Rückstands ergibt 7-(3-Chlorpropyl)-imidazo[1,5-a]pyridin (Rf = 0,24, Essigsäureäthylester) als ein gummiartiges Material. NMR ($CDCl_3$): 3,58 (t, 2H), 6,42 (q, 1H), 7,21 (s, 1H), 7,32 (s, 1H), 7,88 (d, 1H), 8,07 (s, 1H).

Beispiel 30:

Herstellung von 10 000 Tabletten mit einem Gehalt von je 10 mg der aktiven Substanz:

Bestandteile:

| | |
|---|---|
| 5-(4-Carboxybuta-1,3-dienyl)imidazo[1,5-a]pyridin | 100 g |
| Milchzucker | 1157 g |
| Maisstärke | 75 g |
| Polyäthylenglykol 6000 | 75 g |
| Talkpulver | 75 g |
| Magnesiumstearat | 18 g |
| gereinigtes Wasser | q.s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe von mehr Wasser granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 31:

Herstellung von 10 000 Kapseln mit einem Gehalt von je 25 mg der aktiven Substanz:

Bestandteile:

| | |
|---|---|
| 5-[p-(2-Carboxyprop-1-enyl)phenyl]imidazo[1,5-a]pyridin | 250 g |
| Milchzucker | 1650 g |
| Talkpulver | 100 g |

Verfahren:

Sämtliche pulverigen Bestandteile werden mit einem Sieb von 0,6 mm Maschenweite gesiebt. Dann wird der Wirkstoff zuerst mit Talk und nachher mit Milchzucker in einem geeigneten Mischer homogenisiert. Kapseln Nr. 3 werden mit je 200 mg der erhaltenen Mischung in einer Füllmaschine abgefüllt.

In analoger Weise werden auch Tabletten und Kapseln, enthaltend ungefähr 10 bis 100 mg von anderen Verbindungen der Erfindung, z.B. von den in den anderen Beispielen beschriebenen Verbindungen, hergestellt.

Beispiel 32:

0,72 g p-Cyanbenzyltriphenylphosphoniumbromid werden zu einer Lösung von Lithiumdiisopropylamid, hergestellt aus 0,24 ml Diisopropylamin und 1,0 ml einer 1,64 M n-Butyllithium-Lösung in Hexan, in 14 ml Tetrahydrofuran bei 0° unter Stickstoff gegeben. Man rührt 20 Minuten lang und gibt dann 0,19 g 5-Formylimidazo[1,5-a]pyridin, gelöst in 5 ml Tetrahydrofuran, bei 0° hinzu. Man lässt das Reaktionsgemisch auf Raumtemperatur erwärmen, rührt eine Stunde lang und beendet die Reaktion durch Zugabe von 20 ml 0,5-n. Salzsäure. Das Reaktionsgemisch wird mit Äther gewaschen, der pH-Wert auf 9 eingestellt und die wässrige Phase mit Chloroform extrahiert. Der Extrakt wird über Natriumsulfat getrocknet und zur Trokkene eingedampft. Der Rückstand wird aus Chloroform kristallisiert, und man erhält 5-(p-Cyanphenyläthenyl)-imidazo[1,5-a]pyridin, Smp. 228–230°.

Beispiel 33:

Eine Lösung von 0,13 g 5-(p-Cyanphenyläthenyl)-imidazo[1,5-a]pyridin und 0,33 g Kaliumhydroxid in 1,2 ml Äthanol und 1,4 ml Wasser wird 20 Stunden unter Rückfluss gekocht. Das Reaktionsgemisch wird zur Trockene eingedampft und der Rückstand in Wasser aufgenommen. Man wäscht

die wässerige Lösung mit Essigsäureäthylester, stellt den pH-Wert auf 5 ein, filtriert den Niederschlag ab, trocknet ihn und erhält 5-(p-Carboxyphenyläthenyl)-imidazo[1,5-a]pyridin, Smp. 280° (Zersetzung).

Beispiel 34:

Eine Lösung von 58 mg 5-(p-Carboxyphenyläthenyl)-imidazo[1,5-a]pyridin in 30 ml wasserfreiem Äthanol und 0,9 ml konz. Salzsäure wird mit 0,1 g 10% Palladium-auf-Kohle bei 60° 8 Stunden bei einem Druck von 2,9 bar hydriert. Man filtriert, verdampft das Lösungsmittel und erhält ein Öl, das nach Behandlung mit Methanol kristallisiert und dem 5-(p-Äthoxycarbonylphenäthyl)-5,6,7,8-tetrahydroimidazo[1,5-a]pyridin-Hydrochlorid vom Smp. 182–183° entspricht.

Beispiel 35:

Man gibt in mehreren Portionen 1,6 g p-Cyanphenol unter Stickstoff zu 0,675 g 50% Natriumhydrid in 40 ml trockenem Dimethylformamid. Man erhält eine Lösung von Natrium-p-cyanphenoxid, zu der man eine Lösung von 2,5 g 5-(4-Chlorbutyl)-imidazo[1,5-a]pyridin in 7 ml trockenem Dimethylformamid langsam hinzugibt. Das Reaktionsgemisch wird 15 Stunden auf 50° erhitzt, dann abgekühlt und mit Säure auf pH 2 gebracht. Die wässrige Phase wird mit 200 ml Äther extrahiert, mit 50% Natronlauge auf pH 11 eingestellt und nochmals mit 200 ml Äther extrahiert. Der letztere Ätherextrakt wird mit einer Kochsalzlösung gewaschen, über Natriumsulfat getrocknet und eingedampft, wobei man 5-[4-(p-Cyanphenoxy)butyl]-imidazo[1,5-a]pyridin vom Smp. 95–96° erhält.

Beispiel 36:

Eine Lösung von 0,67 g 5-(p-Äthoxycarbonylphenyl)-imidazo[1,5-a]pyridin in 10 ml Äthanol wird mit 8 ml 1-n. Natronlauge 3 Stunden unter Rückfluss gekocht. Man dampft das Äthanol ab, wäscht die wässerige Phase mit Essigsäureäthylester, stellt einen pH-Wert von 1 ein und sammelt den Feststoff, der dem 5-(p-Carboxyphenyl)-imidazo[1,5-a]pyridin-Hydrochlorid vom Smp. 292–294° (Zersetzung) entspricht. Dies ist das Hydrochlorid der Verbindung des Beispiels 8.

Beispiel 37:

Eine Lösung von 2,0 g 5-(p-Äthoxycarbonylphenyl)-imidazo[1,5-a]pyridin und 30 ml konz. Salzsäure in 120 ml wasserfreiem Äthanol wird bei einem Druck von 2,8 bar bei 60° 3 Stunden hydriert, wobei 1,0 g 10% Palladium-auf-Kohle als Katalysator verwendet wird. Man filtriert und dampft zur Trockene ein, kristallisiert den Rückstand aus Isopropanol/Äther und erhält das 5-(p-Äthoxycarbonylphenyl)-5,6,7,8-tetrahydroimidazo[1,5-a]pyridin-Hydrochlorid vom Smp. 164–166°.

Beispiel 38:

Eine Lösung von 0,60 g 5-(p-Äthoxycarbonylphenyl)-5,6,7,8-tetrahydroimidazo[1,5-a]pyridin in 8.0 ml 1-n. Natronlauge und 8,0 ml Äthanol wird 3 Stunden unter Rückfluss gekocht, dann abgekühlt und eingedampft. Der Rückstand wird in 10 ml Wasser gelöst, mit 5 ml Essigsäureäthylester gewaschen und auf pH 5 eingestellt. Man filtriert den Feststoff ab, trocknet ihn und erhält 5-(p-Carboxyphenyl)-5,6,7,8-tetrahydroimidazo[1,5-a]pyridin vom Smp. 309–310° (Zersetzung.

**Patentansprüche für die Vertragsstaaten: BE CH DE FR GB IT LI LU NL SE**

1. Imidazo[1,5-a]pyridin-Derivate der allgemeinen Formel I

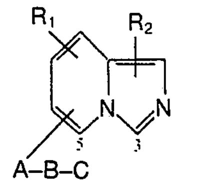

(I)

und ihre 5,6,7,8-Tetrahydroderivate, worin

$R_1$ Wasserstoff, Halogen, Niederalkyl, Niederalkoxy, Hydroxy oder Arylniederalkoxy, worin Aryl Phenyl oder durch Niederalkoxy, Niederalkyl, Halogen oder Trifluormethyl mono- oder disubstituiertes Phenyl ist, bedeutet,

$R_2$ Wasserstoff, Halogen oder Niederalkyl darstellt,

C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

(a) A unsubstituiertes oder durch Niederalkyl substituiertes Äthenylen (Vinylen) und B Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen, Phenylen-(thio oder oxy)-$C_1$–$C_4$-alkylen, oder

(b) A eine Bindung und B $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, und ihre Salze,

wobei die mit «nieder» bezeichneten Reste höchstens 7 Kohlenstoffatome aufweisen.

2. Verbindungen der allgemeinen Formel I gemäss Anspruch 1, worin

$R_1$ Wasserstoff, Halogen, Niederalkyl, Niederalkoxy, Hydroxy, Phenyl-niederalkoxy oder Phenyl-niederalkoxy, das im Phenylring durch Niederalkoxy, Niederalkyl oder Halogen mono- oder disubstituiert ist, bedeutet,

A Äthenylen oder durch Niederalkyl substituiertes Äthenylen bedeutet,

B für Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-

$C_2$–$C_4$-alkenylen, Phenylen oder Phenylen-(thio oder oxy)-$C_1$–$C_4$-alkylen steht,

und $R_2$ und C wie in Anspruch 1 definiert sind, und ihre Salze.

3. Verbindungen der allgemeinen Formel I

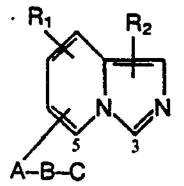

(I)

und ihre 5,6,7,8-Tetrahydro-Derivate, worin $R_1$ Wasserstoff, Halogen, Niederalkyl, Niederalkoxy, Hydroxy oder Aryl-niederalkoxy, worin Aryl unsubstituiertes oder durch Niederalkoxy, Niederalkyl, Halogen oder Trifluormethyl mono- oder disubstituiertes Phenyl ist, bedeutet, $R_2$ Wasserstoff, Halogen oder Niederalkyl darstellt, C Carboxy, Niederalkoxycarbonyl, unsubstituiertes oder mono- oder di-niederalkyl-substituiertes Carbamoyl, Cyan, Hydroxymethyl, Formyl, 5-Tetrazolyl, unsubstituiertes oder durch Niederalkyl substituiertes 4,5-Dihydro-2-oxazolyl, oder Hydroxycarbamoyl bedeutet, A für eine einfache Bindung steht und B $C_1$–$C_4$-Alkylenphenylen-$C_1$–$C_4$-alkylen, $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, und ihre Salze, wobei die mit «nieder» bezeichneten Reste höchstens 7 Kohlenstoffatome aufweisen.

4. Verbindungen der allgemeinen Formel I, und ihre 5,6,7,8-Tetrahydroderivate, gemäss Anspruch 1 oder 3, worin die Gruppe –A–B–C an die 5-Stellung des Imidazo[1,5-a]pyridinkerns gebunden ist.

5. Verbindungen der allgemeinen Formel I, und ihre 5,6,7,8-Tetrahydroderivate, gemäss Anspruch 3, worin die Gruppe –A–B–C an die 5-Stellung des Imidazo[1,5-a]pyridinkerns gebunden ist und B Niederalkylen-(thio oder oxy)-niederalkylen mit 4 bis 10 Kohlenstoffatomen, Phenylen oder Phenylen-niederalkenylen mit 8 bis 10 Kohlenstoffatomen bedeutet.

6. Verbindungen gemäss Anspruch 1 von der allgemeinen Formel II

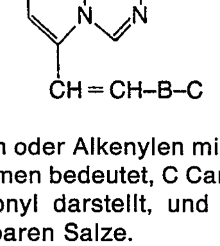

(II)

worin B Alkylen oder Alkenylen mit jeweils 2 bis 4 Kohlenstoffatomen bedeutet, C Carboxy oder Niederalkoxycarbonyl darstellt, und ihre therapeutisch verwendbaren Salze.

7. Verbindungen gemäss Anspruch 1 der allgemeinen Formel III

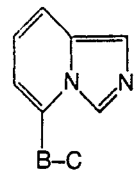

(III)

worin

B Niederalkylen-(thio oder oxy)-niederalkylen mit 4 bis 10 Kohlenstoffatomen, Phenylen, Phenylen-niederalkylen oder Niederalkylen-phenylen mit jeweils 7 bis 10 Kohlenstoffatomen, Alkylenthiophenylen oder Alkylenoxyphenylen mit jeweils 7 bis 10 Kohlenstoffatomen oder Phenylen-niederalkenylen mit 8 bis 10 Kohlenstoffatomen bedeutet,

C Carboxy, Niederalkoxycarbonyl oder Carbamoyl darstellt, und ihre therapeutisch verwendbaren Salze.

8. 5-(4-Carboxybuta-1,3-dienyl)-imidazo[1,5-a]pyridin gemäss Anspruch 1 und seine therapeutisch verwendbaren Salze.

9. 5-[p-(2-Carboxy-prop-1-enyl)phenyl]-imidazo[1,5-a]pyridin gemäss Anspruch 1 und seine therapeutisch verwendbaren Salze.

10. 5-(9-Carboxynona-6,8-dienyl)-imidazo[1,5-a]pyridin gemäss Anspruch 1 und seine therapeutisch verwendbaren Salze.

11. Pharmazeutische Präparate enthaltend eine Verbindung gemäss einem der Ansprüche 1 bis 10, oder ein therapeutisch verwendbares Salz davon, zusammen mit einem pharmazeutischen Trägermaterial.

12. Die Verbindungen der Ansprüche 1 bis 10 zur Anwendung in einem Verfahren zur therapeutischen Behandlung des menschlichen oder tierischen Körpers.

13. Die Verbindungen der Ansprüche 1 bis 10 zur Anwendung als Hemmer der Thromboxan-Synthetase.

14. Verwendung der Verbindungen der Ansprüche 1 bis 10 zur Herstellung von pharmazeutischen Präparaten.

15. Verfahren zur Herstellung von Verbindungen der allgemeinen Formel I gemäss Anspruch 1, worin $R_2$ Wasserstoff oder Niederalkyl bedeutet, dadurch gekennzeichnet, dass man eine Verbindung der Formel IV

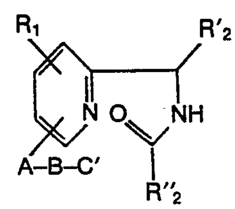

(IV)

worin $R_2'$ und $R_2''$ Wasserstoff oder einer der Reste $R_2'$ und $R_2''$ Niederalkyl bedeuten, $R_1$, A und B die unter Formel I angegebene Bedeutung haben und C′ Carboxy, Niederalkoxycarbonyl, unsubstituiertes, mono- oder di-niederalkyl-substituiertes Carbamoyl, Cyan, Hydroxymethyl, Niederalkanoyloxymethyl, veräthertes Hydroxymethyl, Halogenmethyl, 5-Tetrazolyl, unsubstituiertes oder durch Niederalkyl substituiertes 4,5-Dihydro-2-oxazolyl, Hydroxycarbamoyl, Halogen oder $C_1$–$C_2$-

Alkylendioxymethyl bedeutet, ringschliesst, und eine erhaltene Verbindung der Formel Ia

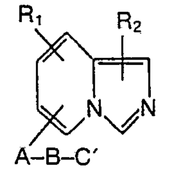

(Ia)

worin sich C′ von C unterscheidet, in eine Verbindung der Formel I umwandelt.

16. Verfahren zur Herstellung von Verbindungen der Formel I gemäss Anspruch 1, worin A unsubstituiertes oder durch Niederalkyl substituiertes Äthenylen bedeutet, dadurch gekennzeichnet, dass man eine Verbindung der Formel VII

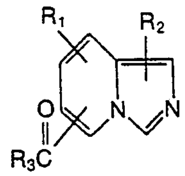

(VII)

worin $R_1$ und $R_2$ die unter Formel I angegebene Bedeutung haben und $R_3$ Wasserstoff oder Niederalkyl darstellt, mit einer Verbindung der Formel VIII

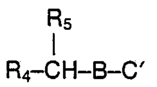

(VIII)

worin $R_4$ Wasserstoff oder Niederalkyl bedeutet, $R_5$ Di-niederalkylphosphono oder Triarylphosphoranyl bedeutet, C′ Carboxy, Niederalkoxycarbonyl, Carbamoyl, mono- oder di-niederalkyl-substituiertes Carbamoyl, Cyan, Halogenmethyl, Niederalkanoyloxymethyl, veräthertes Hydroxymethyl, 5-Tetrazolyl, unsubstituiertes oder durch Niederalkyl substituiertes 4,5-Dihydro-2-oxazolyl, oder Hydroxycarbamoyl bedeutet und B wie unter Formel I definiert ist, kondensiert, und eine erhaltene Verbindung, worin sich C′ von C unterscheidet, in eine Verbindung der Formel I umwandelt.

17. Verfahren zur Herstellung von Verbindungen der Formel I gemäss den Ansprüchen 1 und 3, worin $R_1$ und $R_2$ Wasserstoff oder Niederalkyl darstellen, A eine einfache Bindung bedeutet, B $C_1$–$C_4$-Alkylenphenylen-$C_1$–$C_4$-alkylen, $C_1$–$C_4$-Alkylenphenylen, Niederalkylen-(thio oder oxy)-niederalkylen mit insgesamt 2–12 Kohlenstoffatomen, $C_1$–$C_4$-Alkylen-(thio oder oxy)-phenylen, $C_1$–$C_4$-Alkylenphenylen-$C_2$–$C_4$-alkenylen oder Alkadienylen mit 5 bis 12 Kohlenstoffatomen bedeutet, dadurch gekennzeichnet, dass man eine Verbindung der Formel IX

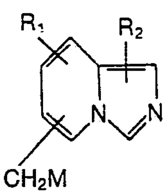

(IX)

worin M ein Alkalimetall bedeutet, $R_1$ und $R_2$ Wasserstoff oder Niederalkyl darstellen, mit einem reaktionsfähigen funktionellen Derivat einer Verbindung der Formel X

HO–B′–C′ (X)

worin B′ $C_1$–$C_4$-Alkylenphenylen-$C_1$–$C_4$-alkylen, $C_1$–$C_4$-Alkylenphenylen, Niederalkylen-(thio oder oxy)-niederalkylen mit insgesamt 2 bis 12 Kohlenstoffatomen, $C_1$–$C_4$-Alkylen-(thio oder oxy)-phenylen, $C_1$–$C_4$-Alkylenphenylen-$C_2$–$C_4$-alkenylen oder $C_1$–$C_7$-Alkadienylen bedeutet, C′ Carboxy, Tri($C_1$–$C_4$-alkoxy)methyl, unsubstituiertes, mono- oder di-niederalkyl-substituiertes Carbamoyl, Cyan, Niederalkanoyloxymethyl, veräthertes Hydroxymethyl, Halogenmethyl, unsubstituiertes oder durch Niederalkyl substituiertes 4,5-Dihydro-2-oxazolyl, 5-Tetrazolyl oder Hydroxycarbamoyl bedeutet, umsetzt und eine erhaltene Verbindung der Formel Ib

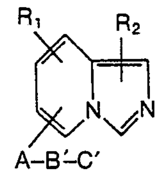

(Ib)

worin A, B′, C′, $R_1$ und $R_2$ die oben angegebene Bedeutung haben, und C′ sich von C unterscheidet, in eine Verbindung der Formel I umwandelt.

18. Verfahren zur Herstellung von Verbindungen der Formel I gemäss Anspruch 1, worin B $C_1$–$C_4$-Alkylen-(thio oder oxy)-phenylen, Phenylen-(thio oder oxy)-$C_1$–$C_4$-alkylen oder Niederalkylen-(thio oder oxy)-niederalkylen mit insgesamt 2 bis 12 Kohlenstoffatomen bedeutet, dadurch gekennzeichnet, dass man ein Zwischenprodukt der Formel Ia, worin B $C_1$–$C_7$-Alkylen oder Phenylen und C′ reaktionsfähiges, funktionell modifiziertes Hydroxymethyl bedeuten, mit einem $C_1$–$C_7$-Alkanol, einem $C_1$–$C_7$-Alkanthiol, einem Phenol oder Thiophenol, welche jeweils durch C oder C′ substituiert sind, umsetzt, und eine erhaltene Verbindung, worin sich C′ von C unterscheidet, in eine Verbindung der Formel I umwandelt.

19. Verfahren zur Herstellung von Verbindungen der Formel I, oder ihren 5,6,7,8-Tetrahydroderivaten, gemäss Anspruch 1, dadurch gekennzeichnet, dass man eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

20. Verfahren zur Herstellung von 5,6,7,8-Tetrahydroimidazo[1,5-a]pyridinen gemäss Anspruch 1, oder ihren Salzen, dadurch gekennzeichnet, dass man ein entsprechendes Imidazo[1,5-a]pyridin der Formel I mit Wasserstoff reduziert.

21. Verfahren gemäss Anspruch 20 zur Herstellung von 5,6,7,8-Tetrahydroimidazo[1,5-a]pyridi-

nen gemäss Anspruch 1, oder ihren Salzen, dadurch gekennzeichnet, dass man ein entsprechendes Imidazo[1,5-a]pyridin der Formel I mit Wasserstoff in Gegenwart von Hydrierungskatalysatoren reduziert.

22. Die nach dem Verfahren der Ansprüche 15–21 erhältlichen Verbindungen.

**Patentansprüche für den Vertragsstaat: AT**

1. Verfahren zur Herstellung von Imidazo[1,5-a]pyridin-Derivaten der allgemeinen Formel I

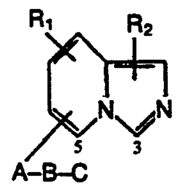

(I)

worin

$R_1$ Wasserstoff, Halogen, Niederalkyl, Niederalkoxy, Hydroxy oder Arylniederalkoxy, worin Aryl, Phenyl oder durch Niederalkoxy, Niederalkyl, Halogen oder Trifluormethyl mono- oder disubstituiertes Phenyl ist, bedeutet,

$R_2$ Wasserstoff oder Niederalkyl darstellt,

C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

(a) A unsubstituiertes oder durch Niederalkyl substituiertes Äthenylen (Vinylen) und B Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen, Phenylen-(thio oder oxy)-$C_1$–$C_4$-alkylen, oder

(b) A eine Bindung und B $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, oder ihren Salzen,

wobei die mit «nieder» bezeichneten Reste höchstens 7 Kohlenstoffatome aufweisen, dadurch gekennzeichnet, dass man eine Verbindung der Formel IV

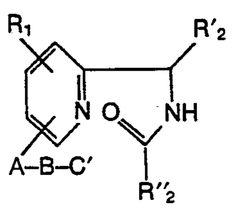

(IV)

worin $R_2'$ und $R_2''$ Wasserstoff oder einer der Reste $R_2'$ und $R_2''$ Niederalkyl bedeuten, $R_1$, A und B die unter Formel I angegebene Bedeutung haben und C' Carboxy, Niederalkoxycarbonyl, unsubstituiertes, mono- oder di-niederalkyl-substituiertes Carbamoyl, Cyan, Hydroxymethyl, Niederalkanoyloxymethyl, veräthertes Hydroxymethyl, Halogenmethyl, 5-Tetrazolyl, unsubstituiertes oder durch Niederalkyl substituiertes 4,5-Dihydro-2-oxazolyl, Hydroxycarbamoyl, Halogen oder $C_1$–$C_2$-Alkylendioxymethyl bedeutet, ringschliesst, und eine erhaltene Verbindung der Formel Ia

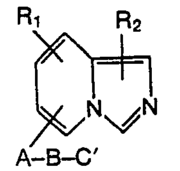

(Ia)

worin sich C' von C unterscheidet, in eine Verbindung der Formel I umwandelt.

2. Verfahren zur Herstellung von Imidazo[1,5-a]pyridin-Derivaten der allgemeinen Formel I

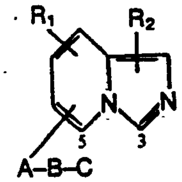

(I)

worin

$R_1$ Wasserstoff, Halogen, Niederalkyl, Niederalkoxy, Hydroxy oder Arylniederalkoxy, worin Aryl, Phenyl oder durch Niederalkoxy, Niederalkyl, Halogen oder Trifluormethyl mono- oder disubstituiertes Phenyl ist, bedeutet,

$R_2$ Wasserstoff, Halogen oder Niederalkyl darstellt,

C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

A unsubstituiertes oder durch Niederalkyl substituiertes Äthenylen (Vinylen) und B Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen oder Phenylen-(thio oder oxy)-$C_1$–$C_4$-alkylen bedeutet, oder ihren Salzen,

wobei die mit «nieder» bezeichneten Reste höchstens 7 Kohlenstoffatome aufweisen, dadurch gekennzeichnet, dass man eine Verbindung der Formel VII

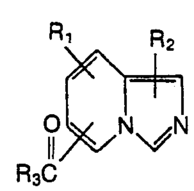

(VII)

worin $R_1$ und $R_2$ die unter Formel I angegebene Bedeutung haben und $R_3$ Wasserstoff oder Niederalkyl darstellt, mit einer Verbindung der Formel VIII

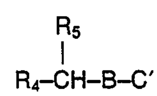

(VIII)

worin $R_4$ Wasserstoff oder Niederalkyl bedeutet, $R_5$ Di-niederalkylphosphono oder Triarylphosphoranyl bedeutet, C′ Carboxy, Niederalkoxycarbonyl, Carbamoyl, mono- oder di-niederalkylsubstituiertes Carbamoyl, Cyan, Halogenmethyl, Niederalkanoyloxymethyl, veräthertes Hydroxymethyl, 5-Tetrazolyl, unsubstituiertes oder durch Niederalkyl substituiertes 4,5-Dihydro-2-oxazolyl, oder Hydroxycarbamoyl bedeutet und B wie unter Formel I definiert ist, kondensiert, und eine erhaltene Verbindung, worin sich C′ von C unterscheidet, in eine Verbindung der Formel I umwandelt.

3. Verfahren zur Herstellung von Imidazo[1,5-a]pyridin-Derivaten der allgemeinen Formel I

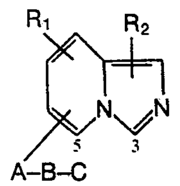

(I)

worin

$R_1$ und $R_2$ Wasserstoff oder Niederalkyl darstellen,

C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

A eine einfache Bindung und

B $C_1$–$C_4$-Alkylenphenylen-$C_1$–$C_4$-alkylen, $C_1$–$C_4$-Alkylenphenylen, Niederalkylen-(thio oder oxy)-niederalkylen mit insgesamt 2–12 Kohlenstoffatomen, $C_1$–$C_4$-Alkylen-(thio oder oxy)-phenylen, $C_1$–$C_4$-Alkylenphenylen-$C_2$–$C_4$-alkenylen oder Alkadienylen mit 5 bis 12 Kohlenstoffatomen bedeutet, oder ihren Salzen, wobei die mit «nieder» bezeichneten Reste höchstens 7 Kohlenstoffatome aufweisen, dadurch gekennzeichnet, dass man eine Verbindung der Formel IX

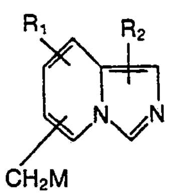

(IX)

worin M ein Alkalimetall bedeutet, $R_1$ und $R_2$ Wasserstoff oder Niederalkyl darstellen, mit einem reaktionsfähigen funktionellen Derivat einer Verbindung der Formel X

$$HO{-}B'{-}C' \quad (X)$$

worin B′ $C_1$–$C_4$-Alkylenphenylen-$C_1$–$C_4$-alkylen, $C_1$–$C_4$-Alkylenphenylen, Niederalkylen-(thio oder oxy)-niederalkylen mit insgesamt 2–12 Kohlenstoffatomen, $C_1$–$C_4$-Alkylen-(thio oder oxy)-phenylen, $C_1$–$C_4$-Alkylenphenylen-$C_2$–$C_4$-alkenylen oder $C_1$–$C_7$-Alkadienylen bedeutet, C′ Carboxy, Tri($C_1$–$C_4$-alkoxy)methyl, unsubstituiertes, mono- oder di-niederalkyl-substituiertes Carbamoyl, Cyan, Niederalkanoyloxymethyl, veräthertes Hydroxymethyl, Halogenmethyl, unsubstituiertes oder durch Niederalkyl substituiertes 4,5-Dihydro-2-oxazolyl, 5-Tetrazolyl oder Hydroxycarbamoyl bedeutet, umsetzt und eine erhaltene Verbindung der Formel Ib

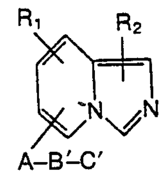

(Ib)

worin A, B′, C′, $R_1$ und $R_2$ die oben angegebene Bedeutung haben, und C′ sich von C unterscheidet, in eine Verbindung der Formel I umwandelt.

4. Verfahren zur Herstellung von Imidazo[1,5-a]pyridin-Derivaten der allgemeinen Formel I

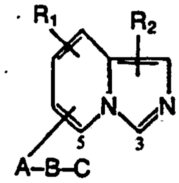

(I)

worin

$R_1$ Wasserstoff, Halogen, Niederalkyl, Niederalkoxy, Hydroxy oder Arylniederalkoxy, worin Aryl Phenyl oder durch Niederalkoxy, Niederalkyl, Halogen oder Trifluormethyl mono- oder disubstituiertes Phenyl ist, bedeutet,

$R_2$ Wasserstoff, Halogen oder Niederalkyl darstellt,

C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

(a) A unsubstituiertes oder durch Niederalkyl substituiertes Äthenylen (Vinylen) und B Phenylen-(thio oder oxy)-$C_1$–$C_4$-alkylen, oder

(b) A eine Bindung und B $C_1$–$C_4$-Alkylen-(thio oder oxy)-phenylen, Phenylen-(thio oder oxy)-$C_1$–$C_4$-alkylen oder Niederalkylen-(thio oder oxy)-niederalkylen mit insgesamt 2 bis 12 Kohlenstoffatomen bedeuten, oder ihren Salzen, wobei die mit «nieder» bezeichneten Reste höchstens 7 Kohlenstoffatome aufweisen, dadurch gekennzeichnet, dass man ein Zwischenprodukt der Formel Ia, worin B $C_1$–$C_7$-Alkylen oder Phenylen und C′ reaktionsfähiges, funktionell modifiziertes Hydroxymethyl bedeuten, mit einem $C_1$–$C_7$-Alkanol, einem $C_1$–$C_7$-Alkanthiol, einem Phenol oder Thiophenol, welche jeweils durch C oder C′ substituiert sind, umsetzt und eine erhaltene Verbindung, worin sich C′ von C unterscheidet, in eine Verbindung der Formel I umwandelt.

5. Verfahren zur Herstellung von Imidazo[1,5-a]pyridin-Derivaten der allgemeinen Formel I

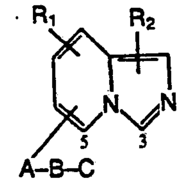

(I)

oder ihren 5,6,7,8-Tetrahydroderivaten, worin $R_1$ Wasserstoff, Halogen, Niederalkyl, Niederalkoxy, Hydroxy oder Arylniederalkoxy, worin Aryl Phenyl oder durch Niederalkoxy, Niederalkyl, Halogen oder Trifluormethyl mono- oder disubstituiertes Phenyl ist, bedeutet, $R_2$ Wasserstoff, Halogen oder Niederalkyl darstellt, C Carboxy, Niederalkoxycarbonyl, unsubstituiertes, mono- oder di-niederalkyl-substituiertes Carbamoyl, Cyan, Formyl, Hydroxymethyl, 5-Tetrazolyl, unsubstituiertes oder durch Niederalkyl substituiertes 4,5-Dihydro-2-oxazolyl, oder Hydroxycarbamoyl bedeutet und

(a) A unsubstituiertes oder durch Niederalkyl substituiertes Äthenylen (Vinylen) und B Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen, Phenylen-(thio oder oxy)-$C_1$–$C_4$-alkylen, oder

(b) A eine Bindung und B $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, oder ihren Salzen, wobei die mit «nieder» bezeichneten Reste höchstens 7 Kohlenstoffatome aufweisen, dadurch gekennzeichnet, dass man eine erhaltene Verbindung der Formel I, oder ein 5,6,7,8-Tetrahydroderivat davon, in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

6. Verfahren zur Herstellung von 5,6,7,8-Tetrahydroimidazo[1,5-a]pyridinen gemäss Anspruch 5, oder ihren Salzen, dadurch gekennzeichnet, dass man ein entsprechendes Imidazo[1,5-a]pyridin der Formel I mit Wasserstoff reduziert.

7. Verfahren gemäss Anspruch 6 zur Herstellung von 5,6,7,8-Tetrahydroimidazo[1,5-a]pyridinen gemäss Anspruch 5, oder ihren Salzen, dadurch gekennzeichnet, dass man ein entsprechendes Imidazo[1,5-a]pyridin der Formel I mit Wasserstoff in Gegenwart von Hydrierungskatalysatoren reduziert.

8. Verfahren gemäss einem der Ansprüche 1 bis 2 oder 4 bis 5, dadurch gekennzeichnet, dass man Verbindungen der allgemeinen Formel I, worin $R_1$ Wasserstoff, Halogen, Niederalkyl, Niederalkoxy, Hydroxy, Phenyl-niederalkoxy oder Phenyl-niederalkoxy, das im Phenylring durch Niederalkoxy, Niederalkyl oder Halogen mono- oder disubstituiert ist, bedeutet,

A Äthenylen oder durch Niederalkyl substituiertes Äthenylen bedeutet,

B für Alkylen mit 1 bis 12 Kohlenstoffatomen, Alkinylen oder Alkenylen mit 2 bis 12 Kohlenstoffatomen, Phenylen-$C_1$–$C_4$-alkylen, Phenylen-$C_2$–$C_4$-alkenylen, Phenylen oder Phenylen-(thio oder oxy)-$C_1$–$C_4$-alkylen steht, und

$R_2$ und C wie in Anspruch 1, 2, 4 oder 5 definiert sind, oder ihre Salze herstellt.

9. Verfahren gemäss einem der Ansprüche 1 oder 3 bis 7, dadurch gekennzeichnet, dass man Verbindungen der allgemeinen Formel I, oder ihre 5,6,7,8-Tetrahydro-Derivate, worin

$R_1$ Wasserstoff, Halogen, Niederalkyl, Niederalkoxy, Hydroxy oder Aryl-niederalkoxy, worin Aryl unsubstituiertes oder durch Niederalkoxy, Niederalkyl, Halogen oder Trifluormethyl mono- oder di-substituiertes Phenyl ist, bedeutet,

$R_2$ Wasserstoff, Halogen oder Niederalkyl darstellt,

C Carboxy, Niederalkoxycarbonyl, unsubstituiertes oder mono- oder di-niederalkyl-substituiertes Carbamoyl, Cyan, Hydroxymethyl, Formyl, 5-Tetrazolyl, unsubstituiertes oder durch Niederalkyl substituiertes 4,5-Dihydro-2-oxazolyl, oder Hydroxycarbamoyl bedeutet,

A für eine einfache Bindung steht und

B $C_1$–$C_4$-Alkylenphenylen-$C_1$–$C_4$-alkylen, $C_1$–$C_4$-Alkylenphenylen, Phenylen-$C_1$–$C_4$-alkylen, Phenylen, Niederalkylen-(thio oder oxy)-niederalkylen mit insgesamt 2–12 Kohlenstoffatomen, $C_1$–$C_4$-Alkylen-(thio oder oxy)-phenylen, Phenylen-(thio oder oxy)-$C_1$–$C_4$-alkylen, Phenylen-$C_2$–$C_4$-alkenylen, $C_1$–$C_4$-Alkylenphenylen-$C_2$–$C_4$-alkenylen oder Alkadienylen mit 5 bis 12 Kohlenstoffatomen bedeutet, oder ihre Salze herstellt.

10. Verfahren gemäss einem der Ansprüche 1 bis 9, dadurch gekennzeichnet, dass man Verbindungen der allgemeinen Formel I, oder ihre 5,6,7,8-Tetrahydroderivate, worin die Gruppe –A–B–C an die 5-Stellung des Imidazo[1,5-a]pyridinkerns gebunden ist, herstellt.

11. Verfahren gemäss Anspruch 9, dadurch gekennzeichnet, dass man Verbindungen der allgemeinen Formel I, oder ihre 5,6,7,8-Tetrahydroderivate, worin die Gruppe –A–B–C an die 5-Stellung des Imidazo[1,5-a]pyridinkerns gebunden ist und B Niederalkylen-(thio oder oxy)-niederalkylen mit 4 bis 10 Kohlenstoffatomen, Phenylen oder Phenylen-niederalkenylen mit 8 bis 10 Kohlenstoffatomen bedeutet, herstellt.

12. Verfahren gemäss einem der Ansprüche 1 bis 2 oder 5, dadurch gekennzeichnet, dass man Verbindungen von der allgemeinen Formel II

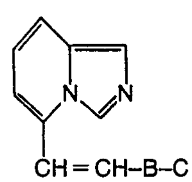

(II)

worin B Alkylen oder Alkenylen mit jeweils 2 bis 4 Kohlenstoffatomen bedeutet, C Carboxy oder Niederalkoxycarbonyl darstellt, oder ihre therapeutisch verwendbaren Salze herstellt.

13. Verfahren gemäss einem der Ansprüche 1 oder 3 bis 5, dadurch gekennzeichnet, dass man Verbindungen von der allgemeinen Formel III

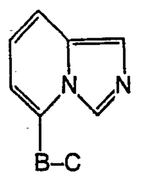

(III)

worin B Niederalkylen-(thio oder oxy)-niederalkylen mit 4 bis 10 Kohlenstoffatomen, Phenylen, Phenylen-niederalkylen oder Niederalkylen-phenylen mit jeweils 7 bis 10 Kohlenstoffatomen, Alkylenthiophenylen oder Alkylenoxyphenylen mit jeweils 7 bis 10 Kohlenstoffatomen oder Phenylen-niederalkenylen mit 8 bis 10 Kohlenstoffatomen bedeutet, C Carboxy, Niederalkoxycarbonyl oder Carbamoyl darstellt, oder ihre therapeutisch verwendbaren Salze herstellt.

14. Verfahren gemäss einem der Ansprüche 1 bis 2 oder 5, dadurch gekennzeichnet, dass man 5-(4-Carboxybuta-1,3-dienyl)-imidazo[1,5-a]pyridin oder ein therapeutisch verwendbares Salz davon herstellt.

15. Verfahren gemäss einem der Ansprüche 1 oder 5, dadurch gekennzeichnet, dass man 5-[p-(2-Carboxy-prop-1-enyl)phenyl]-imidazo[1,5-a]pyridin oder ein therapeutisch verwendbares Salz davon herstellt.

16. Verfahren gemäss einem der Ansprüche 1, 3 oder 5, dadurch gekennzeichnet, dass man 5-(9-Carboxynona-6,8-dienyl)-imidazo[1,5-a]pyridin oder ein therapeutisch verwendbares Salz davon herstellt.

17. Verfahren zur Herstellung eines pharmazeutischen Präparates, gekennzeichnet durch die Verarbeitung eines erfindungsgemässen Wirkstoffes gemäss einem der Ansprüche 1 bis 16 mit einem pharmazeutischen Trägermaterial.

**Claims for the Contracting States: BE, CH, DE, FR, GB, IT, LI, LU, NL, SE**

1. Imidazo[1,5-a]pyridine derivatives of the general formula I

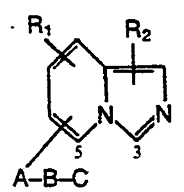

(I)

and 5,6,7,8-tetrahydro derivatives thereof, wherein

$R_1$ represents hydrogen, halogen, lower alkyl, lower alkoxy, hydroxy or aryl-lower alkoxy wherein aryl is phenyl or phenyl mono- or di-substituted by lower alkoxy, lower alkyl, halogen or by trifluoromethyl,

$R_2$ represents hydrogen, halogen or lower alkyl,

C represents carboxy, lower alkoxycarbonyl, unsubstituted or mono- or di-lower alkyl-substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl, and

(a) A represents unsubstituted ethenylene (vinylene) or ethenylene (vinylene) substituted by lower alkyl and B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene, phenylene-(thio or oxy)-$C_1$–$C_4$-alkylene, or

(b) A represents a bond and B represents $C_1$–$C_4$-alkylenephenylene, phenylene-$C_1$–$C_4$-alkylene, phenylene, lower alkylene-(thio or oxy)-lower alkylene of 2 to 12 carbon atoms in total, $C_1$–$C_4$-alkylene-(thio or oxy)-phenylene, phenylene-(thio or oxy)-$C_1$–$C_4$-alkylene, phenylene-$C_2$–$C_4$-alkenylene, $C_1$–$C_4$-alkylenephenylene-$C_2$–$C_4$-alkenylene, or alkadienylene of 5 to 12 carbon atoms, and salts thereof,

wherein the radicals referred to as "lower" have at most 7 carbon atoms.

2. Compounds of the general formula I according to claim 1, wherein

$R_1$ represents hydrogen, halogen, lower alkyl, lower alkoxy, hydroxy, phenyl-lower alkoxy or phenyl-lower alkoxy mono- or di-substituted on the phenyl ring by lower alkoxy, lower alkyl or by halogen,

A represents ethenylene or ethenylene substituted by lower alkyl,

B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene or phenylene-(thio or oxy)-$C_1$–$C_4$-alkylene, and

$R_2$ and C are as defined in claim 1,

and salts thereof.

3. Compounds of the general formula I

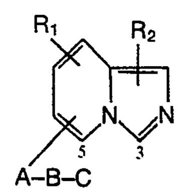

(I)

and 5,6,7,8-tetrahydro derivatives thereof, wherein $R_1$ represents hydrogen, halogen, lower alkyl, lower alkoxy, hydroxy or aryl-lower alkoxy wherein aryl is unsubstituted phenyl or phenyl mono- or di-substituted by lower alkoxy, lower alkyl, halogen or by trifluoromethyl, $R_2$ represents hydrogen, halogen or lower alkyl, C represents carboxy, lower alkoxycarbonyl, unsubstituted or mono- or di-lower alkyl-substituted carbamoyl, cyano, hydroxymethyl, formyl, 5-tetrazolyl, unsubstituted 4,5-dihydro-2-oxazolyl or 4,5-dihydro-2-oxazolyl substituted by lower alkyl, or hydroxycarbamoyl, A represents a single bond and B represents $C_1$–$C_4$-alkylenephenylene-$C_1$–$C_4$-alkylene, $C_1$–$C_4$-alkylenephenylene, phenylene-$C_1$–$C_4$-alkylene, phenylene, lower alkylene-(thio or oxy)-lower alkylene of 2 to 12 carbon atoms in total, $C_1$–$C_4$-alkylene-(thio or oxy)-phenylene, phenylene-(thio or oxy)-$C_1$–$C_4$-alkylene, phenylene-$C_2$–$C_4$-alkenylene, $C_1$–$C_4$-alkylenephenylene-$C_2$–$C_4$-alkenylene, or alkadienylene of 5 to 12 carbon atoms, and salts thereof, wherein the radicals referred to as "lower" have at most 7 carbon atoms.

4. Compounds of the general formula I, and 5,6,7,8-tetrahydro derivatives thereof, according to claim 1 or 3, wherein the group –A–B–C is bonded to the 5-position of the imidazo[1,5-a]pyridine nucleus.

5. Compounds of the general formula I, and 5,6,7,8-tetrahydro derivatives thereof, according to claim 3, wherein the group –A–B–C is bonded to the 5-position of the imidazo[1,5-a]pyridine nucleus and B represents lower alkylene-(thio or oxy)-lower alkylene of 4 to 10 carbon atoms, phenylene or phenylene-lower alkenylene of 8 to 10 carbon atoms.

6. Compounds according to claim 1 of the general formula II

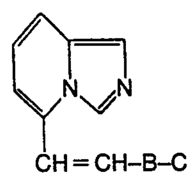

(II)

wherein B represents alkylene or alkenylene of 2 to 4 carbon atoms each, C represents carboxy or lower alkoxycarbonyl, and pharmaceutically acceptable salts thereof.

7. Compounds according to claim 1 of the general formula III

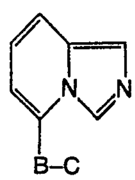

(III)

wherein

B represents lower alkylene-(thio or oxy)-lower alkylene of 4 to 10 carbon atoms, phenylene, phenylene-lower alkylene or lower alkylene-phenylene of 7 to 10 carbon atoms each, alkylenethiophenylene or alkyleneoxyphenylene of 7 to 10 carbon atoms each or phenylene-lower alkenylene of 8 to 10 carbon atoms,

C represents carboxy, lower alkoxycarbonyl or carbamoyl, and pharmaceutically acceptable salts thereof.

8. 5-(4-carboxybuta-1,3-dienyl)-imidazo[1,5-a]-pyridine according to claim 1 and pharmaceutically acceptable salts thereof.

9. 5-[p-(2-carboxyprop-1-enyl)phenyl]-imidazo[1,5-a]pyridine according to claim 1 and pharmaceutically acceptable salts thereof.

10. 5-(9-carboxynona-6,8-dienyl)-imidazo[1,5-a]pyridine according to claim 1 and pharmaceutically acceptable salts thereof.

11. Pharmaceutical compositions containing a compound according to any one of claims 1 to 10, or a pharmaceutically acceptable salt thereof, together with a pharmaceutical carrier.

12. The compounds of claims 1 to 10 for use in a method for the therapeutic treatment of the human or animal body.

13. The compounds of claims 1 to 10 for use as inhibitors of thromboxane synthetase.

14. The use of the compounds of claims 1 to 10 for the preparation of pharmaceutical compositions.

15. A process for the manufacture of compounds of the general formula I according to claim 1, wherein $R_2$ represents hydrogen or lower alkyl, characterised in that a compound of the formula IV

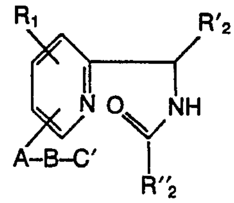

(IV)

wherein $R_2'$ and $R_2''$ represent hydrogen or one of the radicals $R_2'$ and $R_2''$ represents lower alkyl, $R_1$, A and B have the meanings given under formula I and C′ represents carboxy, lower alkoxycarbonyl, unsubstituted or mono- or di-lower alkyl-substituted carbamoyl, cyano, hydroxymethyl, lower alkanoyloxymethyl, etherified hydroxymethyl, halomethyl, 5-tetrazolyl, unsubstituted 4,5-dihydro-2-oxazolyl or 4,5-dihydro-2-oxazolyl substituted by lower alkyl, or hydroxycarbamoyl, halogen or $C_1$–$C_2$-alkylenedioxymethyl, is cyclised, and a resulting compound of the formula Ia

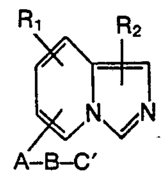

(Ia)

wherein C′ differs from C is converted into a compound of the formula I.

16. A process for the manufacture of compounds of the formula I according to claim 1, wherein A represents unsubstituted ethenylene or ethenylene substituted by lower alkyl, characterised in that a compound of the formula VII

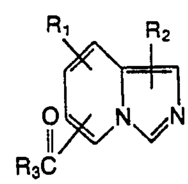

(VII)

wherein $R_1$ and $R_2$ have the meanings given under formula I and $R_3$ represents hydrogen or lower alkyl, is condensed with a compound of the formula VIII

$R_5$
|
$R_4$–CH–B–C′ (VIII)

wherein $R_4$ represents hydrogen or lower alkyl, $R_5$ represents di-lower alkylphosphono or triarylphosphoranyl, C′ represents carboxy, lower alkoxycarbonyl, carbamoyl, mono- or di-lower alkyl-substituted carbamoyl, cyano, halomethyl, lower alkanoyloxymethyl, etherified hydroxymethyl, 5-tetrazolyl, unsubstituted 4,5-dihydro-2-oxazolyl or 4,5-dihydro-2-oxazolyl substituted by lower alkyl, or hydroxycarbamoyl, and B is as defined under

formula I, and a resulting compound wherein C′ differs from C ist converted into a compound of the formula I.

17. A process for the manufacture of compounds of the formula I according to claims 1 and 3, wherein $R_1$ and $R_2$ represent hydrogen or lower alkyl, A represents a single bond, B represents $C_1$–$C_4$-alkylenephenylene-$C_1$–$C_4$-alkylene, $C_1$–$C_4$-alkylenephenylene, lower alkylene-(thio or oxy)-lower alkylene of 2 to 12 carbon atoms in total, $C_1$–$C_4$-alkylene-(thio or oxy)-phenylene, $C_1$–$C_4$-alkylenephenylene-$C_2$–$C_4$-alkenylene, or alkadienylene of 5 to 12 carbon atoms, characterised in that a compound of the formula IX

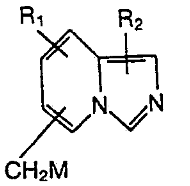

(IX)

wherein M represents an alkali metal, and $R_1$ and $R_2$ represent hydrogen or lower alkyl, is reacted with a reactive functional derivative of a compound of the formula X

HO–B′–C′ (X)

wherein B′ represents $C_1$–$C_4$-alkylenephenylene-$C_1$–$C_4$-alkylene, $C_1$–$C_4$-alkylenephenylene, lower alkylene-(thio or oxy)-lower alkylene of 2 to 12 carbon atoms in total, $C_1$–$C_4$-alkylene-(thio or oxy)-phenylene, $C_1$–$C_4$-alkylenephenylene-$C_2$–$C_4$-alkenylene or $C_1$–$C_7$-alkadienylene, C′ represents carboxy, tri-($C_1$–$C_4$-alkoxy)-methyl, unsubstituted or mono- or di-lower alkyl-substituted carbamoyl, cyano, lower alkanoyloxymethyl, etherified hydroxymethyl, halomethyl, unsubstituted 4,5-dihydro-2-oxazolyl or 4,5-dihydro-2-oxazolyl substituted by lower alkyl, or 5-tetrazolyl or hydroxycarbamoyl, and a resulting compound of the formula Ib

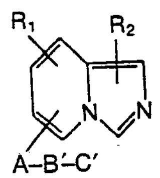

(Ib)

wherein A, B′, C′, $R_1$ and $R_2$ have the meanings specified above and C′ differs from C is converted into a compound of the formula I.

18. A process for the manufacture of compounds of the formula I according to claim 1, wherein B represents $C_1$–$C_4$-alkylene-(thio or oxy)-phenylene, phenylene-(thio or oxy)-$C_1$–$C_4$-alkylene or lower alkylene-(thio or oxy)-lower alkylene of 2 to 12 carbon atoms in total, characterised in that an intermediate of the formula Ia wherein B represents $C_1$–$C_7$-alkylene or phenylene and C′ represents reactive functionally modified hydroxymethyl is reacted with a $C_1$–$C_7$-alkanol, a $C_1$–$C_7$-alkanethiol, a phenol or thiophenol each of which is substituted by C or C′, and a resulting compound wherein C′ differs from C is converted into a compound of the formula I.

19. A process for the manufacture of compounds of the formula I, or 5,6,7,8-tetrahydro derivatives thereof, according to claim 1, characterised in that a resulting compound of the formula I is converted into a different compound of the invention, and/or, if desired, a resulting free compound is converted into a salt or a resulting salt is converted into the free compound or into a different salt, and/or, if desired, a resulting mixture of isomers or racemates is separated into the individual isomers or racemates, and/or, if desired, resulting racemates are resolved into the optical antipodes.

20. A process for the manufacture of 5,6,7,8-tetrahydroimidazo[1,5-a]pyridines according to claim 1, or salts thereof, characterised in that a corresponding imidazo[1,5-a]pyridine of the formula I is reduced with hydrogen.

21. A process according to claim 20 for the manufacture of 5,6,7,8-tetrahydroimidazo[1,5-a]pyridines according to claim 1, or salts thereof, characterised in that a corresponding imidazo-[1,5-a]pyridine of the formula I is reduced with hydrogen in the presence of hydrogenation catalysts.

22. The compounds obtainable by the process of claims 15 to 21.

**Claims for the Contracting State: AT**

1. A process for the manufacture of imidazo[1,5-a]pyridine derivatives of the general formula I

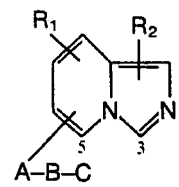

(I)

wehrein

$R_1$ represents hydrogen, halogen, lower alkyl, lower alkoxy, hydroxy or aryl-lower alkoxy wherein aryl is phenyl or phenyl mono- or di-substituted by lower alkoxy, lower alkyl, halogen or by trifluoromethyl,

$R_2$ represents hydrogen or lower alkyl,

C represents carboxy, lower alkoxycarbonyl, unsubstituted or mono- or di-lower alkyl-substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl, and

(a) A represents unsubstituted ethenylene (vinylene) or ethenylene (vinylene) substituted by lower alkyl and B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene, phenylene-(thio or oxy)-$C_1$–$C_4$-alkylene, or

(b) A represents a bond and B represents $C_1$–$C_4$-alkylenephenylene, phenylene-$C_1$–$C_4$-alkylene, phenylene, lower alkylene-(thio or oxy)-lower alkylene of 2 to 12 carbon atoms in total, $C_1$–$C_4$-alkylene-(thio or oxy)-phenylene, phenylene-(thio

or oxy)-$C_1$–$C_4$-alkylene, phenylene-$C_2$–$C_4$-alkenylene, $C_1$–$C_4$-alkylenephenylene-$C_2$–$C_4$-alkenylene, or alkadienylene of 5 to 12 carbon atoms, or salts thereof,
wherein the radicals referred to as "lower" have at most 7 carbon atoms, characterised in that a compound of the formula IV

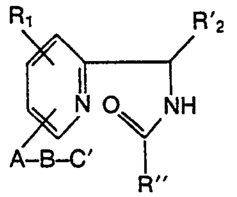

(IV)

wherein $R_2'$ and $R_2''$ represent hydrogen or one of the radicals $R_2'$ and $R_2''$ represents lower alkyl, $R_1$, A and B have the meanings given under formula I and C' represents carboxy, lower alkoxycarbonyl, unsubstituted or mono- or di-lower alkyl-substituted carbamoyl, cyano, hydroxymethyl, lower alkanoyloxymethyl, etherified hydroxymethyl, halomethyl, 5-tetrazolyl, unsubstituted 4,5-dihydro-2-oxazolyl or 4,5-dihydro-2-oxazolyl substituted by lower alkyl, hydroxycarbamoyl, halogen or $C_1$–$C_2$-alkylenedioxymethyl, is cyclised, and a resulting compound of the formula Ia

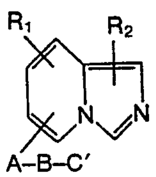

(Ia)

wherein C' differs from C is converted into a compound of the formula I.

2. A process for the manufacture of imidazo[1,5-a]pyridine derivatives of the general formula I

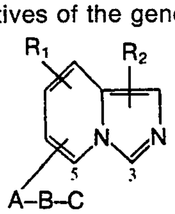

(I)

wherein

$R_1$ represents hydrogen, halogen, lower alkyl, lower alkoxy, hydroxy or aryl-lower alkoxy wherein aryl is phenyl or phenyl mono- or di-substituted by lower alkoxy, lower alkyl, halogen or by trifluoromethyl,

$R_2$ represents hydrogen, halogen or lower alkyl,

C represents carboxy, lower alkoxycarbonyl, unsubstituted or mono- or di-lower alkyl-substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl, and

A represents unsubstituted ethenylene (vinylene) or ethenylene (vinylene) substituted by lower alkyl and B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene or phenylene-(thio or oxy)-$C_1$–$C_4$-alkylene, or salts thereof,
wherein the radicals referred to as "lower" have at most 7 carbon atoms, characterised in that a compound of the formula VII

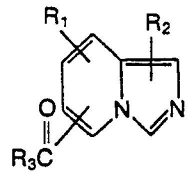

(VII)

wherein $R_1$ and $R_2$ have the meanings given under formula I and $R_3$ represents hydrogen or lower alkyl, is condensed with a compound of the formula VIII

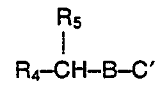

(VIII)

wherein $R_4$ represents hydrogen or lower alkyl, $R_5$ represents di-lower alkylphosphono or triarylphosphoranyl, C' represents carboxy, lower alkoxycarbonyl, carbamoyl, mono- or di-lower alkyl-substituted carbamoyl, cyano, halomethyl, lower alkanoyloxymethyl, etherified hydroxymethyl, 5-tetrazolyl, unsubstituted 4,5-dihydro-2-oxazolyl or 4,5-dihydro-2-oxazolyl substituted by lower alkyl, or hydroxycarbamoyl, and B is as defined under formula I, and a resulting compound wherein C' differs from C is converted into a compound of the formula I.

3. A process for the manufacture of imidazo[1,5-a]pyridine derivatives of the general formula I

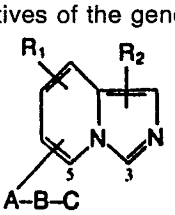

**(I)**

wherein

$R_1$ und $R_2$ represent hydrogen or lower alkyl,

C represents carboxy, lower alkoxycarbonyl, unsubstituted or mono- or di-lower alkyl-substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl and

A represents a single bond and

B represents $C_1$–$C_4$-alkylenephenylene-$C_1$–$C_4$-alkylene, $C_1$–$C_4$-alkylenephenylene, lower alkylene-(thio or oxy)-lower alkylene of 2 to 12 carbon atoms in total, $C_1$–$C_4$-alkylene-(thio or oxy)-phenylene, $C_1$–$C_4$-alkylenephenylene-$C_2$–$C_4$-alkenylene, or alkadienylene of 5 to 12 carbon atoms, or salts thereof, wherein the radicals referred to as "lower" have at most 7 carbon atoms, characterised in that a compound of the formula IX

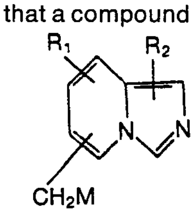

(IX)

wherein M represents an alkali metal, and $R_1$ and $R_2$ represent hydrogen or lower alkyl, is reacted with a reactive functional derivative of a compound of the formula X

$$HO–B'–C' \qquad (X),$$

wherein B' represents $C_1$–$C_4$-alkylenephenylene-$C_1$–$C_4$-alkylene, $C_1$–$C_4$-alkylenephenylene, lower alkylene-(thio or oxy)-lower alkylene of 2 to 12 carbon atoms in total, $C_1$–$C_4$-alkylene-(thio or oxy)-phenylene, $C_1$–$C_4$-alkylenephenylene-$C_2$–$C_4$-alkenylene or $C_1$–$C_7$-alkadienylene, C' represents carboxy, tri($C_1$–$C_4$-alkoxy)-methyl, unsubstituted or mono- or di-lower alkyl-substituted carbamoyl, cyano, lower alkanoyloxymethyl, etherified hydroxymethyl, halomethyl, unsubstituted 4,5-dihydro-2-oxazolyl or 4,5-dihydro-2-oxazolyl substituted by lower alkyl, 5-tetrazolyl or hydroxycarbamoyl, and a resulting compound of the formula Ib

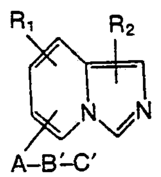
(Ib)

wherein A, B', C', $R_1$ and $R_2$ have the meanings specified above and C' differs from C is converted into a compound of the formula I.

4. A process for the manufacture of imidazo[1,5-a]pyridine derivatives of the general formula I

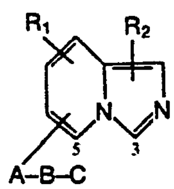
(I)

wherein

$R_1$ represents hydrogen, halogen, lower alkyl, lower alkoxy, hydroxy or aryl-lower alkoxy wherein aryl is phenyl or phenyl mono- or di-substituted by lower alkoxy, lower alkyl, halogen or by trifluoromethyl,

$R_2$ represents hydrogen, halogen or lower alkyl,

C represents carboxy, lower alkoxycarbonyl, unsubstituted or mono- or di-lower alkyl, substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl and

(a) A represents unsubstituted ethenylene (vinylene) or ethenylene (vinylene) substituted by lower alkyl and B represents phenylene-(thio or oxy)-$C_1$–$C_4$-alkylene, or

(b) A represents a bond and B represents $C_1$–$C_4$-alkylene-(thio or oxy)-phenylene, phenylene-(thio or oxy)-$C_1$–$C_4$-alkylene or lower alkylene-(thio or oxy)-lower alkylene of 2 to 12 carbon atoms in total, or salts thereof, wherein the radicals referred to as "lower" have at most 7 carbon atoms, characterised in that an intermediate of the formula Ia wherein B represents $C_1$–$C_7$-alkylene or phenylene and C' represents reactive functionally modified hydroxymethyl is reacted with a $C_1$–$C_7$-alkanol, a $C_1$–$C_7$-alkanethiol, a phenol or thiophenol each of which is substituted by C or C', and a resulting compound wherein C' differs from C is converted into a compound of the formula I.

5. A process for the manufacture of imidazo[1,5-a]pyridine derivatives of the general formula I

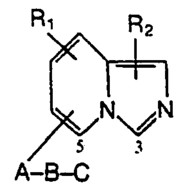
(I)

or 5,6,7,8-tetrahydro derivatives thereof, wherein $R_1$ represents hydrogen, halogen, lower alkyl, lower alkoxy, hydroxy or aryl-lower alkoxy wherein aryl is phenyl or phenyl mono- or di-substituted by lower alkoxy, lower alkyl, halogen or by trifluoromethyl, $R_2$ represents hydrogen, halogen or lower alkyl, C represents carboxy, lower alkoxycarbonyl, unsubstituted or mono- or di-lower alkyl-substituted carbamoyl, cyano, formyl, hydroxymethyl, 5-tetrazolyl, unsubstituted 4,5-dihydro-2-oxazolyl or 4,5-dihydro-2-oxazolyl substituted by lower alkyl, or hydroxycarbamoyl and

(a) A represents unsubstituted ethenylene (vinylene) or ethenylene (vinylene) substituted by lower alkyl and B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene, phenylene-(thio or oxy)-$C_1$–$C_4$-alkylene, or

(b) A represents a bond and B represents $C_1$–$C_4$-alkylenephenylene, phenylene-$C_1$–$C_4$-alkylene, phenylene, lower alkylene-(thio or oxy)-lower alkylene of 2 to 12 carbon atoms in total, $C_1$–$C_4$-alkylene-(thio or oxy)-phenylene, phenylene-(thio or oxy)-$C_1$–$C_4$-alkylene, phenylene-$C_2$–$C_4$-alkenylene, $C_1$–$C_4$-alkylenephenylene-$C_2$–$C_4$-alkenylene, or alkadienylene of 5 to 12 carbon atoms, or salts thereof, wherein the radicals referred to as "lower" have at most 7 carbon atoms, characterised in that a resulting compound of the formula I, or a 5,6,7,8-tetrahydro derivative thereof, is converted into a different compound of the invention, and/or, if desired, a resulting free compound is converted into a salt or a resulting salt is converted into the free compound or into a different salt, and/or, if desired, a resulting mixture of isomers or racemates is separated into the individual isomers or racemates, and/or, if desired, resulting racemates are resolved into the optical antipodes.

6. A process for the manufacture of 5,6,7,8-tetrahydroimidazo[1,5-a]pyridines according to claim 5, or salts thereof, characterised in that a corresponding imidazo[1,5-a]pyridine of the formula I is reduced with hydrogen.

7. A process according to claim 6 for the manufacture of 5,6,7,8-tetrahydroimidazo[1,5-a]pyridines according to claim 5, or salts thereof, characterised in that a corresponding imidazo[1,5-a]pyridine of the formula I is reduced with

hydrogen in the presence of hydrogenation catalysts.

8. A process according to any one of claims 1 and 2 or 4 and 5, characterised in that compounds of the general formula I wherein $R_1$ represents hydrogen, halogen, lower alkyl, lower alkoxy, hydroxy, phenyl-lower alkoxy or phenyl-lower alkoxy mono- or di-substituted on the phenyl ring by lower alkoxy, lower alkyl or by halogen, A represents ethenylene or ethenylene substituted by lower alkyl, B represents alkylene of 1 to 12 carbon atoms, alkynylene or alkenylene of 2 to 12 carbon atoms each, phenylene-$C_1$–$C_4$-alkylene, phenylene-$C_2$–$C_4$-alkenylene, phenylene or phenylene-(thio or oxy)-$C_1$–$C_4$-alkylene, and $R_2$ and C are as defined in claim 1, 2, 4 or 5, or salts thereof, are manufactured.

9. A process according to any one of claims 1 or 3 to 7, characterised in that compounds of the general formula I, or 5,6,7,8-tetrahydro derivatives thereof, wherein

$R_1$ represents hydrogen, halogen, lower alkyl, lower alkoxy, hydroxy or aryl-lower alkoxy wherein aryl is unsubstituted phenyl or phenyl mono- or di-substituted by lower alkoxy, lower alkyl, halogen or by trifluoromethyl,

$R_2$ represents hydrogen, halogen or lower alkyl,
C represents carboxy, lower alkoxycarbonyl, unsubstituted or mono- or di-lower alkyl-substituted carbamoyl, cyano, hydroxymethyl, formyl, 5-tetrazolyl, unsubstituted 4,5-dihydro-2-oxazolyl or 4,5-dihydro-2-oxazolyl substituted by lower alkyl, or hydroxycarbamoyl,

A represents a single bond and
B represents $C_1$–$C_4$-alkylenephenylene-$C_1$–$C_4$-alkylene, $C_1$–$C_4$-alkylenephenylene, phenylene-$C_1$–$C_4$-alkylene, phenylene, lower alkylene-(thio or oxy)-lower alkylene of 2 to 12 carbon atoms in total, $C_1$–$C_4$-alkylene-(thio or oxy)-phenylene, phenylene-(thio or oxy)-$C_1$–$C_4$-alkylene, phenylene-$C_2$–$C_4$-alkenylene, $C_1$–$C_4$-alkylenephenylene-$C_2$–$C_4$-alkenylene, or alkadienylene of 5 to 12 carbon atoms, or salts thereof, are manufactured.

10. A process according to any one of claims 1 to 9, characterised in that compounds of the general formula I, or 5,6,7,8-tetrahydro derivatives thereof, wherein the group –A–B–C is bonded to the 5-position of the imidazo[1,5-a]pyridine nucleus are manufactured.

11. A process according to claim 9, characterised in that compounds of the general formula I, or 5,6,7,8-tetrahydro derivatives thereof, wherein the group –A–B–C is bonded to the 5-position of the imidazo[1,5-a]-pyridine nucleus and B represents lower alkylene-(thio or oxy)-lower alkylene of 4 to 10 carbon atoms, phenylene or phenylene-lower alkenylene of 8 to 10 carbon atoms are manufactured.

12. A process according to any one of claims 1 and 2 or 5, characterised in that compounds of the general formula II

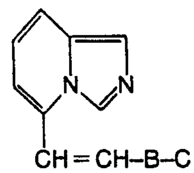

(II)

wherein B represents alkylene or alkenylene of 2 to 4 carbon atoms each, C represents carboxy or lower alkoxycarbonyl, or pharmaceutically acceptable salts thereof, are manufactured.

13. A process according to any one of claims 1 or 3 to 5, characterised in that compounds of the general formula III

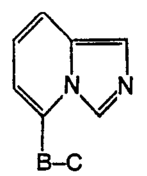

(III)

wherein B represents lower alkylene-(thio or oxy)-lower alkylene of 4 to 10 carbon atoms, phenylene, phenylene-lower alkylene or lower alkylene-phenylene of 7 to 10 carbon atoms each, alkylenethiophenylene or alkyleneoxyphenylene of 7 to 10 carbon atoms each or phenylene-lower alkenylene of 8 to 10 carbon atoms, C represents carboxy, lower alkoxycarbonyl or carbamoyl, or pharmaceutically acceptable salts thereof, are manufactured.

14. A process according to any one of claims 1 and 2 or 5, characterised in that 5-(4-carboxybuta-1,3-dienyl)-imidazo[1,5-a]pyridine, or a pharmaceutically acceptable salt thereof, is manufactured.

15. A process according to either one of claims 1 or 5, characterised in that 5-[p-(2-carboxyprop-1-enyl)-phenyl]-imidazo[1,5-a]pyridine, or a pharmaceutically acceptable salt thereof, is manufactured.

16. A process according to any one of claims 1, 3 or 5, characterised in that 5-(9-carboxynona-6,8-dienyl)-imidazo[1,5-a]pyridine, or a pharmaceutically acceptable salt thereof, is manufactured.

17. A process for the preparation of pharmaceutical composition, characterised by the processing of an active ingredient according to the invention according to any one of claims 1 to 16 with a pharmaceutical carrier.

**Revendications pour les Etats Contractants: BE, CH, DE, FR, GB, IT, LI, LU, NL, SE**

1. Dérivés de l'imidazo[1,5-a]pyridine de formule générale I

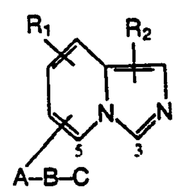

(I)

et leurs dérivés 5,6,7,8-tétrahydrogénés, dans lesquels

$R_1$ représente l'hydrogène, un halogène, un groupe alkyle inférieur, alcoxy inférieur, hydroxy ou aryl-alcoxy inférieur dans lequel le groupe aryle est un groupe phényle ou phényle mono- ou di-substitué par des groupes alcoxy inférieurs, alkyle inférieurs, des halogènes ou des groupes trifluorométhyle,

$R_2$ représente l'hydrogène, un halogène ou un groupe alkyle inférieur,

C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par un groupe alkyle inférieur, ou hydroxycarbamoyle, et

a) A représente un groupe éthénylène (vinylène) non substitué ou substitué par un groupe alkyle inférieur et B représente un groupe alkylène en C 1–C 12, alcynylène ou alcénylène en C 2–C 12, phénylène-alkylène en C 1–C 4, phénylène-alcénylène en C 2–C 4, phénylène, phénylène-(thio ou oxy)-alkylène en C 1–C 4, ou bien

b) A représente une liaison et B un groupe (alkylène en C 1–C 4)-phénylène, phénylène-alkylène en C 1–C 4, phénylène, (alkylène inférieur)-(thio ou oxy)-alkylène inférieur contenant au total 2 à 12 atomes de carbone, (alkylène en C 1–C 4)-(thio ou oxy)-phénylène, phénylène-(thio ou oxy)-alkylène en C 1–C 4, phénylène-alcénylène en C 2–C 4, (alkylène en C 1–C 4)-phénylène-alcénylène en C 2–C 4 ou alcadiénylène en C 5–C 12, et leurs sels, les groupes qualifiés d'«inférieurs» contenant au maximum 7 atomes de carbone.

2. Composés de formule générale I selon la revendication 1, dans lesquels

$R_1$ représente l'hydrogène, un halogène, un groupe alkyle inférieur, alcoxy inférieur, hydroxy, phényl-alcoxy inférieur ou phényl-alcoxy inférieur mono- ou di-substitué dans le cycle phényle par des groupes alcoxy inférieurs, alkyle inférieurs ou des halogènes,

A représente un groupe éthénylène ou éthénylène substitué par un groupe alkyle inférieur,

B représente un groupe alkylène en C 1–C 12, alcynylène ou alcénylène en C 2–C 12, phénylène-alkylène en C 1–C 4, phénylène-alcénylène en C 2–C 4, phénylène ou phénylène-(thio ou oxy)-alkylène en C 1–C 4, et

$R_2$ et C ont les significations indiquées dans la revendication 1, et leurs sels.

3. Composés de formule générale I

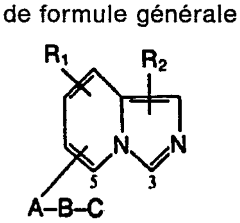

(I)

et leurs dérivés 5,6,7,8-tétrahydrogénés, dans lesquels $R_1$ représente l'hydrogène, un halogène, un groupe alkyle inférieur, alcoxy inférieur, hydroxy ou aryl-alcoxy inférieur dans lequel le groupe aryle est un groupe phényle non substitué ou mono- ou di-substitué par des groupes alcoxy inférieurs, alkyle inférieurs, des halogènes ou des groupes trifluorométhyle, $R_2$ représente l'hydrogène, un halogène ou un groupe alkyle inférieur, C représente un groupe carboxy, (alcoxy inférieur)-carbonyle, carbamoyle non substitué ou mono- ou di-substitué par des groupes alkyle inférieurs, cyano, hydroxyméthyle, formyle, 5-tétrazolyle, 4,5-dihydro-2-oxazolyle non substitué ou substitué par un groupe alkyle inférieur, ou hydroxycarbamoyle, A représente une liaison simple et B un groupe (alkylène en C 1–C 4)-phénylène-alkylène en C 1–C 4, (alkylène en C 1–C 4)-phénylène, phénylènealkylène en C 1–C 4, phénylène, (alkylène inférieur)-(thio ou oxy)-alkylène inférieur contenant au total 2 à 12 atomes de carbone, (alkylène en C 1–C 4)-thio ou oxy)-phénylène, phénylène-(thio ou oxy)-alkylène en C 1–C 4, phénylène-alcényle en C 2–C 4, (alkylène en C 1–C 4)-phénylène-alcénylène en C 2–C 4 ou alcadiénylène en C 5–C 12, et leurs sels, les groupes qualifiés d'«inférieurs» contenant au maximum 7 atomes de carbone.

4. Composés de formule générale I et leurs dérivés 5,6,7,8-tétrahydrogénés selon revendication 1 ou 3, dans lesquels le groupe –A–B–C est fixé en position 5 du cycle imidazo[1,5-a]pyridine.

5. Composés de formule générale I et leurs dérivés 5,6,7,8-tétrahydrogénés selon la revendication 3, dans lesquels le groupe –A–B–C est fixé en position 5 du cycle imidazo[1,5-a]pyridine et B représente un groupe (alkylène inférieur)-(thio ou oxy)-alkylène inférieur en C 4–C 10, phénylène ou phénylène-alcénylène inférieur en C 8–C 10.

6. Composés selon la revendication 1, répondant à la formule générale II

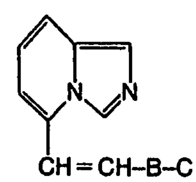

(II)

dans laquelle B représente un groupe alkylène ou alcénylène contenant chacun 2 à 4 atomes de carbone, C représente un groupe carboxy ou (alcoxy inférieur)-carbonyle et leurs sels acceptables pour l'usage pharmaceutique.

7. Composés selon la revendication 1, de formule générale III

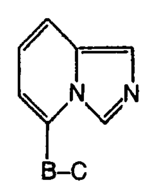

(III)

dans laquelle

B représente un groupe (alkylène inférieur)-(thio ou oxy)-alkylène inférieur en C 4–C 10, phénylène, phénylène-alkylène inférieur ou (alkylène inférieur)-phénylène contenant chacun 7 à

10 atomes de carbone, alkylène-thiophénylène ou alkylène-oxyphénylène contenant chacun 7 à 10 atomes de carbone ou phénylène-alcénylène inférieur en C 8–C 10,

C représente un groupe carboxy, (alcoxy inférieur)-carbonyle ou carbamoyle, et leurs sels acceptables pour l'usage pharmaceutique.

8. La 5-(4-carboxybuta-1,3-diényl)-imidazo[1,5-a]pyridine selon la revendication 1 et ses sels acceptables pour l'usage pharmaceutique.

9. La 5-[p-(2-carboxypropa-1-ényl)-phényl]imidazo[1,5-a]pyridine selon la revendication 1 et ses sels acceptables pour l'usage pharmaceutique.

10. La 5-(9-carboxynona-6,8-diényl)-imidazo[1,5-a]pyridine selon la revendication 1 et ses sels acceptables pour l'usage pharmaceutique.

11. Compositions pharmaceutiques contenant un composé selon l'une des revendications 1 à 10 ou un sel d'un tel composé acceptable pour l'usage pharmaceutique, avec un véhicule pharmaceutique.

12. Les composés des revendications 1 à 10, pour l'utilisation dans un procédé pour le traitement thérapeutique de l'organisme humain ou animal.

13. Les composés des revendications 1 à 10, pour l'utilisation en tant qu'inhibiteurs de la thromboxanne-synthétase.

14. Utilisation des composés selon les revendications 1 à 10, pour la préparation de compositions pharmaceutiques.

15. Procédé de préparation des composés de formule générale I selon la revendication 1, dans lesquels $R_2$ représente l'hydrogène ou un groupe alkyle inférieur, caractérisé en ce que l'on cyclise un composé de formule IV

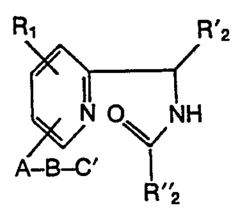

(IV)

dans laquelle $R_2'$ et $R_2''$ représentent l'hydrogène ou bien l'un des symboles $R_2'$ et $R_2''$ représentent un groupe alkyle inférieur, $R_1$, A et B ont les significations indiquées en référence à la formule I et C' représente un groupe carboxy, (alcoxy inférieur)-carbonyle, carbamoyle non substitué, mono- ou di-substitué par des groupes alkyle inférieurs, cyano, hydroxyméthyle, (alcanoyle inférieur)-oxyméthyle, hydroxyméthyle éthérifié, halogénométhyle, 5-tétrazolyle, 4,5-dihydro-2-oxazolyle non substitué ou substitué par un groupe alkyle inférieur, hydroxycarbamoyle, un halogène ou un groupe (alkylène en C 1–C 2)-dioxyméthyle, ce qui donne un composé de formule Ia

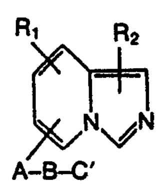

(Ia)

dans laquelle C' est différent de C, qu'on convertit en un composé de formule I.

16. Procédé de préparation des composés de formule I selon la revendication 1, dans lesquels A représente un groupe éthénylène non substitué ou substitué par un groupe alkyle inférieur, caractérisé en ce que l'on condense un composé de formule VII

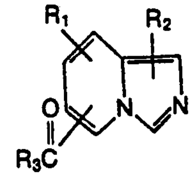

(VII)

dans laquelle $R_1$ et $R_2$ ont les significations indiquées en référence à la formule I et $R_3$ représente l'hydrogène ou un groupe alkyle inférieur, avec un composé de formule VIII

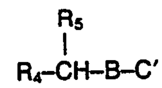

(VIII)

dans laquelle $R_4$ représente l'hydrogène ou un groupe alkyle inférieur, $R_5$ représente un groupe di-(alkyle inférieur)-phosphono ou triarylphosphoranyle, C' représente un groupe carboxy, (alcoxy inférieur)-carbonyle, carbamoyle, carbamoyle mono- ou di-substitué par des groupes alkyle inférieurs, cyano, halogénométhyle, (alcanoyle inférieur)-oxyméthyle, hydroxyméthyle éthérifié, 5-tétrazolyle, 4,5-dihydro-2-oxazolyle non substitué ou substitué par un groupe alkyle inférieur, ou hydroxy carbamoyle, et B a les significations indiquées en référence à la formule I, et on convertit le composé obtenu dans lequel C' est différent de C en un composé de formule I.

17. Procédé de préparation des composés de formule I selon les revendications 1 et 3, dans lesquelles $R_1$ et $R_2$ représentent l'hydrogène ou un groupe alkyle inférieur, A une liaison simple, B un groupe (alkylène en C 1–C 4)-phénylène-alkylène en C 1–C 4, (alkylène en C 1–C 4)-phénylène, (alkylène inférieur)-(thio ou oxy)-alkylène inférieur contenant au total 2 à 12 atomes de carbone, (alkylène en C 1–C 4)-(thio ou oxy)-phénylène, (alkylène en C 1–C 4)-phénylène-alcénylène en C 2–C 4 ou alcadiénylène en C 5–C 12, caractérisé en ce que l'on fait réagir un composé de formule IX

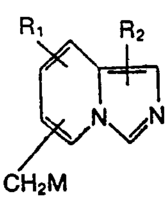

(IX)

dans laquelle M représente un métal alcalin, $R_1$ et $R_2$ représentent l'hydrogène ou des groupes alkyle inférieurs, avec un dérivé fonctionnel réactif d'un composé de formule X

HO–B'–C' (X)

dans laquelle B' représente un groupe (alkylène en C 1–C 4)-phénylène-alkylène en C 1–C 4, (alkylène en C 1–C 4)-phénylène, (alkylène inférieur)-(thio ou oxy)-alkylène inférieur contenant au total 2 à 12 atomes de carbone, (alkylène en C 1–C 4)-(thio ou oxy)-phénylène, (alkylène en C 1–C 4)-phénylène-alcénylène en C 2–C 4 ou alcadiénylène en C 1–C 7, C′ représente un groupe carboxy, tri-(alcoxy en C 1–C 4)-méthyle, carbamoyle non substitué, mono- ou di-substitué par des groupes alkyle inférieurs, cyano, (alcanoyle inférieur)-oxyméthyle, hydroxyméthyle éthérifié, halogénométhyle, 4,5-dihydro-2-oxazolyle non substitué ou substitué par un groupe alkyle inférieur, 5-tétrazolyle ou hydroxycarbamoyle, ce qui donne un composé de formule Ib

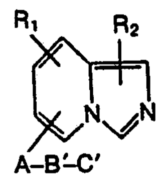
**(Ib)**

dans laquelle A, B′, C′, $R_1$ et $R_2$ ont les significations indiquées ci-dessus et C′ est différent de C, qu'on convertit en un composé de formule I.

18. Procédé de préparation des composés de formule I selon la revendication 1, dans lesquels B représente un groupe (alkylène en C 1–C 4)-(thio ou oxy)-phénylène, phénylène-(thio ou oxy)-alkylène en C 1–C 4 ou (alkylène inférieur)-(thio ou oxy)-alkylène inférieur contenant au total 2 à 12 atomes de carbone, caractérisé en ce que l'on fait réagir un produit intermédiaire de formule Ia dans laquelle B représente un groupe alkylène en C 1–C 7 ou phénylène et C′ représente un groupe hydroxyméthyle ayant subi une modification fonctionnelle et réactif, avec un alcanol en C 1–C 7, un alcane-thiol en C 1–C 7, un phénol ou un thiophénol, substitués chacun par C ou C′, et on convertit un composé obtenu, dans lequel C′ est différent de C, en un composé de formule I.

19. Procédé de préparation des composés de formule I ou de leurs dérivés 5,6,7,8-tétrahydrogénés selon la revendication 1, caractérisé en ce que l'on convertit un composé obtenu répondant à la formule I en un autre composé de l'invention et/ou, si on le désire, on convertit un composé obtenu à l'état libre en un sel ou un sel obtenu en le composé libre ou en un autre sel et/ou si on le désire, on sépare un mélange d'isomères ou de racémates obtenu en les isomères ou racémates individuels et/ou, si on le désire, on résout les racémates obtenus en les antipodes optiques.

20. Procédé de préparation des 5,6,7,8-tétrahydroimidazo[1,5-a]pyridines selon la revendication 1, ou de leurs sels, caractérisé en ce que l'on réduit par l'hydrogène les imidazo[1,5-a]pyridines correspondantes de formule I.

21. Procédé selon la revendication 20, pour la préparation des 5,6,7,8-tétrahydroimidazo[1,5-a]pyridines selon la revendication 1, ou de leurs sels, caractérisé en ce que l'on réduit par l'hydrogène, en présence de catalyseurs d'hydrogénation, les imidazo[1,5-a]pyridines correspondantes de formule I.

22. Les composés obtenus par le procédé des revendications 15 à 21.

**Revendications pour l'Etat Contractant: AT**

1. Procédé de préparation des dérivés de l'imidazo[1,5-a]pyridine de formule générale I

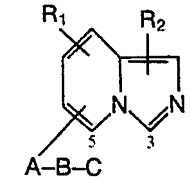
(I)

dans laquelle

$R_1$ représente l'hydrogène, un halogène, un groupe alkyle inférieur, alcoxy inférieur, hydroxy ou aryl-alcoxy inférieur dans lequel le groupe aryle est un groupe phényle ou un groupe phényle mono- ou di-substitué par des groupes alcoxy inférieurs, alkyle inférieurs, des halogènes ou des groupes trifluorométhyle,

$R_2$ représente l'hydrogène ou un groupe alkyle inférieur,

C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par un groupe alkyle inférieur, ou hydroxycarbamoyle, et

a) A représente un groupe éthénylène (vinylène) non substitué ou substitué par un groupe alkyle inférieur et B un groupe alkylène en C 1–C 12, alcynylène ou alcénylène en C 2–C 12, phénylène-alkylène en C 1–C 4, phénylène-alcénylène en C 2–C 4, phénylène, phénylène-(thio ou oxy)-alkylène en C 1–C 4, ou bien

b) A représente une liaison et B un groupe (alkylène en C 1–C 4)-phénylène, phénylène-alkylène en C 1–C 4, phénylène, (alkylène inférieur)-(thio ou oxy)-alkylène inférieur contenant au total 2 à 12 atomes de carbone, (alkylène en C 1–C 4)-(thio ou oxy)-phénylène, phénylène-(thio ou oxy)-alkylène en C 1–C 4, phénylène-alcénylène en C 2–C 4, (alkylène en C 1–C 4)-phénylène-alcénylène en C 2–C 4 ou alcadiénylène en C 5–C 12, ou de leurs sels, les groupes qualifiés d'«inférieurs» contenant au maximum 7 atomes de carbone,

caractérisé en ce que l'on cyclise un composé de formule IV

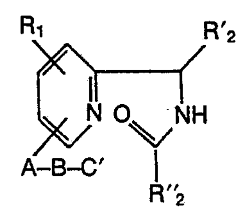
(IV)

dans laquelle $R_2'$ et $R_2''$ représentent l'hydrogène ou bien l'un des symboles $R_2'$ et $R_2''$ représente un groupe alkyle inférieur, $R_1$, A et B ont les significations indiquées en référence à la formule I et C′ représente un groupe carboxy, (alcoxy inférieur)-carbonyle, carbamoyle non substitué, mono- ou di-substitué par des groupes alkyle inférieurs, cyano, hydroxyméthyle, (alcanoyle inférieur)-oxyméthyle, hydroxyméthyle éthérifié, halogénométhyle, 5-tétrazolyle, 4,5-dihydro-2-oxazolyle non substitué ou substitué par un groupe alkyle inférieur, hydroxycarbamoyle, un halogène ou un groupe (alkylène en C 1–C 2)-dioxyméthyle, ce qui donne un composé de formule Ia

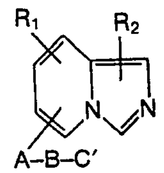

(Ia)

dans laquelle C′ est différent de C, qu'on convertit en un composé de formule I.

2. Procédé de préparation des dérivés de l'imidazo[1,5-a]pyridine de formule générale I

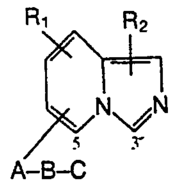

(I)

dans laquelle

$R_1$ représente l'hydrogène, un halogène, un groupe alkyle inférieur, alcoxy inférieur, hydroxy ou aryl-alcoxy inférieur dans lequel le groupe aryle est un groupe phényle ou phényle mono- ou di-substitué par des groupes alcoxy inférieurs, alkyle inférieurs, des halogènes ou des groupes trifluorométhyle,

$R_2$ représente l'hydrogène, un halogène ou un groupe alkyle inférieur,

C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par des groupes alkyle inférieurs, ou hydroxycarbamoyle, et

A représente un groupe éthénylène (vinylène) non substitué ou substitué par un groupe alkyle inférieur et B un groupe alkylène en C 1–C 12, alcynylène ou alcénylène en C 2–C 12, phénylène-alkylène en C 1–C 4, phénylène-alcénylène en C 2–C 4, phénylène ou phénylène-(thio ou oxy)-alkylène en C 1–C 4, ou de leurs sels, les groupes qualifiés d'«inférieurs» contenant au maximum 7 atomes de carbone,

caractérisé en ce que l'on condense un composé de formule VII

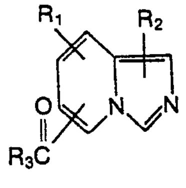

(VII)

dans laquelle $R_1$ et $R_2$ ont les significations indiquées en référence à la formule I et $R_3$ représente l'hydrogène ou un groupe alkyle inférieur, avec un composé de formule VIII

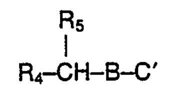

(VIII)

dans laquelle $R_4$ représente l'hydrogène ou un groupe alkyle inférieur, $R_5$ représente un groupe di-(alkyle inférieur)-phosphono ou triarylphosphoranyle, C′ représente un groupe carboxy, (alcoxy inférieur)-carbonyle, carbamoyle, carbamoyle mono- ou di-substitué par des groupes alkyle inférieurs, cyano, halogénométhyle, (alcanoyle inférieur)-oxyméthyle, hydroxyméthyle éthérifié, 5-tétrazolyle, 4,5-dihydro-2-oxazolyle non substitué ou substitué par un groupe alkyle inférieur, ou hydroxycarbamoyle et B a les significations indiquées en référence à la formule I, et on convertit un composé obtenu dans lequel C′ est différent de C en un composé de formule I.

3. Procédé de préparation des dérivés de l'imidazo[1,5-a]pyridine de formule générale I

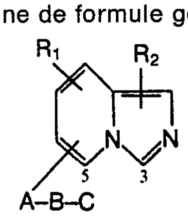

(I)

dans laquelle

$R_1$ et $R_2$ représentent l'hydrogène ou des groupes alkyle inférieurs,

C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par un groupe alkyle inférieur, ou hydroxycarbamoyle, et

A représente une liaison simple, et

B représente un groupe (alkylène en C 1–C 4)-phénylène-alkylène en C 1–C 4, (alkylène en C 1–C 4)-phénylène, (alkylène inférieur)-(thio ou oxy)-alkylène inférieur contenant au total 2 à 12 atomes de carbone, (alkylène en C 1–C 4)-(thio- ou oxy)-phénylène, (alkylène en C 1–C 4)-phénylène-alcénylène en C 2–C 4 ou alcadiénylène en C 5–C 12, ou de leurs sels, les groupes qualifiés d'«infèrieurs» contenant au maximum 7 atomes de carbone, caractérisé en ce que l'on fait réagir un composé de formule IX

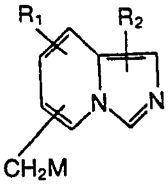

(IX)

dans laquelle M représente un métal alcalin, $R_1$ et $R_2$ l'hydrogène ou des groupes alkyle inférieurs, avec un dérivé fonctionnel réactif d'un composé de formule X

HO–B′–C′ (X)

dans laquelle B′ représente un groupe (alkylène en C 1–C 4)-phénylène-alkylène en C 1–C 4, (alkylène en C 1–C 4)-phénylène, (alkylène inférieur)-(thio ou oxy)-alkylène inférieur contenant au total 2 à 12 atomes de carbone, (alkylène en C 1–C 4)-(thio ou oxy)-phénylène, (alkylène en C 1–C 4)-phénylène-alcénylène en C 2–C 4 ou alcadiénylène en C 1–C 7, C′ représente un groupe carboxy, tri-(alcoxy en C 1–C 4)-méthyle, carbamoyle non substitué, mono- ou di-substitué par des groupes alkyle inférieurs, cyano, (alcanoyle inférieur)-oxyméthyle, hydroxyméthyle éthérifié, halogénométhyle, 4,5-dihydro-2-oxazolyle non substitué ou substitué par un groupe alkyle inférieur, 5-tétrazolyle ou hydroxycarbamoyle, ce qui donne un composé de formule Ib

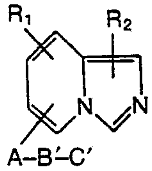

(Ib)

dans laquelle A, B′, C′, $R_1$ et $R_2$ ont les significations indiquées ci-dessus et C′ diffère de C, qu'on convertit en un composé de formule I.

4. Procédé de préparation des dérivés de l'imidazo[1,5-a]pyridine de formule générale I

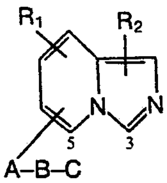

(I)

dans laquelle

$R_1$ représente l'hydrogène, un halogène, un groupe alkyle inférieur, alcoxy inférieur, hydroxy ou aryl-alcoxy inférieur dans lequel le groupe aryle est un groupe phényle ou un groupe phényle mono- ou di-substitué par des groupes alcoxy inférieurs, alkyle inférieurs, des halogènes ou des groupes trifluorométhyle,

$R_2$ représente l'hydrogène, un halogène ou un groupe alkyle inférieur,

C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par un groupe alkyle inférieur, ou hydroxycarbamoyle, et

a) A représente un groupe éthénylène (vinylène) non substitué ou substitué par un groupe alkyle inférieur et B représente un groupe phénylène-(thio ou oxy)-alkylène en C 1–C 4, ou bien

b) A représente une liaison et B un groupe (alkylène en C 1–C 4)-(thio ou oxy)-phénylène, phénylène-(thio ou oxy)-alkylène en C 1–C 4) ou (alkylène inférieur)-(thio ou oxy)-alkylène inférieur contenant au total 2 à 12 atomes de carbone, ou de leurs sels, les groupes qualifiés d'«inférieurs» contenant au maximum 7 atomes de carbone, caractérisé en ce que l'on fait réagir un produit intermédiaire de formule Ia, dans laquelle B représente un groupe alkylène en C 1–C 7 ou phénylène et C′ représente un groupe hydroxyméthyle qui a subi une modification fonctionnelle et qui est réactif, avec un alcanol en C 1–C 7, un alcane-thiol en C 1–C 7, un phénol ou un thiophénol substitués chacun par C ou C′, ce qui donne un composé dans lequel C′ est différent de C qu'on convertit en un composé de formule I.

5. Procédé de préparation des dérivés de l'imidazo[1,5-a]pyridine de formule générale I

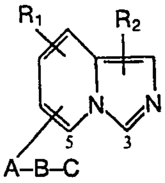

(I)

ou de leurs dérivés 5,6,7,8-tétrahydrogénés, dans lesquels $R_1$ représente l'hydrogène, un halogène, un groupe alkyle inférieur, alcoxy inférieur, hydroxy ou arylalcoxy inférieur dans lequel le groupe aryle est un groupe phényle ou phényle mono- ou di-substitué par des groupes alcoxy inférieurs, alkyle inférieurs, des halogènes ou des groupes trifluorométhyle, $R_2$ représente l'hydrogène, un halogène ou un groupe alkyle inférieur, C représente un groupe carboxy, (alcoxy inférieur)-carbonyle, carbamoyle non substitué, mono- ou di-substitué par des groupes alkyle inférieurs, cyano, formyle, hydroxyméthyle, 5-tétrazolyle, 4,5-dihydro-2-oxazolyle non substitué ou substitué par un groupe alkyle inférieur, ou hydroxycarbamoyle, et

a) A représente un groupe éthénylène (vinylène) non substitué ou substitué par un groupe alkyle inférieur et B représente un groupe alkylène en C 1–C 12, alcynylène ou alcénylène en C 2–C 12, phénylène-alkylène en C 1–C 4, phénylène-alcénylène en C 2–C 4, phénylène, phénylène-(thio ou oxy)-alkylène en C 1–C 4, ou bien

b) A représente une liaison et B représente un groupe (alkylène en C 1–C 4)-phénylène, phénylène-alkylène en C 1–C 4, phénylène, (alkylène inférieur)-(thio ou oxy)-alkylène inférieur contenant au total 2 à 12 atomes de carbone, (alkylène en C 1–C 4)-(thio ou oxy)-phénylène, phénylène-(thio ou oxy)-alkylène en C 1–C 4, phénylène-alcénylène en C 2–C 4, (alkylène en C 1–C 4)-phénylène-alcénylène en C 2–C 4 ou alcadiénylène en C 5–C 12, ou de leurs sels, les groupes qualifiés d'«inférieurs» contenant au maximum 7 atomes de carbone, caractérisé en ce que l'on convertit un composé obtenu, répondant à la formule I, ou un dérivé 5,6,7,8-tétrahydrogéné d'un tel composé, en un autre composé de l'invention et/ou, si on le désire on convertit un composé obtenu à l'état libre en un sel ou un sel obtenu en le composé libre ou en un autre sel, et/ou, si on le désire, on sépare un mélange d'isomères ou de racémates en les isomères ou racémates individuels et/ou, si on le désire, on résout les racémates obtenus en les antipodes optiques.

6. Procédé de préparation des 5,6,7,8-tétrahydroimidazo[1,5-a]pyridines selon la revendication 5 ou de leurs sels, caractérisé en ce que l'on réduit par l'hydrogène une imidazo[1,5-a]pyridine correspondante de formule I.

7. Procédé selon la revendication 6, pour préparer les 5,6,7,8-tétrahydroimidazo[1,5-a]pyridines selon la revendication 5 ou leurs sels, caractérisé en ce que l'on réduit une imidazo[1,5-a]pyridine correspondante de formule I par l'hydrogène en présence de catalyseurs d'hydrogénation.

8. Procédé selon l'une des revendications 1 à 2 ou 4 à 5, caractérisé en ce que l'on prépare des composés de formule générale I dans laquelle $R_1$ représente l'hydrogène, un halogène, un groupe alkyle inférieur, alcoxy inférieur, hydroxy, phényl-alcoxy inférieur ou phényl-alcoxy inférieur mono- ou di-substitué dans le noyau phényle par des groupes alcoxy inférieurs, alkyle inférieurs ou des halogènes, A représente un groupe éthénylène ou éthénylène substitué par un groupe alkyle inférieur, B représente un groupe alkyléne en C 1–C 12, alcynylène ou alcénylène en C 2–C 12, phénylène-alkylène en C 1–C 4, phénylène-alcénylène en C 2–C 4, phénylène ou phénylène-(thio ou oxy)-alkylène en C 1–C 4 et $R_2$ et C ont les significations indiquées dans la revendication 1, 2, 4 ou 5, ou leurs sels.

9. Procédé selon l'une des revendications 1 ou 3 à 7, caractérisé en ce que l'on prépare des composés de formule gènérale I ou leurs dérivés 5,6,7,8-tétrahydrogénés dans lesquels:

$R_1$ représente l'hydrogène, un halogène, un groupe alkyle inférieur, alcoxy inférieur, hydroxy ou aryl-alcoxy inférieur dans lesquels le groupe aryle est un groupe phényle non substitué ou mono- ou di-substitué par des groupes alcoxy inférieurs, alkyle inférieurs, des halogènes ou des groupes trifluorométhyle,

$R_2$ représente l'hydrogène, un halogène ou un groupe alkyle inférieur,

C représente un groupe carboxy, (alcoxy inférieur)-carbonyle, carbamoyle non substitué ou mono- ou di-substitué par des groupes alkyle inférieurs, cyano, hydroxyméthyle, formyle, 5-tétrazolyle, 4,5-dihydro-2-oxazolyle non substitué ou substitué par un groupe alkyle inférieur, ou hydroxycarbamoyle,

A représente une liaison simple, et

B représente un groupe (alkylène en C 1–C 4)-phénylène-alkylène en C 1–C 4, (alkylène en C 1–C 4)-phénylène, phénylène-alkylène en C 1–C 4, phénylène, (alkylène inférieur)-(thio ou oxy)-alkylène inférieur contenant au total 2 à 12 atomes de carbone, (alkylène en C 1–C 4)-thio ou oxy)-phénylène, phénylène-(thio ou oxy)-alkyléne en C 1–C 4, phénylène-alcénylène en C 2–C 4, (alkylène en C 1–C 4)-phénylène-alcénylène en C 2–C 4 ou alcadiénylène en C 5–C 12, ou leurs sels.

10. Procédé selon l'une des revendications 1 à 9, caractérisé en ce que l'on prépare des composés de formule générale I ou leurs dérivés 5,6,7,8-tétrahydrogénés dans lesquels le groupe –A–B–C est fixé en position 5 du cycle imidazo[1,5-a]pyridine.

11. Procédé selon la revendication 9, caractérisé en ce que l'on prépare des composés de formule générale I ou leurs dérivés 5,6,7,8-tétrahydrogénés dans lesquels le groupe –A–B–C est fixé en position 5 du cycle imidazo[1,5-a]pyridine et B représente un groupe (alkylène inférieur)-(thio ou oxy)-alkylène inférieur en C 4–C 10, phénylène ou phénylène-alcénylène inférieur en C 8–C 10.

12. Procédé selon l'une des revendications 1 à 2 ou 5, caractérisé en ce que l'on prépare des composés de formule générale II

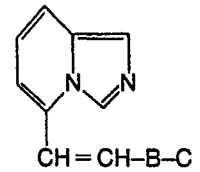

(II)

dans laquelle B représente un groupe alkylène ou alcénylène contenant chacun 2 à 4 atomes de carbone, C représente un groupe carboxy ou (alcoxy infèrieur)-carbonyle, ou leurs sels acceptables pour l'usage pharmaceutique.

13. Procédé selon l'une des revendications 1 ou 3 à 5, caractérisé en ce que l'on prépare des composés de formule générale III

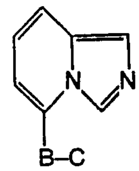

(III)

dans laquelle B représente un groupe (alkylène inférieur)-(thio ou oxy)-alkylène inférieur en C4–C 10, phénylène, phénylène-alkylène inférieur ou (alkylène inférieur)-phénylène contenant chacun 7 à 10 atomes de carbone, alkylène-thiophénylène ou alkylène-oxyphénylène contenant chacun 7 à 10 atomes de carbone ou phènylène-alcénylène inférieur en C 8–C 10, C représente un groupe carboxy, (alcoxy inférieur)-carbonyle ou carbamoyle, ou leurs sels acceptables pour l'usage pharmaceutique.

14. Procédé selon l'une des revendications 1 à 2 ou 5, caractérisé en ce que l'on prépare la 5-(4-carboxybuta-1,3-diényl)-imidazo[1,5-a]pyridine ou un sel acceptable pour l'usage pharmaceutique de ce composé.

15. Procédé selon l'une des revendications 1 ou 5, caractérisé en ce que l'on prépare la 5-[p-(2-carboxy-propa-1-ényl)-phényl]-imidazo[1,5-a]pyridine ou un sel acceptable pour l'usage pharmaceutique de ce composé.

16. Procédé selon l'une des revendications 1, 3 ou 5, caractérisé en ce que l'on prépare la 5-(9-carboxynona-6,8-diényl)-imidazo[1,5-a]pyridine ou un sel acceptable pour l'usage pharmaceutique de ce composé.

17. Procédé de préparation d'une composition pharmaceutique, caractérisé en ce que l'on combine une substance active selon l'invention, selon l'une des revendications 1 à 16, avec un véhicule pharmaceutique.